# EUROPEAN PATENT APPLICATION

(11) **EP 1 980 267 A1**
(43) Date of publication of application: **15.10.2008**
(21) Application number: 07106161.8
(22) Date of filing: 13.04.2007
(51) Int. Cl.: A61K 39/09, C07K 16/12, C07K 14/315

(54) **S. pneumoniae transcytosis protein**

(71) Applicant: Rijksuniversiteit Groningen, 9712 CP Groningen (NL)
(72) Inventor: The designation of the inventor has not yet been filed
(74) Representative: van Loon, C.J.J.

(57) **Abstract**

The present invention relates to a vaccine formulation providing protection against infection of a subject by a meningeal pathogen, said formulation comprising an effective amount of the protein MgtA or an immunogenic part thereof together with at least one of a pharmaceutically acceptable diluent, carrier, excipient or adjuvant therefore.

SEQUENCE LISTING

<110>  Rijksuniversiteit Groningen

<120>  S. pneumoniae transcytosis protein

<130>  P80523EP00

<140>  EP 07106161.8
<141>  2007-04-13

<160>  38

<170>  PatentIn version 3.3

<210>  1
<211>  2745
<212>  DNA
<213>  Streptococcus pneumoniae


<220>
<221>  CDS
<222>  (1)..(2745)

<400>  1
atg aca gaa aga gag tct gtt ttg cac aca atg tca agg agg aga cac      48
Met Thr Glu Arg Glu Ser Val Leu His Thr Met Ser Arg Arg Arg His
1               5                   10                  15

atg tca aaa gaa caa aaa cgc caa gcg ttt tat act cag agc cct gaa      96
Met Ser Lys Glu Gln Lys Arg Gln Ala Phe Tyr Thr Gln Ser Pro Glu
            20                  25                  30

gag gtc ttg cag gct gtg gat gcg acc gag caa ggt ttg tca tca agt      144
Glu Val Leu Gln Ala Val Asp Ala Thr Glu Gln Gly Leu Ser Ser Ser
        35                  40                  45

gag gcg gaa aag cgc ctt gcc gaa ttt ggg cac aat gaa ctc gaa gaa      192
Glu Ala Glu Lys Arg Leu Ala Glu Phe Gly His Asn Glu Leu Glu Glu
    50                  55                  60

ggc gag aaa cga tca atc ttg gtc aaa ttc atc gag caa ttt aag gat      240
Gly Glu Lys Arg Ser Ile Leu Val Lys Phe Ile Glu Gln Phe Lys Asp
65                  70                  75                  80

ttg atg att atc atc cta gtt gcg gca gca atc ttg tca gtc gtg act      288
Leu Met Ile Ile Ile Leu Val Ala Ala Ala Ile Leu Ser Val Val Thr
                85                  90                  95

tct ggt ggg gaa gat atc gca gat gcc att atc atc cta gct gtg gtt      336
Ser Gly Gly Glu Asp Ile Ala Asp Ala Ile Ile Ile Leu Ala Val Val
            100                 105                 110

atc atc aac gct gcc ttt ggt gtt tac caa gaa gga aaa gct gaa gaa      384
Ile Ile Asn Ala Ala Phe Gly Val Tyr Gln Glu Gly Lys Ala Glu Glu
        115                 120                 125

gct att gaa gcc ctc aaa tcc atg tct agt cca gtt gcc cgc gtt ctt      432
Ala Ile Glu Ala Leu Lys Ser Met Ser Ser Pro Val Ala Arg Val Leu
    130                 135                 140

cgt gat gga cac atg gca gag att gac tct aaa gaa ttg gta cca ggc      480
Arg Asp Gly His Met Ala Glu Ile Asp Ser Lys Glu Leu Val Pro Gly
145                 150                 155                 160

gat atc gtt gcc ctt gaa gca ggt gac gtg gta cca gcg gac cta cgt      528
Asp Ile Val Ala Leu Glu Ala Gly Asp Val Val Pro Ala Asp Leu Arg

                165                 170                 175

ttg ata gaa gcc aac tct ctt aaa att gaa gaa gca gcc ttg aca ggt 576
Leu Ile Glu Ala Asn Ser Leu Lys Ile Glu Glu Ala Ala Leu Thr Gly
            180                 185                 190

gaa tct gta cca gtc gaa aaa gac ttg tca gtc gag ctt gcg aca gat 624
Glu Ser Val Pro Val Glu Lys Asp Leu Ser Val Glu Leu Ala Thr Asp
        195                 200                 205

gct ggt att ggt gac cgt gtc aac atg gcc ttc caa aac tca aac gta 672
Ala Gly Ile Gly Asp Arg Val Asn Met Ala Phe Gln Asn Ser Asn Val
    210                 215                 220

acc tat ggt cgt ggg atg ggt gtt gtt gtc aat aca ggt atg tac act 720
Thr Tyr Gly Arg Gly Met Gly Val Val Val Asn Thr Gly Met Tyr Thr
225                 230                 235                 240

gaa gtt ggt cat att gct ggt atg ctt caa gat gcg gat gag act gat 768
Glu Val Gly His Ile Ala Gly Met Leu Gln Asp Ala Asp Glu Thr Asp
                245                 250                 255

aca cca ctc aaa caa aat ttg aac aac ctt tct aag gtc ttg acc tat 816
Thr Pro Leu Lys Gln Asn Leu Asn Asn Leu Ser Lys Val Leu Thr Tyr
            260                 265                 270

gct atc ttg gtc att gcc ctt gtt act ttt gta gtg ggt gtc ttc att 864
Ala Ile Leu Val Ile Ala Leu Val Thr Phe Val Val Gly Val Phe Ile
        275                 280                 285

caa ggg aaa aat cca ctt ggt gag ttg ttg act tct gtt gcc ctt gcc 912
Gln Gly Lys Asn Pro Leu Gly Glu Leu Leu Thr Ser Val Ala Leu Ala
    290                 295                 300

gtt gca gcc att cca gaa gga ctt cct gct atc gtt acc atc gtt ctt 960
Val Ala Ala Ile Pro Glu Gly Leu Pro Ala Ile Val Thr Ile Val Leu
305                 310                 315                 320

tct ctt ggt act caa gtt ttg gcc aaa cga cat tcc atc gtt cgt aag 1008
Ser Leu Gly Thr Gln Val Leu Ala Lys Arg His Ser Ile Val Arg Lys
                325                 330                 335

ttg cca gca gtt gaa aca ctt ggt tca act gaa atc atc gct tct gat 1056
Leu Pro Ala Val Glu Thr Leu Gly Ser Thr Glu Ile Ile Ala Ser Asp
            340                 345                 350

aag act ggt acg ctg act atg aac aag atg aca gtc gaa aaa gtc ttc 1104
Lys Thr Gly Thr Leu Thr Met Asn Lys Met Thr Val Glu Lys Val Phe
        355                 360                 365

tac gat gcg gtt cta cat gac tca gct gat gat att gaa cta ggt ctt 1152
Tyr Asp Ala Val Leu His Asp Ser Ala Asp Asp Ile Glu Leu Gly Leu
    370                 375                 380

gaa atg cca cta ctt cgt tca gtt gtc ttg gcc aat gat acg aaa atc 1200
Glu Met Pro Leu Leu Arg Ser Val Val Leu Ala Asn Asp Thr Lys Ile
385                 390                 395                 400

gat gtg gaa ggt aac ttg att ggt gac cca acc gaa aca gcc ttt atc 1248
Asp Val Glu Gly Asn Leu Ile Gly Asp Pro Thr Glu Thr Ala Phe Ile
                405                 410                 415

caa tat gcc ttg gac aag ggc tat gat gtc aaa ggt ttc tta gag aaa 1296
Gln Tyr Ala Leu Asp Lys Gly Tyr Asp Val Lys Gly Phe Leu Glu Lys
            420                 425                 430

tat cct cgt gta gct gaa ttg cca ttt gac tct gac cgt aag ctc atg 1344
Tyr Pro Arg Val Ala Glu Leu Pro Phe Asp Ser Asp Arg Lys Leu Met

435                 440                 445

tca aca gtt cac cca ttg cca gat ggt cgt ttc ctt gta gca gtc aag     1392
Ser Thr Val His Pro Leu Pro Asp Gly Arg Phe Leu Val Ala Val Lys
    450                 455                 460

ggt gcg cca gac caa ctc tta aaa cgt tgt ctt ctt cgt gat aag gct     1440
Gly Ala Pro Asp Gln Leu Leu Lys Arg Cys Leu Leu Arg Asp Lys Ala
465                 470                 475                 480

ggg gat att gct ccg att gat gag aag gtt aca aat ctc att cgt aca     1488
Gly Asp Ile Ala Pro Ile Asp Glu Lys Val Thr Asn Leu Ile Arg Thr
                485                 490                 495

aac aat tct gaa atg gct cat caa gcc ttg cgt gtc ctt gca ggt gct     1536
Asn Asn Ser Glu Met Ala His Gln Ala Leu Arg Val Leu Ala Gly Ala
            500                 505                 510

tat aag att atc gat agt att cca gaa aat ctc act tct gaa gag ctt     1584
Tyr Lys Ile Ile Asp Ser Ile Pro Glu Asn Leu Thr Ser Glu Glu Leu
        515                 520                 525

gaa aat gat tta att ttt act ggt ttg att ggg atg att gac cct gaa     1632
Glu Asn Asp Leu Ile Phe Thr Gly Leu Ile Gly Met Ile Asp Pro Glu
    530                 535                 540

cgt cct gaa gcc gct gag gct gtt cgt gtg gct aag gaa gcg gga atc     1680
Arg Pro Glu Ala Ala Glu Ala Val Arg Val Ala Lys Glu Ala Gly Ile
545                 550                 555                 560

cgt cca att atg att aca ggt gac cac caa gac aca gcg gaa gcc att     1728
Arg Pro Ile Met Ile Thr Gly Asp His Gln Asp Thr Ala Glu Ala Ile
                565                 570                 575

gcc aaa cgt ttg gga atc att gac gca aac gat aca gaa ggt cac gtt     1776
Ala Lys Arg Leu Gly Ile Ile Asp Ala Asn Asp Thr Glu Gly His Val
            580                 585                 590

tta act ggt gct gaa ctc aat gaa ctg tca gat gaa gaa ttt gaa aaa     1824
Leu Thr Gly Ala Glu Leu Asn Glu Leu Ser Asp Glu Glu Phe Glu Lys
        595                 600                 605

gtc gtt ggt caa tac tct gtt tat gcc cgt gtg tct cca gaa cac aag     1872
Val Val Gly Gln Tyr Ser Val Tyr Ala Arg Val Ser Pro Glu His Lys
    610                 615                 620

gtt cgt atc gtc aag gct tgg caa aaa caa ggt aaa gtc gtt gcc atg     1920
Val Arg Ile Val Lys Ala Trp Gln Lys Gln Gly Lys Val Val Ala Met
625                 630                 635                 640

aca ggt gac ggt gtc aat gac gcg cca gct ctg aaa aca gcc gat atc     1968
Thr Gly Asp Gly Val Asn Asp Ala Pro Ala Leu Lys Thr Ala Asp Ile
                645                 650                 655

ggt atc ggt atg gga atc act ggt aca gag gtt tct aag ggg gct tct     2016
Gly Ile Gly Met Gly Ile Thr Gly Thr Glu Val Ser Lys Gly Ala Ser
            660                 665                 670

gat atg att ctt gca gat gat aac ttt gcg act att atc gtc gca gtg     2064
Asp Met Ile Leu Ala Asp Asp Asn Phe Ala Thr Ile Ile Val Ala Val
        675                 680                 685

gaa gaa gga cgt aag gtc ttc tca aac att caa aag act att cag tac     2112
Glu Glu Gly Arg Lys Val Phe Ser Asn Ile Gln Lys Thr Ile Gln Tyr
    690                 695                 700

cta ctt tct gct aat act gct gaa gta tta acc atc ttc cta tca acc     2160
Leu Leu Ser Ala Asn Thr Ala Glu Val Leu Thr Ile Phe Leu Ser Thr

705                 710                 715                 720

ttg ttt ggt tgg gat gtc tta cag ccg gtt cat ctt ttg tgg atc aac      2208
Leu Phe Gly Trp Asp Val Leu Gln Pro Val His Leu Leu Trp Ile Asn
                725                 730                 735

ttg gta acg gat acc ttc cca gct atc gct ctt ggt gtt gaa cct gcg      2256
Leu Val Thr Asp Thr Phe Pro Ala Ile Ala Leu Gly Val Glu Pro Ala
            740                 745                 750

gaa cct ggt gtc atg aat cat aaa cca cgt gga cgc aag gca agc ttc      2304
Glu Pro Gly Val Met Asn His Lys Pro Arg Gly Arg Lys Ala Ser Phe
        755                 760                 765

ttc tca ggt ggt gtt ttg agt tct atc att tat caa ggt gta ctc caa      2352
Phe Ser Gly Gly Val Leu Ser Ser Ile Ile Tyr Gln Gly Val Leu Gln
    770                 775                 780

gca gct ctt gtt atg agt gtt tat ggc ctt gcg att gct tac cca gtt      2400
Ala Ala Leu Val Met Ser Val Tyr Gly Leu Ala Ile Ala Tyr Pro Val
785                 790                 795                 800

cat gtg ggt gac aat cat gct att cat gca gat gcc cta acg atg gcc      2448
His Val Gly Asp Asn His Ala Ile His Ala Asp Ala Leu Thr Met Ala
                805                 810                 815

ttt gca acc ctt ggt ttg att cag ctc ttc cat gcc tac aat gtc aag      2496
Phe Ala Thr Leu Gly Leu Ile Gln Leu Phe His Ala Tyr Asn Val Lys
            820                 825                 830

tct gtc tac caa tcc atc ttg aca gtt ggc cca ttc aag tct aag acc      2544
Ser Val Tyr Gln Ser Ile Leu Thr Val Gly Pro Phe Lys Ser Lys Thr
        835                 840                 845

ttt aac tgg tcc atc ttg gta tcc ttt atc ctt ctc atg gca aca atc      2592
Phe Asn Trp Ser Ile Leu Val Ser Phe Ile Leu Leu Met Ala Thr Ile
    850                 855                 860

gtt gta gaa ccg ctt gaa gga atc ttc cac gta acc aaa cta gac ttg      2640
Val Val Glu Pro Leu Glu Gly Ile Phe His Val Thr Lys Leu Asp Leu
865                 870                 875                 880

tca caa tgg gga att gtt atg gct gga agc ttc tca atg att atc atc      2688
Ser Gln Trp Gly Ile Val Met Ala Gly Ser Phe Ser Met Ile Ile Ile
                885                 890                 895

gtc gaa atc gtt aag ttt att caa cgc aaa ctc ggt ttt gac aag aat      2736
Val Glu Ile Val Lys Phe Ile Gln Arg Lys Leu Gly Phe Asp Lys Asn
            900                 905                 910

gcg att taa                                                          2745
Ala Ile

<210>  2
<211>  914
<212>  PRT
<213>  Streptococcus pneumoniae

<400>  2

Met Thr Glu Arg Glu Ser Val Leu His Thr Met Ser Arg Arg Arg His
1               5                   10                  15

Met Ser Lys Glu Gln Lys Arg Gln Ala Phe Tyr Thr Gln Ser Pro Glu
            20                  25                  30

Glu Val Leu Gln Ala Val Asp Ala Thr Glu Gln Gly Leu Ser Ser Ser
        35                  40                  45

Glu Ala Glu Lys Arg Leu Ala Glu Phe Gly His Asn Glu Leu Glu Glu
    50                  55                  60

Gly Glu Lys Arg Ser Ile Leu Val Lys Phe Ile Glu Gln Phe Lys Asp
65                  70                  75                  80

Leu Met Ile Ile Ile Leu Val Ala Ala Ala Ile Leu Ser Val Val Thr
                85                  90                  95

Ser Gly Gly Glu Asp Ile Ala Asp Ala Ile Ile Ile Leu Ala Val Val
            100                 105                 110

Ile Ile Asn Ala Ala Phe Gly Val Tyr Gln Glu Gly Lys Ala Glu Glu
        115                 120                 125

Ala Ile Glu Ala Leu Lys Ser Met Ser Ser Pro Val Ala Arg Val Leu
    130                 135                 140

Arg Asp Gly His Met Ala Glu Ile Asp Ser Lys Glu Leu Val Pro Gly
145                 150                 155                 160

Asp Ile Val Ala Leu Glu Ala Gly Asp Val Val Pro Ala Asp Leu Arg
                165                 170                 175

Leu Ile Glu Ala Asn Ser Leu Lys Ile Glu Glu Ala Ala Leu Thr Gly
            180                 185                 190

Glu Ser Val Pro Val Glu Lys Asp Leu Ser Val Glu Leu Ala Thr Asp
        195                 200                 205

Ala Gly Ile Gly Asp Arg Val Asn Met Ala Phe Gln Asn Ser Asn Val
    210                 215                 220

Thr Tyr Gly Arg Gly Met Gly Val Val Val Asn Thr Gly Met Tyr Thr
225                 230                 235                 240

Glu Val Gly His Ile Ala Gly Met Leu Gln Asp Ala Asp Glu Thr Asp
                245                 250                 255

Thr Pro Leu Lys Gln Asn Leu Asn Asn Leu Ser Lys Val Leu Thr Tyr
            260                 265                 270

Ala Ile Leu Val Ile Ala Leu Val Thr Phe Val Val Gly Val Phe Ile
        275                 280                 285

Gln Gly Lys Asn Pro Leu Gly Glu Leu Leu Thr Ser Val Ala Leu Ala
    290                 295                 300

Val Ala Ala Ile Pro Glu Gly Leu Pro Ala Ile Val Thr Ile Val Leu
305                 310                 315                 320

Ser Leu Gly Thr Gln Val Leu Ala Lys Arg His Ser Ile Val Arg Lys
                325                 330                 335

Leu Pro Ala Val Glu Thr Leu Gly Ser Thr Glu Ile Ile Ala Ser Asp
            340                 345                 350

Lys Thr Gly Thr Leu Thr Met Asn Lys Met Thr Val Glu Lys Val Phe
        355                 360                 365

Tyr Asp Ala Val Leu His Asp Ser Ala Asp Asp Ile Glu Leu Gly Leu
    370                 375                 380

Glu Met Pro Leu Leu Arg Ser Val Val Leu Ala Asn Asp Thr Lys Ile
385                 390                 395                 400

Asp Val Glu Gly Asn Leu Ile Gly Asp Pro Thr Glu Thr Ala Phe Ile
                405                 410                 415

Gln Tyr Ala Leu Asp Lys Gly Tyr Asp Val Lys Gly Phe Leu Glu Lys
            420                 425                 430

Tyr Pro Arg Val Ala Glu Leu Pro Phe Asp Ser Asp Arg Lys Leu Met
        435                 440                 445

Ser Thr Val His Pro Leu Pro Asp Gly Arg Phe Leu Val Ala Val Lys
    450                 455                 460

Gly Ala Pro Asp Gln Leu Leu Lys Arg Cys Leu Leu Arg Asp Lys Ala
465                 470                 475                 480

Gly Asp Ile Ala Pro Ile Asp Glu Lys Val Thr Asn Leu Ile Arg Thr
                485                 490                 495

Asn Asn Ser Glu Met Ala His Gln Ala Leu Arg Val Leu Ala Gly Ala
            500                 505                 510

Tyr Lys Ile Ile Asp Ser Ile Pro Glu Asn Leu Thr Ser Glu Glu Leu
        515                 520                 525

Glu Asn Asp Leu Ile Phe Thr Gly Leu Ile Gly Met Ile Asp Pro Glu
    530                 535                 540

Arg Pro Glu Ala Ala Glu Ala Val Arg Val Ala Lys Glu Ala Gly Ile
545                 550                 555                 560

Arg Pro Ile Met Ile Thr Gly Asp His Gln Asp Thr Ala Glu Ala Ile
                565                 570                 575

Ala Lys Arg Leu Gly Ile Ile Asp Ala Asn Asp Thr Glu Gly His Val
            580                 585                 590

Leu Thr Gly Ala Glu Leu Asn Glu Leu Ser Asp Glu Glu Phe Glu Lys
        595                 600                 605

Val Val Gly Gln Tyr Ser Val Tyr Ala Arg Val Ser Pro Glu His Lys
    610                 615                 620

Val Arg Ile Val Lys Ala Trp Gln Lys Gln Gly Lys Val Val Ala Met
625                 630                 635                 640

Thr Gly Asp Gly Val Asn Asp Ala Pro Ala Leu Lys Thr Ala Asp Ile
                645                 650                 655

Gly Ile Gly Met Gly Ile Thr Gly Thr Glu Val Ser Lys Gly Ala Ser
            660                 665                 670

Asp Met Ile Leu Ala Asp Asp Asn Phe Ala Thr Ile Ile Val Ala Val
        675                 680                 685

Glu Glu Gly Arg Lys Val Phe Ser Asn Ile Gln Lys Thr Ile Gln Tyr
    690                 695                 700

Leu Leu Ser Ala Asn Thr Ala Glu Val Leu Thr Ile Phe Leu Ser Thr
705                 710                 715                 720

Leu Phe Gly Trp Asp Val Leu Gln Pro Val His Leu Leu Trp Ile Asn
                725                 730                 735

Leu Val Thr Asp Thr Phe Pro Ala Ile Ala Leu Gly Val Glu Pro Ala
            740                 745                 750

Glu Pro Gly Val Met Asn His Lys Pro Arg Gly Arg Lys Ala Ser Phe
        755                 760                 765

Phe Ser Gly Gly Val Leu Ser Ser Ile Ile Tyr Gln Gly Val Leu Gln
    770                 775                 780

Ala Ala Leu Val Met Ser Val Tyr Gly Leu Ala Ile Ala Tyr Pro Val
785                 790                 795                 800

His Val Gly Asp Asn His Ala Ile His Ala Asp Ala Leu Thr Met Ala
                805                 810                 815

Phe Ala Thr Leu Gly Leu Ile Gln Leu Phe His Ala Tyr Asn Val Lys
            820                 825                 830

Ser Val Tyr Gln Ser Ile Leu Thr Val Gly Pro Phe Lys Ser Lys Thr
        835                 840                 845

Phe Asn Trp Ser Ile Leu Val Ser Phe Ile Leu Leu Met Ala Thr Ile
    850                 855                 860

Val Val Glu Pro Leu Glu Gly Ile Phe His Val Thr Lys Leu Asp Leu
865                 870                 875                 880

Ser Gln Trp Gly Ile Val Met Ala Gly Ser Phe Ser Met Ile Ile Ile
                885                 890                 895

Val Glu Ile Val Lys Phe Ile Gln Arg Lys Leu Gly Phe Asp Lys Asn
            900                 905                 910

Ala Ile

<210>  3
<211>  2565
<212>  DNA
<213>  Streptococcus pneumoniae

<220>
<221>  CDS
<222>  (1)..(2565)

<400>  3
atg aca gaa aga gag tct gtt ttg cac aca atg tca agg agg aga cac      48
Met Thr Glu Arg Glu Ser Val Leu His Thr Met Ser Arg Arg Arg His
1               5                   10                  15

atg tca aaa gaa caa aaa cgc caa gcg ttt tat act cag agc cct gaa      96
Met Ser Lys Glu Gln Lys Arg Gln Ala Phe Tyr Thr Gln Ser Pro Glu
            20                  25                  30

gag gtc ttg cag gct gtg gat gcg acc gag caa ggt ttg tca tca agt      144
Glu Val Leu Gln Ala Val Asp Ala Thr Glu Gln Gly Leu Ser Ser Ser
        35                  40                  45

gag gcg gaa aag cgc ctt gcc gaa ttt ggg cac aat tac caa gaa gga      192
Glu Ala Glu Lys Arg Leu Ala Glu Phe Gly His Asn Tyr Gln Glu Gly
    50                  55                  60

aaa gct gaa gaa gct att gaa gcc ctc aaa tcc atg tct agt cca gtt      240
Lys Ala Glu Glu Ala Ile Glu Ala Leu Lys Ser Met Ser Ser Pro Val
65                  70                  75                  80

gcc cgc gtt ctt cgt gat gga cac atg gca gag att gac tct aaa gaa      288
Ala Arg Val Leu Arg Asp Gly His Met Ala Glu Ile Asp Ser Lys Glu
                85                  90                  95

ttg gta cca ggc gat atc gtt gcc ctt gaa gca ggt gac gtg gta cca      336
Leu Val Pro Gly Asp Ile Val Ala Leu Glu Ala Gly Asp Val Val Pro
            100                 105                 110

gcg gac cta cgt ttg ata gaa gcc aac tct ctt aaa att gaa gaa gca      384
Ala Asp Leu Arg Leu Ile Glu Ala Asn Ser Leu Lys Ile Glu Glu Ala
        115                 120                 125

gcc ttg aca ggt gaa tct gta cca gtc gaa aaa gac ttg tca gtc gat      432
Ala Leu Thr Gly Glu Ser Val Pro Val Glu Lys Asp Leu Ser Val Asp
    130                 135                 140

ctt gcg aca gat gct ggc att ggt gac cgt gtc aac atg gcc ttc caa 480
Leu Ala Thr Asp Ala Gly Ile Gly Asp Arg Val Asn Met Ala Phe Gln
145                 150                 155                 160

aac tca aac gta acc tat ggt cgt ggg atg ggt gtt gtt gtc aat aca 528
Asn Ser Asn Val Thr Tyr Gly Arg Gly Met Gly Val Val Val Asn Thr
                165                 170                 175

ggt atg tat act gaa gtt ggt cat att gct ggt atg ctt caa gat gcg 576
Gly Met Tyr Thr Glu Val Gly His Ile Ala Gly Met Leu Gln Asp Ala
            180                 185                 190

gat gag act gat aca cca ctc aaa caa aac ttg aac aac ctt tct aag 624
Asp Glu Thr Asp Thr Pro Leu Lys Gln Asn Leu Asn Asn Leu Ser Lys
        195                 200                 205

gtc ttg acc tat gct atc ttg gtc att gcc ctt gtt act ttt gta gtg 672
Val Leu Thr Tyr Ala Ile Leu Val Ile Ala Leu Val Thr Phe Val Val
    210                 215                 220

ggt gtc ttc att caa ggg aaa aat cca ctt ggt gag ttg ttg act tct 720
Gly Val Phe Ile Gln Gly Lys Asn Pro Leu Gly Glu Leu Leu Thr Ser
225                 230                 235                 240

gtt gcc ctt gct gtt gca gcc att cca gaa gga ctt cct gct atc gtt 768
Val Ala Leu Ala Val Ala Ala Ile Pro Glu Gly Leu Pro Ala Ile Val
                245                 250                 255

acc atc gtt ctt tct ctt ggt act caa gtt ttg gcc aaa cga cat tcc 816
Thr Ile Val Leu Ser Leu Gly Thr Gln Val Leu Ala Lys Arg His Ser
            260                 265                 270

atc gtt cgt aag ttg cca gca gtt gaa aca ctt ggt tca act gaa atc 864
Ile Val Arg Lys Leu Pro Ala Val Glu Thr Leu Gly Ser Thr Glu Ile
        275                 280                 285

atc gct tct gat aag act ggt acg ctg act atg aac aag atg aca gtc 912
Ile Ala Ser Asp Lys Thr Gly Thr Leu Thr Met Asn Lys Met Thr Val
    290                 295                 300

gaa aaa gtc ttc tac gat gcg gtt cta cat gac tca gct gat gat att 960
Glu Lys Val Phe Tyr Asp Ala Val Leu His Asp Ser Ala Asp Asp Ile
305                 310                 315                 320

gaa cta ggt ctt gaa atg cca cta ctt cgt tca gtt gtc ttg gcc aat 1008
Glu Leu Gly Leu Glu Met Pro Leu Leu Arg Ser Val Val Leu Ala Asn
                325                 330                 335

gat acg aaa atc gat gtg gaa ggt aac ttg att ggt gac cca acc gaa 1056
Asp Thr Lys Ile Asp Val Glu Gly Asn Leu Ile Gly Asp Pro Thr Glu
            340                 345                 350

aca gcc ttt atc caa tat gcc ttg gac aag ggc tat gat gtc aaa ggt 1104
Thr Ala Phe Ile Gln Tyr Ala Leu Asp Lys Gly Tyr Asp Val Lys Gly
        355                 360                 365

ttc tta gag aaa tat cct cgt gta gct gaa ttg cca ttt gac tct gac 1152
Phe Leu Glu Lys Tyr Pro Arg Val Ala Glu Leu Pro Phe Asp Ser Asp
    370                 375                 380

cgt aag ctc atg tca aca gtt cac cca ttg cca gat agt cgt ttc ctt 1200
Arg Lys Leu Met Ser Thr Val His Pro Leu Pro Asp Ser Arg Phe Leu
385                 390                 395                 400

gta gca gtc aag ggt gcg cca gac caa ctc tta aaa cgt tgt ctt ctt 1248
Val Ala Val Lys Gly Ala Pro Asp Gln Leu Leu Lys Arg Cys Leu Leu
                405                 410                 415

cgt gat aag gct ggg gat att gct ccg att gat gag aag gtt aca aat 1296
Arg Asp Lys Ala Gly Asp Ile Ala Pro Ile Asp Glu Lys Val Thr Asn
            420                 425                 430

ctc att cat aca aac aat tct gaa atg gct cat caa gcc ttg cgt gtc 1344
Leu Ile His Thr Asn Asn Ser Glu Met Ala His Gln Ala Leu Arg Val
        435                 440                 445

ctt gca ggt gct tat aag att atc gat agt att cca gaa aat ctc act 1392
Leu Ala Gly Ala Tyr Lys Ile Ile Asp Ser Ile Pro Glu Asn Leu Thr
    450                 455                 460

tct gaa gag ctt gaa aat gat tta att ttt act ggt ttg att ggg atg 1440
Ser Glu Glu Leu Glu Asn Asp Leu Ile Phe Thr Gly Leu Ile Gly Met
465                 470                 475                 480

att gac cct gaa cgt cct gaa gcc gct gag gct gtt cgt gtg gct aag 1488
Ile Asp Pro Glu Arg Pro Glu Ala Ala Glu Ala Val Arg Val Ala Lys
                485                 490                 495

gaa gcg gga atc cgt cca att atg att aca ggt gac cac caa gac aca 1536
Glu Ala Gly Ile Arg Pro Ile Met Ile Thr Gly Asp His Gln Asp Thr
            500                 505                 510

gcg gaa gcc att gcc aaa cgt ttg gga atc att gac gca aac gat aca 1584
Ala Glu Ala Ile Ala Lys Arg Leu Gly Ile Ile Asp Ala Asn Asp Thr
        515                 520                 525

gaa ggt cac gtt tta act ggt gct gaa ctc aat gaa ctg tca gat gaa 1632
Glu Gly His Val Leu Thr Gly Ala Glu Leu Asn Glu Leu Ser Asp Glu
    530                 535                 540

gaa ttt gaa aaa gtt gtt ggt caa tac tct gtt tat gcc cgt gtg tct 1680
Glu Phe Glu Lys Val Val Gly Gln Tyr Ser Val Tyr Ala Arg Val Ser
545                 550                 555                 560

cca gaa cac aag gtt cgt atc gtc aag gct tgg caa aaa caa ggt aaa 1728
Pro Glu His Lys Val Arg Ile Val Lys Ala Trp Gln Lys Gln Gly Lys
                565                 570                 575

gtc gtt gcc atg aca ggt gac ggt gtc aat gac gcg cca gct ctg aaa 1776
Val Val Ala Met Thr Gly Asp Gly Val Asn Asp Ala Pro Ala Leu Lys
            580                 585                 590

aca gcc gat att ggt atc ggt atg gga atc act ggt aca gag gtt tct 1824
Thr Ala Asp Ile Gly Ile Gly Met Gly Ile Thr Gly Thr Glu Val Ser
        595                 600                 605

aag ggg gct tct gat atg att ctt gca gat gat aac ttt gcg act att 1872
Lys Gly Ala Ser Asp Met Ile Leu Ala Asp Asp Asn Phe Ala Thr Ile
    610                 615                 620

atc gtc gca gtg gaa gaa gga cgt aag gtc ttc tca aac att caa aag 1920
Ile Val Ala Val Glu Glu Gly Arg Lys Val Phe Ser Asn Ile Gln Lys
625                 630                 635                 640

act att cag tac cta ctt tct gct aat act gct gaa gta tta acc atc 1968
Thr Ile Gln Tyr Leu Leu Ser Ala Asn Thr Ala Glu Val Leu Thr Ile
                645                 650                 655

ttc cta tca acc ttg ttt ggt tgg gat gtc ttg cag ccg gtt cat ctt 2016
Phe Leu Ser Thr Leu Phe Gly Trp Asp Val Leu Gln Pro Val His Leu
            660                 665                 670

ttg tgg atc aac ttg gta acg gat acc ttc cca gct atc gct ctt ggt 2064
Leu Trp Ile Asn Leu Val Thr Asp Thr Phe Pro Ala Ile Ala Leu Gly
        675                 680                 685

gtt gaa cct gcg gaa cct ggt gtc atg aat cat aaa cca cgt gga cgc 2112
Val Glu Pro Ala Glu Pro Gly Val Met Asn His Lys Pro Arg Gly Arg
    690                 695                 700

aag gca agc ttc ttc tca ggt ggt gtt ttg agt tct atc att tat caa 2160
Lys Ala Ser Phe Phe Ser Gly Gly Val Leu Ser Ser Ile Ile Tyr Gln
705                 710                 715                 720

ggt gta ctc caa gca gct ctt gtt atg agt gtt tat ggc ctt gcg att 2208
Gly Val Leu Gln Ala Ala Leu Val Met Ser Val Tyr Gly Leu Ala Ile
                725                 730                 735

gct tac cca gtt cat gtg ggt gac aat cat gct att cat gca gat gcc 2256
Ala Tyr Pro Val His Val Gly Asp Asn His Ala Ile His Ala Asp Ala
            740                 745                 750

cta acg atg gcc ttt gca acc ctt ggt ttg att cag ctc ttc cat gcc 2304
Leu Thr Met Ala Phe Ala Thr Leu Gly Leu Ile Gln Leu Phe His Ala
        755                 760                 765

tac aat gtc aag tct gtc tac caa tcc atc ttg aca gtt ggc cca ttc 2352
Tyr Asn Val Lys Ser Val Tyr Gln Ser Ile Leu Thr Val Gly Pro Phe
    770                 775                 780

aag tct aag acc ttt aac tgg tcc atc ttg gta tcc ttt atc ctt ctc 2400
Lys Ser Lys Thr Phe Asn Trp Ser Ile Leu Val Ser Phe Ile Leu Leu
785                 790                 795                 800

atg gca aca atc gtt gta gaa ccg ctt gaa gga atc ttc cac gta acc 2448
Met Ala Thr Ile Val Val Glu Pro Leu Glu Gly Ile Phe His Val Thr
                805                 810                 815

aaa cta gac ttg tca caa tgg gga att gtt atg gct gga agc ttc tca 2496
Lys Leu Asp Leu Ser Gln Trp Gly Ile Val Met Ala Gly Ser Phe Ser
            820                 825                 830

atg att atc atc gtc gaa atc gtt aag ttt att caa cgc aaa ctc ggt 2544
Met Ile Ile Ile Val Glu Ile Val Lys Phe Ile Gln Arg Lys Leu Gly
        835                 840                 845

ttt gac aag aat gcg att taa 2565
Phe Asp Lys Asn Ala Ile
    850

<210> 4
<211> 854
<212> PRT
<213> Streptococcus pneumoniae

<400> 4

Met Thr Glu Arg Glu Ser Val Leu His Thr Met Ser Arg Arg Arg His
1               5                   10                  15

Met Ser Lys Glu Gln Lys Arg Gln Ala Phe Tyr Thr Gln Ser Pro Glu
            20                  25                  30

Glu Val Leu Gln Ala Val Asp Ala Thr Glu Gln Gly Leu Ser Ser Ser
        35                  40                  45

Glu Ala Glu Lys Arg Leu Ala Glu Phe Gly His Asn Tyr Gln Glu Gly
    50                  55                  60

Lys Ala Glu Glu Ala Ile Glu Ala Leu Lys Ser Met Ser Ser Pro Val
65                  70                  75                  80

Ala Arg Val Leu Arg Asp Gly His Met Ala Glu Ile Asp Ser Lys Glu
                85                  90                  95

Leu Val Pro Gly Asp Ile Val Ala Leu Glu Ala Gly Asp Val Val Pro
            100                 105                 110

Ala Asp Leu Arg Leu Ile Glu Ala Asn Ser Leu Lys Ile Glu Glu Ala
        115                 120                 125

Ala Leu Thr Gly Glu Ser Val Pro Val Glu Lys Asp Leu Ser Val Asp
    130                 135                 140

Leu Ala Thr Asp Ala Gly Ile Gly Asp Arg Val Asn Met Ala Phe Gln
145                 150                 155                 160

Asn Ser Asn Val Thr Tyr Gly Arg Gly Met Gly Val Val Val Asn Thr
                165                 170                 175

Gly Met Tyr Thr Glu Val Gly His Ile Ala Gly Met Leu Gln Asp Ala
            180                 185                 190

Asp Glu Thr Asp Thr Pro Leu Lys Gln Asn Leu Asn Asn Leu Ser Lys
        195                 200                 205

Val Leu Thr Tyr Ala Ile Leu Val Ile Ala Leu Val Thr Phe Val Val
    210                 215                 220

Gly Val Phe Ile Gln Gly Lys Asn Pro Leu Gly Glu Leu Leu Thr Ser
225                 230                 235                 240

Val Ala Leu Ala Val Ala Ala Ile Pro Glu Gly Leu Pro Ala Ile Val
                245                 250                 255

Thr Ile Val Leu Ser Leu Gly Thr Gln Val Leu Ala Lys Arg His Ser
            260                 265                 270

Ile Val Arg Lys Leu Pro Ala Val Glu Thr Leu Gly Ser Thr Glu Ile
        275                 280                 285

Ile Ala Ser Asp Lys Thr Gly Thr Leu Thr Met Asn Lys Met Thr Val
    290                 295                 300

Glu Lys Val Phe Tyr Asp Ala Val Leu His Asp Ser Ala Asp Asp Ile
305                 310                 315                 320

Glu Leu Gly Leu Glu Met Pro Leu Leu Arg Ser Val Val Leu Ala Asn
                325                 330                 335

Asp Thr Lys Ile Asp Val Glu Gly Asn Leu Ile Gly Asp Pro Thr Glu
            340                 345                 350

Thr Ala Phe Ile Gln Tyr Ala Leu Asp Lys Gly Tyr Asp Val Lys Gly
        355                 360                 365

Phe Leu Glu Lys Tyr Pro Arg Val Ala Glu Leu Pro Phe Asp Ser Asp
    370                 375                 380

Arg Lys Leu Met Ser Thr Val His Pro Leu Pro Asp Ser Arg Phe Leu
385                 390                 395                 400

Val Ala Val Lys Gly Ala Pro Asp Gln Leu Leu Lys Arg Cys Leu Leu
                405                 410                 415

Arg Asp Lys Ala Gly Asp Ile Ala Pro Ile Asp Glu Lys Val Thr Asn
            420                 425                 430

Leu Ile His Thr Asn Asn Ser Glu Met Ala His Gln Ala Leu Arg Val
        435                 440                 445

Leu Ala Gly Ala Tyr Lys Ile Ile Asp Ser Ile Pro Glu Asn Leu Thr
    450                 455                 460

Ser Glu Glu Leu Glu Asn Asp Leu Ile Phe Thr Gly Leu Ile Gly Met
465                 470                 475                 480

Ile Asp Pro Glu Arg Pro Glu Ala Ala Glu Ala Val Arg Val Ala Lys
                485                 490                 495

Glu Ala Gly Ile Arg Pro Ile Met Ile Thr Gly Asp His Gln Asp Thr
            500                 505                 510

Ala Glu Ala Ile Ala Lys Arg Leu Gly Ile Ile Asp Ala Asn Asp Thr
        515                 520                 525

Glu Gly His Val Leu Thr Gly Ala Glu Leu Asn Glu Leu Ser Asp Glu
    530                 535                 540

Glu Phe Glu Lys Val Val Gly Gln Tyr Ser Val Tyr Ala Arg Val Ser
545                 550                 555                 560

Pro Glu His Lys Val Arg Ile Val Lys Ala Trp Gln Lys Gln Gly Lys
                565                 570                 575

Val Val Ala Met Thr Gly Asp Gly Val Asn Asp Ala Pro Ala Leu Lys
            580                 585                 590

Thr Ala Asp Ile Gly Ile Gly Met Gly Ile Thr Gly Thr Glu Val Ser
        595                 600                 605

Lys Gly Ala Ser Asp Met Ile Leu Ala Asp Asp Asn Phe Ala Thr Ile
    610                 615                 620

Ile Val Ala Val Glu Glu Gly Arg Lys Val Phe Ser Asn Ile Gln Lys
625                 630                 635                 640

Thr Ile Gln Tyr Leu Leu Ser Ala Asn Thr Ala Glu Val Leu Thr Ile
                645                 650                 655

Phe Leu Ser Thr Leu Phe Gly Trp Asp Val Leu Gln Pro Val His Leu
            660                 665                 670

Leu Trp Ile Asn Leu Val Thr Asp Thr Phe Pro Ala Ile Ala Leu Gly
        675                 680                 685

Val Glu Pro Ala Glu Pro Gly Val Met Asn His Lys Pro Arg Gly Arg
    690                 695                 700

Lys Ala Ser Phe Phe Ser Gly Gly Val Leu Ser Ser Ile Ile Tyr Gln
705                 710                 715                 720

Gly Val Leu Gln Ala Ala Leu Val Met Ser Val Tyr Gly Leu Ala Ile
                725                 730                 735

Ala Tyr Pro Val His Val Gly Asp Asn His Ala Ile His Ala Asp Ala
            740                 745                 750

Leu Thr Met Ala Phe Ala Thr Leu Gly Leu Ile Gln Leu Phe His Ala
        755                 760                 765

Tyr Asn Val Lys Ser Val Tyr Gln Ser Ile Leu Thr Val Gly Pro Phe
    770                 775                 780

Lys Ser Lys Thr Phe Asn Trp Ser Ile Leu Val Ser Phe Ile Leu Leu
785                 790                 795                 800

Met Ala Thr Ile Val Val Glu Pro Leu Glu Gly Ile Phe His Val Thr
                805                 810                 815

Lys Leu Asp Leu Ser Gln Trp Gly Ile Val Met Ala Gly Ser Phe Ser
            820                 825                 830

Met Ile Ile Ile Val Glu Ile Val Lys Phe Ile Gln Arg Lys Leu Gly
        835                 840                 845

Phe Asp Lys Asn Ala Ile
    850

<210>  5
<211>  893
<212>  PRT

<213>  Streptococcus pyogenes

<400>  5

Met Ser Lys Glu Gln Arg His Glu Ala Phe Tyr Thr Gln Ser Glu Glu
1               5                   10                  15

Thr Val Leu Ala Gln Leu Glu Thr Ser Arg Glu Gly Leu Thr Ser Ala
            20                  25                  30

Gln Ala Lys Glu Arg Leu Ala Glu Tyr Gly Arg Asn Glu Leu Asp Glu
        35                  40                  45

Gly Glu Lys Arg Ser Leu Phe Met Lys Phe Leu Asp Gln Phe Lys Asp
    50                  55                  60

Leu Met Ile Ile Ile Leu Ile Val Ala Ala Leu Leu Ser Val Leu Thr
65                  70                  75                  80

Glu Gly Met Glu Gly Leu Thr Asp Ala Ile Ile Ile Leu Ala Val Val
                85                  90                  95

Ile Leu Asn Ala Ala Phe Gly Val Tyr Gln Glu Gly Gln Ala Glu Ala
            100                 105                 110

Ala Ile Glu Ala Leu Lys Ser Met Ser Ser Pro Leu Ala Arg Ile Arg
        115                 120                 125

Arg Asp Gly His Val Thr Glu Ile Asp Ser Lys Glu Leu Val Pro Gly
    130                 135                 140

Asp Ile Val Leu Leu Glu Ala Gly Asp Val Val Pro Ala Asp Leu Arg
145                 150                 155                 160

Leu Leu Glu Ala Asn Ser Leu Lys Ile Glu Glu Ala Ala Leu Thr Gly
                165                 170                 175

Glu Ser Val Pro Val Glu Lys Asp Leu Ser Thr Ala Val Ser Glu Asp
            180                 185                 190

Ala Gly Ile Gly Asp Arg Val Asn Met Gly Tyr Gln Asn Ser Asn Val
        195                 200                 205

Thr Tyr Gly Arg Gly Ile Gly Val Ile Thr Asn Thr Gly Met Tyr Thr
    210                 215                 220

Glu Val Gly His Ile Ala Gly Met Leu Ala Asn Ala Asp Glu Thr Asp
225                 230                 235                 240

Thr Pro Leu Lys Gln Asn Leu Asp Asn Leu Ser Lys Ile Leu Thr Tyr
                245                 250                 255

Ala Ile Leu Val Ile Ala Ala Val Thr Phe Ala Val Gly Val Phe Leu
            260                 265                 270

Arg Gly Gln His Pro Leu Glu Gly Leu Met Thr Ser Val Ala Leu Ala
        275                 280                 285

Val Ala Ala Ile Pro Glu Gly Leu Pro Ala Ile Val Thr Val Val Leu
    290                 295                 300

Ser Leu Gly Thr Gln Val Leu Ala Lys Arg Asn Ala Ile Ile Arg Lys
305                 310                 315                 320

Leu Pro Ala Val Glu Thr Leu Gly Ser Thr Glu Ile Ile Ala Ser Asp
                325                 330                 335

Lys Thr Gly Thr Leu Thr Met Asn Gln Met Thr Val Glu Lys Val Tyr
            340                 345                 350

Thr Asn Gly Thr Leu Gln Ser Ser Ser Ala Asp Ile Ala Phe Asp Asn
        355                 360                 365

Thr Thr Leu Arg Val Met Asn Phe Ala Asn Asp Thr Lys Val Asp Pro
    370                 375                 380

Ser Gly Lys Leu Ile Gly Asp Pro Thr Glu Thr Ala Leu Val Glu Phe
385                 390                 395                 400

Gly Leu Asp His Asn Phe Asp Val Arg Glu Ala Met Val Ala Glu Pro
                405                 410                 415

Arg Val Ala Glu Leu Pro Phe Asp Ser Asp Arg Lys Leu Met Ser Thr
            420                 425                 430

Ile His Lys Gln Ala Asp Gly Lys Tyr Phe Ile Ala Val Lys Gly Ala
        435                 440                 445

Pro Asp Gln Leu Leu Lys Arg Val Thr Gln Ile Glu Glu Asn Gly Gln
    450                 455                 460

Ile Arg Pro Ile Thr Asp Ala Asp Lys Lys Thr Ile Leu Asp Thr Asn
465                 470                 475                 480

Lys Ser Leu Ala Lys Gln Ala Leu Arg Val Leu Met Met Ala Tyr Lys
                485                 490                 495

Tyr Ser Asp Ala Leu Pro Thr Leu Glu Thr Glu Ile Val Glu Ala Asn
            500                 505                 510

Leu Val Phe Ser Gly Leu Val Gly Met Ile Asp Pro Glu Arg Pro Glu
        515                 520                 525

Ala Ala Gln Ala Val Lys Val Ala Lys Glu Ala Gly Ile Arg Pro Ile
    530                 535                 540

Met Ile Thr Gly Asp His Gln Asp Thr Ala Lys Ala Ile Ala Lys Arg
545                 550                 555                 560

Leu Gly Ile Ile Glu Glu Asp Gly Val Asp His Val Phe Thr Gly Ala
                565                 570                 575

Glu Leu Asn Glu Leu Ser Asp Glu Glu Phe Gln Lys Val Phe Lys Gln
            580                 585                 590

Tyr Ser Val Tyr Ala Arg Val Ser Pro Glu His Lys Val Arg Ile Val
        595                 600                 605

Lys Ala Trp Gln Asn Glu Gly Lys Val Val Ala Met Thr Gly Asp Gly
    610                 615                 620

Val Asn Asp Ala Pro Ser Leu Lys Thr Ala Asp Ile Gly Ile Gly Met
625                 630                 635                 640

Gly Ile Thr Gly Thr Glu Val Ser Lys Gly Ala Ser Asp Met Val Leu
                645                 650                 655

Ala Asp Asp Asn Phe Ala Thr Ile Ile Val Ala Val Glu Glu Gly Arg
            660                 665                 670

Lys Val Phe Ser Asn Ile Gln Lys Thr Ile Gln Tyr Leu Leu Ser Ala
        675                 680                 685

Asn Met Ala Glu Val Phe Thr Ile Phe Leu Ala Thr Leu Phe Gly Trp
    690                 695                 700

Asp Val Leu Gln Pro Val His Leu Leu Trp Ile Asn Leu Val Thr Asp
705                 710                 715                 720

Thr Leu Pro Ala Ile Ala Leu Gly Val Glu Pro Ala Glu Pro Gly Val
                725                 730                 735

Met Lys His Lys Pro Arg Gly Arg Lys Ser Ser Phe Phe Asp Gly Gly
            740                 745                 750

Val Lys Glu Ala Ile Leu Tyr Gln Gly Ala Phe Gln Thr Ile Leu Val
        755                 760                 765

Leu Gly Val Tyr Gly Phe Ala Leu Met Phe Pro Glu His Thr Ser Tyr
    770                 775                 780

His Asp Val His Ala Asp Ala Leu Thr Met Ala Tyr Val Thr Leu Gly
785                 790                 795                 800

Leu Ile Gln Leu Val His Ala Tyr Asn Val Lys Ser Val Tyr Gln Ser
                805                 810                 815

Ile Phe Thr Val Gly Leu Phe Lys Asn Lys Leu Phe Asn Tyr Ser Ile
            820                 825                 830

Pro Val Ala Phe Val Ala Leu Met Ala Thr Val Val Val Pro Gly Phe
        835                 840                 845

Asn Gln Phe Phe His Val Thr His Leu Thr Ile Thr Gln Trp Leu Val
    850                 855                 860

Val Ile Ile Gly Ser Leu Leu Met Val Val Leu Val Glu Leu Val Lys
865                 870                 875                 880

Ala Val Gln Arg Ser Leu Gly Gln Asp Glu Lys Ala Ile
                885                 890

<210>  6
<211>  893
<212>  PRT
<213>  Streptococcus pyogenes

<400>  6

Met Ser Lys Glu Gln Arg His Glu Ala Phe Tyr Thr Gln Ser Glu Glu
1               5                   10                  15

Thr Val Leu Ala Gln Leu Glu Thr Ser Arg Glu Gly Leu Thr Ser Ala
            20                  25                  30

Gln Ala Lys Glu Arg Leu Ala Glu Tyr Gly Arg Asn Glu Leu Asp Glu
        35                  40                  45

Gly Glu Lys Arg Ser Leu Phe Met Lys Phe Leu Asp Gln Phe Lys Asp
    50                  55                  60

Leu Met Ile Ile Ile Leu Ile Val Ala Ala Leu Leu Ser Val Leu Thr
65                  70                  75                  80

Glu Gly Met Glu Gly Leu Thr Asp Ala Ile Ile Ile Leu Ala Val Val
                85                  90                  95

Ile Leu Asn Ala Ala Phe Gly Val Tyr Gln Glu Gly Gln Ala Glu Ala
            100                 105                 110

Ala Ile Glu Ala Leu Lys Ser Met Ser Ser Pro Leu Ala Arg Ile Arg
        115                 120                 125

Arg Asp Gly His Val Thr Glu Ile Asp Ser Lys Glu Leu Val Pro Gly
    130                 135                 140

Asp Ile Val Leu Leu Glu Ala Gly Asp Val Val Pro Ala Asp Leu Arg

145                 150                 155                 160

Leu Leu Glu Ala Asn Ser Leu Lys Ile Glu Glu Ala Ala Leu Thr Gly
                165                 170                 175

Glu Ser Val Pro Val Glu Lys Asp Leu Ser Thr Ala Val Ser Glu Asp
            180                 185                 190

Ala Gly Ile Gly Asp Arg Val Asn Met Gly Tyr Gln Asn Ser Asn Val
        195                 200                 205

Thr Tyr Gly Arg Gly Ile Gly Val Ile Thr Asn Thr Gly Met Tyr Thr
    210                 215                 220

Glu Val Gly His Ile Ala Gly Met Leu Ala Asn Ala Asp Glu Thr Asp
225                 230                 235                 240

Thr Pro Leu Lys Gln Asn Leu Asp Asn Leu Ser Lys Ile Leu Thr Tyr
                245                 250                 255

Ala Ile Leu Val Ile Ala Ala Val Thr Phe Ala Val Gly Val Phe Leu
            260                 265                 270

Arg Gly Gln His Pro Leu Glu Gly Leu Met Thr Ser Val Ala Leu Ala
        275                 280                 285

Val Ala Ala Ile Pro Glu Gly Leu Pro Ala Ile Val Thr Val Val Leu
    290                 295                 300

Ser Leu Gly Thr Gln Val Leu Ala Lys Arg Asn Ala Ile Ile Arg Lys
305                 310                 315                 320

Leu Pro Ala Val Glu Thr Leu Gly Ser Thr Glu Ile Ile Ala Ser Asp
                325                 330                 335

Lys Thr Gly Thr Leu Thr Met Asn Gln Met Thr Val Glu Lys Val Tyr
            340                 345                 350

Thr Asn Gly Thr Leu Gln Ser Ser Ser Ala Asp Ile Ala Phe Asp Asn
        355                 360                 365

Thr Thr Leu Arg Val Met Asn Phe Ala Asn Asp Thr Lys Val Asp Pro
    370                 375                 380

Ser Gly Lys Leu Ile Gly Asp Pro Thr Glu Thr Ala Leu Val Glu Phe
385                 390                 395                 400

Gly Leu Asp His Asn Phe Asp Val Arg Glu Ala Met Val Ala Glu Pro
                405                 410                 415

Arg Val Ala Glu Leu Pro Phe Asp Ser Asp Arg Lys Leu Met Ser Thr

            420                 425                 430

Ile His Lys Gln Ala Asp Gly Lys Tyr Phe Ile Ala Val Lys Gly Ala
        435                 440                 445

Pro Asp Gln Leu Leu Lys Arg Val Thr Gln Ile Glu Glu Asn Gly Gln
    450                 455                 460

Ile Arg Pro Ile Thr Asp Ala Asp Lys Lys Thr Ile Leu Asp Thr Asn
465                 470                 475                 480

Lys Ser Leu Ala Lys Gln Ala Leu Arg Val Leu Met Met Ala Tyr Lys
                485                 490                 495

Tyr Ser Asp Ala Leu Pro Thr Leu Glu Thr Glu Ile Val Glu Ala Asn
            500                 505                 510

Leu Val Phe Ser Gly Leu Val Gly Met Ile Asp Pro Glu Arg Pro Glu
        515                 520                 525

Ala Ala Gln Ala Val Lys Val Ala Lys Glu Ala Gly Ile Arg Pro Ile
    530                 535                 540

Met Ile Thr Gly Asp His Gln Asp Thr Ala Lys Ala Ile Ala Lys Arg
545                 550                 555                 560

Leu Gly Ile Ile Glu Glu Asp Gly Val Asp His Val Phe Thr Gly Ala
                565                 570                 575

Glu Leu Asn Glu Leu Ser Asp Glu Glu Phe Gln Lys Val Phe Lys Gln
            580                 585                 590

Tyr Ser Val Tyr Ala Arg Val Ser Pro Glu His Lys Val Arg Ile Val
        595                 600                 605

Lys Ala Trp Gln Asn Glu Gly Lys Val Val Ala Met Thr Gly Asp Gly
    610                 615                 620

Val Asn Asp Ala Pro Ser Leu Lys Thr Ala Asp Ile Gly Ile Gly Met
625                 630                 635                 640

Gly Ile Thr Gly Thr Glu Val Ser Lys Gly Ala Ser Asp Met Val Leu
                645                 650                 655

Ala Asp Asp Asn Phe Ala Thr Ile Ile Val Ala Val Glu Glu Gly Arg
            660                 665                 670

Lys Val Phe Ser Asn Ile Gln Lys Thr Ile Gln Tyr Leu Leu Ser Ala
        675                 680                 685

Asn Met Ala Glu Val Phe Thr Ile Phe Leu Ala Thr Leu Phe Gly Trp

690                    695                    700

Asp Val Leu Gln Pro Val His Leu Leu Trp Ile Asn Leu Val Thr Asp
705                 710                 715                 720

Thr Leu Pro Ala Ile Ala Leu Gly Val Glu Pro Ala Glu Pro Gly Val
                725                 730                 735

Met Lys His Lys Pro Arg Gly Arg Lys Ser Ser Phe Phe Asp Gly Gly
            740                 745                 750

Val Lys Glu Ala Ile Leu Tyr Gln Gly Ala Phe Gln Thr Ile Leu Val
        755                 760                 765

Leu Gly Val Tyr Gly Phe Ala Leu Met Phe Pro Glu His Thr Ser Tyr
    770                 775                 780

His Asp Val His Ala Asp Ala Leu Thr Met Ala Tyr Val Thr Leu Gly
785                 790                 795                 800

Leu Ile Gln Leu Val His Ala Tyr Asn Val Lys Ser Val Tyr Gln Ser
                805                 810                 815

Ile Phe Thr Val Gly Leu Phe Lys Asn Lys Leu Phe Asn Tyr Ser Ile
            820                 825                 830

Pro Val Ala Phe Val Ala Leu Met Ala Thr Val Val Val Pro Gly Phe
        835                 840                 845

Asn Gln Phe Phe His Val Thr His Leu Thr Ile Thr Gln Trp Leu Val
    850                 855                 860

Val Ile Ile Gly Ser Leu Leu Met Val Val Leu Val Glu Leu Val Lys
865                 870                 875                 880

Ala Val Gln Arg Ser Leu Gly Gln Asp Glu Lys Ala Ile
                885                 890

<210>  7
<211>  893
<212>  PRT
<213>  Streptococcus pyogenes

<400>  7

Met Ser Lys Glu Gln Arg His Glu Ala Phe Tyr Thr Gln Ser Glu Glu
1               5                   10                  15

Thr Val Leu Ala Gln Leu Glu Thr Ser Arg Glu Gly Leu Thr Ser Ala
            20                  25                  30

Gln Ala Lys Glu Arg Leu Ala Glu Tyr Gly Arg Asn Glu Leu Asp Glu
        35                  40                  45

Gly Glu Lys Arg Ser Leu Phe Met Lys Phe Leu Asp Gln Phe Lys Asp
    50                  55                  60

Leu Met Ile Ile Ile Leu Ile Val Ala Ala Leu Leu Ser Val Leu Thr
65                  70                  75                  80

Glu Gly Met Glu Gly Leu Thr Asp Ala Ile Ile Ile Leu Ala Val Val
                85                  90                  95

Ile Leu Asn Ala Ala Phe Gly Val Tyr Gln Glu Gly Gln Ala Glu Ala
            100                 105                 110

Ala Ile Glu Ala Leu Lys Ser Met Ser Ser Pro Leu Ala Arg Ile Arg
        115                 120                 125

Arg Asp Gly His Val Thr Glu Ile Asp Ser Lys Glu Leu Val Pro Gly
    130                 135                 140

Asp Ile Val Leu Leu Glu Ala Gly Asp Val Val Pro Ala Asp Leu Arg
145                 150                 155                 160

Leu Leu Glu Ala Asn Ser Leu Lys Ile Glu Glu Ala Ala Leu Thr Gly
                165                 170                 175

Glu Ser Val Pro Val Glu Lys Asp Leu Ser Thr Ala Val Ser Glu Asp
            180                 185                 190

Ala Gly Ile Gly Asp Arg Val Asn Met Gly Tyr Gln Asn Ser Asn Val
        195                 200                 205

Thr Tyr Gly Arg Gly Ile Gly Val Ile Thr Asn Thr Gly Met Tyr Thr
    210                 215                 220

Glu Val Gly His Ile Ala Gly Met Leu Ala Asn Ala Asp Glu Thr Asp
225                 230                 235                 240

Thr Pro Leu Lys Gln Asn Leu Asp Asn Leu Ser Lys Ile Leu Thr Tyr
                245                 250                 255

Ala Ile Leu Val Ile Ala Ala Val Thr Phe Ala Val Gly Val Phe Leu
            260                 265                 270

Arg Gly Gln His Pro Leu Glu Gly Leu Met Thr Ser Val Ala Leu Ala
        275                 280                 285

Val Ala Ala Ile Pro Glu Gly Leu Pro Ala Ile Val Thr Val Val Leu
    290                 295                 300

Ser Leu Gly Thr Gln Val Leu Ala Lys Arg Asn Ala Ile Ile Arg Lys
305                 310                 315                 320

Leu Pro Ala Val Glu Thr Leu Gly Ser Thr Glu Ile Ile Ala Ser Asp
                325                 330                 335

Lys Thr Gly Thr Leu Thr Met Asn Gln Met Thr Val Glu Lys Val Tyr
            340                 345                 350

Thr Asn Gly Thr Leu Gln Ser Ser Ser Ala Asp Ile Ala Phe Asp Asn
        355                 360                 365

Thr Thr Leu Arg Val Met Asn Phe Ala Asn Asp Thr Lys Val Asp Pro
    370                 375                 380

Ser Gly Lys Ser Ile Gly Asp Pro Thr Glu Thr Ala Leu Val Gln Phe
385                 390                 395                 400

Gly Leu Asp His Asn Phe Asp Val Arg Glu Ala Met Val Ala Glu Pro
                405                 410                 415

Arg Val Ala Glu Leu Pro Phe Asp Ser Asp Arg Lys Leu Met Ser Thr
            420                 425                 430

Ile His Lys Gln Ala Asp Gly Lys Tyr Phe Ile Ala Val Lys Gly Ala
        435                 440                 445

Pro Asp Gln Leu Leu Lys Arg Val Thr Gln Ile Glu Glu Asn Gly Gln
    450                 455                 460

Ile Arg Pro Ile Thr Asp Ala Asp Lys Lys Thr Ile Leu Asp Thr Asn
465                 470                 475                 480

Lys Ser Leu Ala Lys Gln Ala Leu Arg Val Leu Met Met Ala Tyr Lys
                485                 490                 495

Tyr Ser Asp Ala Leu Pro Thr Leu Glu Thr Glu Ile Val Glu Ala Asn
            500                 505                 510

Leu Val Phe Ser Gly Leu Val Gly Met Ile Asp Pro Glu Arg Pro Glu
        515                 520                 525

Ala Ala Gln Ala Val Lys Val Ala Lys Glu Ala Gly Ile Arg Pro Ile
    530                 535                 540

Met Ile Thr Gly Asp His Gln Asp Thr Ala Lys Ala Ile Ala Lys Arg
545                 550                 555                 560

Leu Gly Ile Ile Glu Glu Asp Gly Val Asp His Val Phe Thr Gly Ala
                565                 570                 575

Glu Leu Asn Glu Leu Ser Asp Glu Glu Phe Gln Lys Val Phe Lys Gln
            580                 585                 590

Tyr Ser Val Tyr Ala Arg Val Ser Pro Glu His Lys Val Arg Ile Val
        595                 600                 605

Lys Ala Trp Gln Asn Glu Gly Lys Val Val Ala Met Thr Gly Asp Gly
    610                 615                 620

Val Asn Asp Ala Pro Ser Leu Lys Thr Ala Asp Ile Gly Ile Gly Met
625                 630                 635                 640

Gly Ile Thr Gly Thr Glu Val Ser Lys Gly Ala Ser Asp Met Val Leu
                645                 650                 655

Ala Asp Asp Asn Phe Ala Thr Ile Ile Val Ala Val Glu Glu Gly Arg
            660                 665                 670

Lys Val Phe Ser Asn Ile Gln Lys Thr Ile Gln Tyr Leu Leu Ser Ala
        675                 680                 685

Asn Met Ala Glu Val Phe Thr Ile Phe Leu Ala Thr Leu Phe Gly Trp
    690                 695                 700

Asp Val Leu Gln Pro Val His Leu Leu Trp Ile Asn Leu Val Thr Asp
705                 710                 715                 720

Thr Leu Pro Ala Ile Ala Leu Gly Val Glu Pro Ala Glu Pro Gly Val
                725                 730                 735

Met Lys His Lys Pro Arg Gly Arg Lys Ser Ser Phe Phe Asp Gly Gly
            740                 745                 750

Val Lys Glu Ala Ile Leu Tyr Gln Gly Ala Phe Gln Thr Ile Leu Val
        755                 760                 765

Leu Gly Val Tyr Gly Phe Ala Leu Met Phe Pro Glu His Thr Ser Tyr
    770                 775                 780

His Asp Val His Ala Asp Ala Leu Thr Met Ala Tyr Val Thr Leu Gly
785                 790                 795                 800

Leu Ile Gln Leu Val His Ala Tyr Asn Val Lys Ser Val Tyr Gln Ser
                805                 810                 815

Ile Phe Thr Val Gly Leu Phe Lys Asn Lys Leu Phe Asn Tyr Ser Ile
            820                 825                 830

Pro Val Ala Phe Val Ala Leu Met Ala Thr Val Val Val Pro Gly Phe
        835                 840                 845

Asn Gln Phe Phe His Val Thr His Leu Thr Ile Thr Gln Trp Leu Val
    850                 855                 860

Val Ile Ile Gly Ser Leu Leu Met Val Val Leu Val Glu Leu Val Lys
865                 870                 875                 880

Ala Val Gln Arg Ser Leu Gly Gln Asp Glu Lys Ala Ile
                885                 890

<210>  8
<211>  893
<212>  PRT
<213>  Streptococcus pyogenes

<400>  8

Met Ser Lys Glu Gln Arg His Glu Ala Phe Tyr Thr Gln Ser Glu Glu
1               5                   10                  15

Thr Val Leu Ala Gln Leu Glu Thr Ser Arg Glu Gly Leu Thr Ser Ala
            20                  25                  30

Gln Ala Lys Glu Arg Leu Ala Glu Tyr Gly Arg Asn Glu Leu Asp Glu
        35                  40                  45

Gly Glu Lys Arg Ser Leu Phe Met Lys Phe Leu Asp Gln Phe Lys Asp
    50                  55                  60

Leu Met Ile Ile Ile Leu Ile Val Ala Ala Leu Leu Ser Val Leu Thr
65                  70                  75                  80

Glu Gly Met Glu Gly Leu Thr Asp Ala Ile Ile Ile Leu Ala Val Val
                85                  90                  95

Ile Leu Asn Ala Ala Phe Gly Val Tyr Gln Glu Gly Gln Ala Glu Ala
            100                 105                 110

Ala Ile Glu Ala Leu Lys Ser Met Ser Ser Pro Leu Ala Arg Ile Arg
        115                 120                 125

Arg Asp Gly His Val Thr Glu Ile Asp Ser Lys Glu Leu Val Pro Gly
    130                 135                 140

Asp Ile Val Leu Leu Glu Ala Gly Asp Val Val Pro Ala Asp Leu Arg
145                 150                 155                 160

Leu Leu Glu Ala Asn Ser Leu Lys Ile Glu Glu Ala Ala Leu Thr Gly
                165                 170                 175

Glu Ser Val Pro Val Glu Lys Asp Leu Ser Thr Ala Val Ser Glu Asp
            180                 185                 190

Ala Gly Ile Gly Asp Arg Val Asn Met Gly Tyr Gln Asn Ser Asn Val
        195                 200                 205

Thr Tyr Gly Arg Gly Ile Gly Val Ile Thr Asn Thr Gly Met Tyr Thr
    210                 215                 220

Glu Val Gly His Ile Ala Gly Met Leu Ala Asn Ala Asp Glu Thr Asp
225                 230                 235                 240

Thr Pro Leu Lys Gln Asn Leu Asp Asn Leu Ser Lys Ile Leu Thr Tyr
                245                 250                 255

Ala Ile Leu Val Ile Ala Ala Val Thr Phe Ala Val Gly Val Phe Leu
            260                 265                 270

Arg Gly Gln His Pro Leu Glu Gly Leu Met Thr Ser Val Ala Leu Ala
        275                 280                 285

Val Ala Ala Ile Pro Glu Gly Leu Pro Ala Ile Val Thr Val Val Leu
    290                 295                 300

Ser Leu Gly Thr Gln Val Leu Ala Lys Arg Asn Ala Ile Ile Arg Lys
305                 310                 315                 320

Leu Pro Ala Val Glu Thr Leu Gly Ser Thr Glu Ile Ile Ala Ser Asp
                325                 330                 335

Lys Thr Gly Thr Leu Thr Met Asn Gln Met Thr Val Glu Lys Val Tyr
            340                 345                 350

Thr Asn Gly Thr Leu Gln Ser Ser Ser Ala Asp Ile Ala Phe Asp Asn
        355                 360                 365

Thr Thr Leu Arg Val Met Asn Phe Ala Asn Asp Thr Lys Val Asp Pro
    370                 375                 380

Ser Gly Lys Leu Ile Gly Asp Pro Thr Glu Thr Ala Leu Val Gln Phe
385                 390                 395                 400

Gly Leu Asp His Asn Phe Asp Val Arg Glu Ala Met Val Ala Glu Pro
                405                 410                 415

Arg Val Ala Glu Leu Pro Phe Asp Ser Asp Arg Lys Leu Met Ser Thr
            420                 425                 430

Ile His Lys Gln Ala Asp Gly Lys Tyr Phe Ile Ala Val Lys Gly Ala
        435                 440                 445

Pro Asp Gln Leu Leu Lys Arg Val Thr Gln Ile Glu Glu Asn Gly Gln
    450                 455                 460

Ile Arg Pro Ile Thr Asp Ala Asp Lys Lys Thr Ile Leu Asp Thr Asn
465                 470                 475                 480

Lys Ser Leu Ala Lys Gln Ala Leu Arg Val Leu Met Met Ala Tyr Lys
                485                 490                 495

Tyr Ser Asp Ala Leu Pro Thr Leu Glu Thr Glu Ile Val Glu Ala Asn
            500                 505                 510

Leu Val Phe Ser Gly Leu Val Gly Met Ile Asp Pro Glu Arg Pro Glu
        515                 520                 525

Ala Ala Gln Ala Val Lys Val Ala Lys Glu Ala Gly Ile Arg Pro Ile
    530                 535                 540

Met Ile Thr Gly Asp His Gln Asp Thr Ala Lys Ala Ile Ala Lys Arg
545                 550                 555                 560

Leu Gly Ile Ile Glu Glu Asp Gly Val Asp His Val Phe Thr Gly Ala
                565                 570                 575

Glu Leu Asn Glu Leu Ser Asp Glu Glu Phe Gln Lys Val Phe Lys Gln
            580                 585                 590

Tyr Ser Val Tyr Ala Arg Val Ser Pro Glu His Lys Val Arg Ile Val
        595                 600                 605

Lys Ala Trp Gln Asn Glu Gly Lys Val Val Gly Met Thr Gly Asp Gly
    610                 615                 620

Val Asn Asp Ala Pro Ser Leu Lys Thr Ala Asp Ile Gly Ile Gly Met
625                 630                 635                 640

Gly Ile Thr Gly Thr Glu Val Ser Lys Gly Ala Ser Asp Met Val Leu
                645                 650                 655

Ala Asp Asp Asn Phe Ala Thr Ile Ile Val Ala Val Glu Glu Gly Arg
            660                 665                 670

Lys Val Phe Ser Asn Ile Gln Lys Thr Ile Gln Tyr Leu Leu Ser Ala
        675                 680                 685

Asn Met Ala Glu Val Phe Thr Ile Phe Leu Ala Thr Leu Phe Gly Trp
    690                 695                 700

Asp Val Leu Gln Pro Val His Leu Leu Trp Ile Asn Leu Val Thr Asp
705                 710                 715                 720

Thr Leu Pro Ala Ile Ala Leu Gly Val Glu Pro Ala Glu Pro Gly Val
                725                 730                 735

Met Lys His Lys Pro Arg Gly Arg Lys Ser Ser Phe Phe Asp Gly Gly
            740                 745                 750

Val Lys Glu Ala Ile Leu Tyr Gln Gly Ala Phe Gln Thr Ile Leu Val
        755                 760                 765

Leu Gly Val Tyr Gly Phe Ala Leu Met Phe Pro Glu His Thr Ser Tyr
    770                 775                 780

His Asp Val His Ala Asp Ala Leu Thr Met Ala Tyr Val Thr Leu Gly
785                 790                 795                 800

Leu Ile Gln Leu Val His Ala Tyr Asn Val Lys Ser Val Tyr Gln Ser
                805                 810                 815

Ile Phe Thr Val Gly Leu Phe Lys Asn Lys Leu Phe Asn Tyr Ser Ile
            820                 825                 830

Pro Val Ala Phe Val Ala Leu Met Ala Thr Val Val Val Pro Gly Phe
        835                 840                 845

Asn Gln Phe Phe His Val Thr His Leu Thr Ile Thr Gln Trp Leu Val
    850                 855                 860

Val Ile Ile Gly Ser Leu Leu Met Val Val Leu Val Glu Leu Val Lys
865                 870                 875                 880

Ala Val Gln Arg Ser Leu Gly Gln Asp Glu Lys Ala Ile
                885                 890

<210>  9
<211>  893
<212>  PRT
<213>  Streptococcus pyogenes

<400>  9

Met Ser Lys Glu Gln Arg His Glu Ala Phe Tyr Thr Gln Ser Glu Glu
1               5                   10                  15

Thr Val Leu Ala Gln Leu Glu Thr Ser Arg Glu Gly Leu Thr Ser Ala
            20                  25                  30

Gln Ala Lys Glu Arg Leu Ala Glu Tyr Gly Arg Asn Glu Leu Asp Glu
        35                  40                  45

Gly Glu Lys Arg Ser Leu Phe Met Lys Phe Leu Asp Gln Phe Lys Asp
    50                  55                  60

Leu Met Ile Ile Ile Leu Ile Val Ala Ala Leu Leu Ser Val Leu Thr
65                  70                  75                  80

Glu Gly Met Glu Gly Leu Thr Asp Ala Ile Ile Ile Leu Ala Val Val
                85                  90                  95

Ile Leu Asn Ala Ala Phe Gly Val Tyr Gln Glu Gly Gln Ala Glu Ala
            100                 105                 110

Ala Ile Glu Ala Leu Lys Ser Met Ser Ser Pro Leu Ala Cys Ile Arg
        115                 120                 125

Arg Asp Gly His Val Thr Glu Ile Asp Ser Lys Glu Leu Val Pro Gly
    130                 135                 140

Asp Ile Val Leu Leu Glu Ala Gly Asp Val Val Pro Ala Asp Leu Arg
145                 150                 155                 160

Leu Leu Glu Ala Asn Ser Leu Lys Ile Glu Glu Ala Ala Leu Thr Gly
                165                 170                 175

Glu Ser Val Pro Val Glu Lys Asp Leu Ser Thr Ala Val Ser Glu Asp
            180                 185                 190

Ala Gly Ile Gly Asp Arg Val Asn Met Gly Tyr Gln Asn Ser Asn Val
        195                 200                 205

Thr Tyr Gly Arg Gly Ile Gly Val Ile Thr Asn Thr Gly Met Tyr Thr
    210                 215                 220

Glu Val Gly His Ile Ala Gly Met Leu Ala Asn Ala Asp Glu Thr Asp
225                 230                 235                 240

Thr Pro Leu Lys Gln Asn Leu Asp Asn Leu Ser Lys Ile Leu Thr Tyr
                245                 250                 255

Ala Ile Leu Val Ile Ala Ala Val Thr Phe Ala Val Gly Val Phe Leu
            260                 265                 270

Arg Gly Gln His Pro Leu Glu Gly Leu Met Thr Ser Val Ala Leu Ala
        275                 280                 285

Val Ala Ala Ile Pro Glu Gly Leu Pro Ala Ile Val Thr Val Val Leu
    290                 295                 300

Ser Leu Gly Thr Gln Val Leu Ala Lys Arg Asn Ala Ile Ile Arg Lys
305                 310                 315                 320

Leu Pro Ala Val Glu Thr Leu Gly Ser Thr Glu Ile Ile Ala Ser Asp
                325                 330                 335

Lys Thr Gly Thr Leu Thr Met Asn Gln Met Thr Val Glu Lys Val Tyr
            340                 345                 350

Thr Asn Gly Thr Leu Gln Ser Ser Ser Ala Asp Ile Ala Phe Asp Asn
        355                 360                 365

Thr Thr Leu Arg Val Met Asn Phe Ala Asn Asp Thr Lys Val Asp Pro
    370                 375                 380

Ser Gly Lys Leu Ile Gly Asp Pro Thr Glu Thr Ala Leu Val Gln Phe
385                 390                 395                 400

Gly Leu Asp His Asn Phe Asp Val Arg Glu Ala Met Val Ala Glu Pro
                405                 410                 415

Arg Val Ala Glu Leu Pro Phe Asp Ser Asp Arg Lys Leu Met Ser Thr
            420                 425                 430

Ile His Lys Gln Ala Asp Gly Lys Tyr Phe Ile Ala Val Lys Gly Ala
        435                 440                 445

Pro Asp Gln Leu Leu Lys Arg Val Thr Gln Ile Glu Glu Asn Gly Gln
    450                 455                 460

Ile Arg Pro Ile Thr Asp Ala Asp Lys Lys Thr Ile Leu Asp Thr Asn
465                 470                 475                 480

Lys Ser Leu Ala Lys Gln Ala Leu Arg Val Leu Met Met Ala Tyr Lys
                485                 490                 495

Tyr Ser Asp Ala Leu Pro Thr Leu Glu Thr Glu Ile Val Glu Ala Asn
            500                 505                 510

Leu Val Phe Ser Gly Leu Val Gly Met Ile Asp Pro Glu Arg Pro Glu
        515                 520                 525

Ala Ala Gln Ala Val Lys Val Ala Lys Glu Ala Gly Ile Arg Pro Ile
    530                 535                 540

Met Ile Thr Gly Asp His Gln Asp Thr Ala Lys Ala Ile Ala Lys Arg
545                 550                 555                 560

Leu Gly Ile Ile Glu Glu Asp Gly Val Asp His Val Phe Thr Gly Ala
                565                 570                 575

Glu Leu Asn Glu Leu Ser Asp Glu Glu Phe Gln Lys Val Phe Lys Gln
            580                 585                 590

Tyr Ser Val Tyr Ala Arg Val Ser Pro Glu His Lys Val Arg Ile Val
        595                 600                 605

Lys Ala Trp Gln Asn Glu Gly Lys Val Val Gly Met Thr Gly Asp Gly
    610                 615                 620

Val Asn Asp Ala Pro Ser Leu Lys Thr Ala Asp Ile Gly Ile Gly Met
625                 630                 635                 640

Gly Ile Thr Gly Thr Glu Val Ser Lys Gly Ala Ser Asp Met Val Leu
                645                 650                 655

Ala Asp Asp Asn Phe Ala Thr Ile Ile Val Ala Val Glu Glu Gly Arg
            660                 665                 670

Lys Val Phe Ser Asn Ile Gln Lys Thr Ile Gln Tyr Leu Leu Ser Ala
        675                 680                 685

Asn Met Ala Glu Val Phe Thr Ile Phe Leu Ala Thr Leu Phe Gly Trp
    690                 695                 700

Asp Val Leu Gln Pro Val His Leu Leu Trp Ile Asn Leu Val Thr Asp
705                 710                 715                 720

Thr Leu Pro Ala Ile Ala Leu Gly Val Glu Pro Ala Glu Pro Gly Val
                725                 730                 735

Met Lys His Lys Pro Arg Gly Arg Lys Ser Ser Phe Phe Asp Gly Gly
            740                 745                 750

Val Lys Glu Ala Ile Leu Tyr Gln Gly Ala Phe Gln Thr Ile Leu Val
        755                 760                 765

Leu Gly Val Tyr Gly Phe Ala Leu Met Phe Pro Glu His Thr Ser Tyr
    770                 775                 780

His Asp Val His Ala Asp Ala Leu Thr Met Ala Tyr Val Thr Leu Gly
785                 790                 795                 800

Leu Ile Gln Leu Val His Ala Tyr Asn Val Lys Ser Val Tyr Gln Ser
                805                 810                 815

Ile Phe Thr Val Gly Leu Phe Lys Asn Lys Leu Phe Asn Tyr Ser Ile
            820                 825                 830

Pro Val Ala Phe Val Ala Leu Met Ala Thr Val Val Val Pro Gly Phe
        835                 840                 845

Asn Gln Phe Phe His Val Thr His Leu Thr Ile Thr Gln Trp Leu Val
    850                 855                 860

Val Ile Ile Gly Ser Leu Leu Met Val Val Leu Val Glu Leu Val Lys
865                 870                 875                 880

Ala Val Gln Arg Ser Leu Gly Gln Asp Glu Lys Ala Ile
                885                 890

<210>  10
<211>  893
<212>  PRT
<213>  Streptococcus pyogenes

<400> 10

Met Ser Lys Glu Gln Arg His Glu Ala Phe Tyr Thr Gln Ser Glu Glu
1               5                   10                  15

Thr Val Leu Ala Gln Leu Glu Thr Ser Arg Glu Gly Leu Thr Ser Ala
            20                  25                  30

Gln Ala Lys Glu Arg Leu Ala Glu Tyr Gly Arg Asn Glu Leu Asp Glu
        35                  40                  45

Gly Glu Lys Arg Ser Leu Phe Met Lys Phe Leu Asp Gln Phe Lys Asp
    50                  55                  60

Leu Met Ile Ile Ile Leu Ile Val Ala Ala Leu Leu Ser Val Leu Thr
65                  70                  75                  80

Glu Gly Met Glu Gly Leu Thr Asp Ala Ile Ile Ile Leu Ala Val Val
                85                  90                  95

Ile Leu Asn Ala Ala Phe Gly Val Tyr Gln Glu Gly Gln Ala Glu Ala
            100                 105                 110

Ala Ile Glu Ala Leu Lys Ser Met Ser Ser Pro Leu Ala Arg Ile Arg
        115                 120                 125

Arg Asp Gly His Val Thr Glu Ile Asp Ser Lys Glu Leu Val Pro Gly
    130                 135                 140

Asp Ile Val Leu Leu Glu Ala Gly Asp Val Val Pro Ala Asp Leu Arg
145                 150                 155                 160

Leu Leu Glu Ala Asn Ser Leu Lys Ile Glu Glu Ala Ala Leu Thr Gly
                165                 170                 175

Glu Ser Val Pro Val Glu Lys Asp Leu Ser Thr Ala Val Ser Glu Asp
            180                 185                 190

Ala Gly Ile Gly Asp Arg Val Asn Met Gly Tyr Gln Asn Ser Asn Val
        195                 200                 205

Thr Tyr Gly Arg Gly Ile Gly Val Ile Thr Asn Thr Gly Met Tyr Thr
    210                 215                 220

Glu Val Gly His Ile Ala Gly Met Leu Ala Asn Ala Asp Glu Thr Asp
225                 230                 235                 240

Thr Pro Leu Lys Gln Asn Leu Asp Asn Leu Ser Lys Ile Leu Thr Tyr
                245                 250                 255

Ala Ile Leu Val Ile Ala Ala Val Thr Phe Ala Val Gly Val Phe Leu

            260                 265                 270

Arg Gly Gln His Pro Leu Glu Gly Leu Met Thr Ser Val Ala Leu Ala
        275                 280                 285

Val Ala Ala Ile Pro Glu Gly Leu Pro Ala Ile Val Thr Val Val Leu
    290                 295                 300

Ser Leu Gly Thr Gln Val Leu Ala Lys Arg Asn Ala Ile Ile Arg Lys
305                 310                 315                 320

Leu Pro Ala Val Glu Thr Leu Gly Ser Thr Glu Ile Ile Ala Ser Asp
                325                 330                 335

Lys Thr Gly Thr Leu Thr Met Asn Gln Met Thr Val Glu Lys Val Tyr
            340                 345                 350

Thr Asn Gly Thr Leu Gln Ser Ser Ser Ala Asp Ile Ala Phe Asp Asn
        355                 360                 365

Thr Thr Leu Arg Val Met Asn Phe Ala Asn Asp Thr Lys Val Asp Pro
    370                 375                 380

Ser Gly Lys Leu Ile Gly Asp Pro Thr Glu Thr Ala Leu Val Gln Phe
385                 390                 395                 400

Gly Leu Asp His Asn Phe Asp Val Arg Glu Ala Met Val Ala Glu Pro
                405                 410                 415

Arg Val Ala Glu Leu Pro Phe Asp Ser Asp Arg Lys Leu Met Ser Thr
            420                 425                 430

Ile His Lys Gln Ala Asp Gly Lys Tyr Phe Ile Ala Val Lys Gly Ala
        435                 440                 445

Pro Asp Gln Leu Leu Lys Arg Val Thr Gln Ile Glu Glu Asn Gly Gln
    450                 455                 460

Ile Arg Pro Ile Thr Asp Ala Asp Lys Lys Thr Ile Leu Asp Thr Asn
465                 470                 475                 480

Lys Ser Leu Ala Lys Gln Ala Leu Arg Val Leu Met Met Ala Tyr Lys
                485                 490                 495

Tyr Ser Asp Ala Leu Pro Thr Leu Glu Thr Glu Ile Val Glu Ala Asn
            500                 505                 510

Leu Val Phe Ser Gly Leu Val Gly Met Ile Asp Pro Glu Arg Pro Glu
        515                 520                 525

Ala Ala Gln Ala Val Lys Val Ala Lys Glu Ala Gly Ile Arg Pro Ile

530                    535                    540

Met Ile Thr Gly Asp His Gln Asp Thr Ala Lys Ala Ile Ala Lys Arg
545                 550                 555                 560

Leu Gly Ile Ile Glu Glu Asp Gly Val Asp His Val Phe Thr Gly Ala
                565                 570                 575

Glu Leu Asn Glu Leu Ser Asp Glu Glu Phe Gln Lys Val Phe Lys Gln
            580                 585                 590

Tyr Ser Val Tyr Ala Arg Val Ser Pro Glu His Lys Val Arg Ile Val
        595                 600                 605

Lys Ala Trp Gln Asn Glu Gly Lys Val Val Ala Met Thr Gly Asp Gly
    610                 615                 620

Val Asn Asp Ala Pro Ser Leu Lys Thr Ala Asp Ile Gly Ile Gly Met
625                 630                 635                 640

Gly Ile Thr Gly Thr Glu Val Ser Lys Gly Ala Ser Asp Met Val Leu
                645                 650                 655

Ala Asp Asp Asn Phe Ala Thr Ile Ile Val Ala Val Glu Glu Gly Arg
            660                 665                 670

Lys Val Phe Ser Asn Ile Gln Lys Thr Ile Gln Tyr Leu Leu Ser Ala
        675                 680                 685

Asn Met Ala Glu Val Phe Thr Ile Phe Leu Ala Thr Leu Phe Gly Trp
    690                 695                 700

Asp Val Leu Gln Pro Val His Leu Leu Trp Ile Asn Leu Val Thr Asp
705                 710                 715                 720

Thr Leu Pro Ala Ile Ala Leu Gly Val Glu Pro Ala Glu Pro Gly Val
                725                 730                 735

Met Lys His Lys Pro Arg Gly Arg Lys Ser Ser Phe Phe Asp Gly Gly
            740                 745                 750

Val Lys Glu Ala Ile Leu Tyr Gln Gly Ala Phe Gln Thr Ile Leu Val
        755                 760                 765

Leu Gly Val Tyr Gly Phe Ala Leu Met Phe Pro Glu His Thr Ser Tyr
    770                 775                 780

His Asp Val His Ala Asp Ala Leu Thr Met Ala Tyr Val Thr Leu Gly
785                 790                 795                 800

Leu Ile Gln Leu Val His Ala Tyr Asn Val Lys Ser Val Tyr Gln Ser

805                         810                         815

Ile Phe Thr Val Gly Leu Phe Lys Asn Lys Leu Phe Asn Tyr Ser Ile
            820                 825                 830

Pro Val Ala Phe Val Ala Leu Met Ala Thr Val Val Val Pro Gly Phe
        835                 840                 845

Asn Gln Phe Phe His Val Thr His Leu Thr Ile Thr Gln Trp Leu Val
    850                 855                 860

Val Ile Ile Gly Ser Leu Leu Met Val Val Leu Val Glu Leu Val Lys
865                 870                 875                 880

Ala Val Gln Arg Ser Leu Gly Gln Asp Glu Lys Ala Ile
                885                 890

<210>  11
<211>  893
<212>  PRT
<213>  Streptococcus pyogenes

<400>  11

Met Ser Lys Glu Gln Arg His Glu Ala Phe Tyr Thr Gln Ser Glu Glu
1               5                   10                  15

Thr Val Leu Ala Gln Leu Glu Thr Ser Arg Glu Gly Leu Thr Ser Ala
            20                  25                  30

Gln Ala Lys Glu Arg Leu Ala Glu Tyr Gly Arg Asn Glu Leu Asp Glu
        35                  40                  45

Gly Glu Lys Arg Ser Leu Phe Met Lys Phe Leu Asp Gln Phe Lys Asp
    50                  55                  60

Leu Met Ile Ile Ile Leu Ile Val Ala Ala Leu Leu Ser Val Leu Thr
65                  70                  75                  80

Glu Gly Met Glu Gly Leu Thr Asp Ala Ile Ile Ile Leu Ala Val Val
                85                  90                  95

Ile Leu Asn Ala Ala Phe Gly Val Tyr Gln Glu Gly Gln Ala Glu Ala
            100                 105                 110

Ala Ile Glu Ala Leu Lys Ser Met Ser Ser Pro Leu Ala Arg Ile Arg
        115                 120                 125

Arg Asp Gly His Val Thr Glu Ile Asp Ser Lys Glu Leu Val Pro Gly
    130                 135                 140

Asp Ile Val Leu Leu Glu Ala Gly Asp Val Val Pro Ala Asp Leu Arg
145                 150                 155                 160

Leu Leu Glu Ala Asn Ser Leu Lys Ile Glu Glu Ala Ala Leu Thr Gly
                165                 170                 175

Glu Ser Val Pro Val Glu Lys Asp Leu Ser Thr Ala Val Ser Glu Asp
            180                 185                 190

Ala Gly Ile Gly Asp Arg Val Asn Met Gly Tyr Gln Asn Ser Asn Val
        195                 200                 205

Thr Tyr Gly Arg Gly Ile Gly Val Ile Thr Asn Thr Gly Met Tyr Thr
    210                 215                 220

Glu Val Gly His Ile Ala Gly Met Leu Ala Asn Ala Asp Glu Thr Asp
225                 230                 235                 240

Thr Pro Leu Lys Gln Asn Leu Asp Asn Leu Ser Lys Ile Leu Thr Tyr
                245                 250                 255

Ala Ile Leu Val Ile Ala Ala Val Thr Phe Ala Val Gly Val Phe Leu
            260                 265                 270

Arg Gly Gln His Pro Leu Glu Gly Leu Met Thr Ser Val Ala Leu Ala
        275                 280                 285

Val Ala Ala Ile Pro Glu Gly Leu Pro Ala Ile Val Thr Val Val Leu
    290                 295                 300

Ser Leu Gly Thr Gln Val Leu Ala Lys Arg Asn Ala Ile Ile Arg Lys
305                 310                 315                 320

Leu Pro Ala Val Glu Thr Leu Gly Ser Thr Glu Ile Ile Ala Ser Asp
                325                 330                 335

Lys Thr Gly Thr Leu Thr Met Asn Gln Met Thr Val Glu Lys Val Tyr
            340                 345                 350

Thr Asn Gly Thr Leu Gln Ser Ser Ser Ala Asp Ile Ala Phe Asp Asn
        355                 360                 365

Thr Thr Leu Arg Val Met Asn Phe Ala Asn Asp Thr Lys Val Asp Pro
    370                 375                 380

Ser Gly Lys Leu Ile Gly Asp Pro Thr Glu Thr Ala Leu Val Gln Phe
385                 390                 395                 400

Gly Leu Asp His Asn Phe Asp Val Arg Glu Ala Met Val Ala Glu Pro
                405                 410                 415

Arg Val Ala Glu Leu Pro Phe Asp Ser Asp Arg Lys Leu Met Ser Thr
            420                 425                 430

Ile His Lys Gln Ala Asp Gly Lys Tyr Phe Ile Ala Val Lys Gly Ala
        435                 440                 445

Pro Asp Gln Leu Leu Lys Arg Val Thr Gln Ile Glu Glu Asn Gly Gln
    450                 455                 460

Ile Arg Pro Ile Thr Asp Ala Asp Lys Lys Thr Ile Leu Asp Thr Asn
465                 470                 475                 480

Lys Ser Leu Ala Lys Gln Ala Leu Arg Val Leu Met Met Ala Tyr Lys
                485                 490                 495

Tyr Ser Asp Ala Leu Pro Thr Leu Glu Thr Glu Ile Val Glu Ala Asn
            500                 505                 510

Leu Val Phe Ser Gly Leu Val Gly Met Ile Asp Pro Glu Arg Pro Glu
        515                 520                 525

Ala Ala Gln Ala Val Lys Val Ala Lys Glu Ala Gly Ile Arg Pro Ile
    530                 535                 540

Met Ile Thr Gly Asp His Gln Asp Thr Ala Lys Ala Ile Ala Lys Arg
545                 550                 555                 560

Leu Gly Ile Ile Glu Glu Asp Gly Val Asp His Val Phe Thr Gly Ala
                565                 570                 575

Glu Leu Asn Glu Leu Ser Asp Glu Glu Phe Gln Lys Val Phe Lys Gln
            580                 585                 590

Tyr Ser Val Tyr Ala Arg Val Ser Pro Glu His Lys Val Arg Ile Val
        595                 600                 605

Lys Ala Trp Gln Asn Glu Gly Lys Val Val Ala Met Thr Gly Asp Gly
    610                 615                 620

Val Asn Asp Ala Pro Ser Leu Lys Thr Ala Asp Ile Gly Ile Gly Met
625                 630                 635                 640

Gly Ile Thr Gly Thr Glu Val Ser Lys Gly Ala Ser Asp Met Val Leu
                645                 650                 655

Ala Asp Asp Asn Phe Ala Thr Ile Ile Val Ala Val Glu Glu Gly Arg
            660                 665                 670

Lys Val Phe Ser Asn Ile Gln Lys Thr Ile Gln Tyr Leu Leu Ser Ala
        675                 680                 685

Asn Met Ala Glu Val Phe Thr Ile Phe Leu Ala Thr Leu Phe Gly Trp
    690                 695                 700

Asp Val Leu Gln Pro Val His Leu Leu Trp Ile Asn Leu Val Thr Asp
705                 710                 715                 720

Thr Leu Pro Ala Ile Ala Leu Gly Val Glu Pro Ala Glu Pro Gly Val
                725                 730                 735

Met Lys His Lys Pro Arg Gly Arg Lys Ser Ser Phe Phe Asp Gly Gly
            740                 745                 750

Val Lys Glu Ala Ile Leu Tyr Gln Gly Ala Phe Gln Thr Ile Leu Val
        755                 760                 765

Leu Gly Val Tyr Gly Phe Ala Leu Met Phe Pro Glu His Thr Ser Tyr
    770                 775                 780

His Asp Val His Ala Asp Ala Leu Thr Met Ala Tyr Val Thr Leu Gly
785                 790                 795                 800

Leu Ile Gln Leu Val His Ala Tyr Asn Val Lys Ser Val Tyr Gln Ser
                805                 810                 815

Ile Phe Thr Val Gly Leu Phe Lys Asn Lys Leu Phe Asn Tyr Ser Ile
            820                 825                 830

Pro Val Ala Phe Val Ala Leu Met Ala Thr Val Val Val Pro Gly Phe
        835                 840                 845

Asn Gln Phe Phe His Val Thr His Leu Thr Ile Thr Gln Trp Leu Val
    850                 855                 860

Val Ile Ile Gly Ser Leu Leu Met Val Val Leu Val Glu Leu Val Lys
865                 870                 875                 880

Ala Val Gln Arg Ser Leu Gly Gln Asp Glu Lys Ala Ile
                885                 890

<210>  12
<211>  893
<212>  PRT
<213>  Streptococcus pyogenes

<400>  12

Met Ser Lys Glu Gln Arg His Glu Ala Phe Tyr Thr Gln Ser Glu Glu
1               5                   10                  15

Thr Val Leu Ala Gln Leu Glu Thr Ser Arg Glu Gly Leu Thr Ser Ala
            20                  25                  30

Gln Ala Lys Glu Arg Leu Ala Glu Tyr Gly Arg Asn Glu Leu Asp Glu
        35                  40                  45

Gly Glu Lys Arg Ser Leu Phe Met Lys Phe Leu Asp Gln Phe Lys Asp
    50                  55                  60

Leu Met Ile Ile Ile Leu Ile Val Ala Ala Leu Leu Ser Val Leu Thr
65                  70                  75                  80

Glu Gly Met Glu Gly Leu Thr Asp Ala Ile Ile Ile Leu Ala Val Val
                85                  90                  95

Ile Leu Asn Ala Ala Phe Gly Val Tyr Gln Glu Gly Gln Ala Glu Ala
            100                 105                 110

Ala Ile Glu Ala Leu Lys Ser Met Ser Ser Pro Leu Ala Arg Ile Arg
        115                 120                 125

Arg Asp Gly His Val Thr Glu Ile Asp Ser Lys Glu Leu Val Pro Gly
    130                 135                 140

Asp Ile Val Leu Leu Glu Ala Gly Asp Val Val Pro Ala Asp Leu Arg
145                 150                 155                 160

Leu Leu Glu Ala Asn Ser Leu Lys Ile Glu Glu Ala Ala Leu Thr Gly
                165                 170                 175

Glu Ser Val Pro Val Glu Lys Asp Leu Ser Thr Ala Val Ser Glu Asp
            180                 185                 190

Ala Gly Ile Gly Asp Arg Val Asn Met Gly Tyr Gln Asn Ser Asn Val
        195                 200                 205

Thr Tyr Gly Arg Gly Ile Gly Val Ile Thr Asn Thr Gly Met Tyr Thr
    210                 215                 220

Glu Val Gly His Ile Ala Gly Met Leu Ala Asn Ala Asp Glu Thr Asp
225                 230                 235                 240

Thr Pro Leu Lys Gln Asn Leu Asp Asn Leu Ser Lys Ile Leu Thr Tyr
                245                 250                 255

Ala Ile Leu Val Ile Ala Ala Val Thr Phe Ala Val Gly Val Phe Leu
            260                 265                 270

Arg Gly Gln His Pro Leu Glu Gly Leu Met Thr Ser Val Ala Leu Ala
        275                 280                 285

Val Ala Ala Ile Pro Glu Gly Leu Pro Ala Ile Val Thr Val Val Leu
    290                 295                 300

Ser Leu Gly Thr Gln Val Leu Ala Lys Arg Asn Ala Ile Ile Arg Lys
305                 310                 315                 320

Leu Pro Ala Val Glu Thr Leu Gly Ser Thr Glu Ile Ile Ala Ser Asp
                325                 330                 335

Lys Thr Gly Thr Leu Thr Met Asn Gln Met Thr Val Glu Lys Val Tyr
            340                 345                 350

Thr Asn Gly Thr Leu Gln Ser Ser Ser Ala Asp Ile Ala Phe Asp Asn
        355                 360                 365

Thr Thr Leu Arg Val Met Asn Phe Ala Asn Asp Thr Lys Val Asp Pro
    370                 375                 380

Ser Gly Lys Leu Ile Gly Asp Pro Thr Glu Thr Ala Leu Val Gln Phe
385                 390                 395                 400

Gly Leu Asp His Asn Phe Asp Val Arg Glu Ala Met Val Ala Glu Pro
                405                 410                 415

Arg Val Ala Glu Leu Pro Phe Asp Ser Asp Arg Lys Leu Met Ser Thr
            420                 425                 430

Ile His Lys Gln Ala Asp Gly Lys Tyr Phe Ile Ala Val Lys Gly Ala
        435                 440                 445

Pro Asp Gln Leu Leu Lys Arg Val Thr Gln Ile Glu Glu Asn Gly Gln
    450                 455                 460

Ile Arg Pro Ile Thr Asp Ala Asp Lys Lys Thr Ile Leu Asp Thr Asn
465                 470                 475                 480

Lys Ser Leu Ala Lys Gln Ala Leu Arg Val Leu Met Met Ala Tyr Lys
                485                 490                 495

Tyr Ser Asp Ala Leu Pro Thr Leu Glu Thr Glu Ile Val Glu Ala Asn
            500                 505                 510

Leu Val Phe Ser Gly Leu Val Gly Met Ile Asp Pro Glu Arg Pro Glu
        515                 520                 525

Ala Ala Gln Ala Val Lys Val Ala Lys Glu Ala Gly Ile Arg Pro Ile
    530                 535                 540

Met Ile Thr Gly Asp His Gln Asp Thr Ala Lys Ala Ile Ala Lys Arg
545                 550                 555                 560

Leu Gly Ile Ile Glu Glu Asp Gly Val Asp His Val Phe Thr Gly Ala
                565                 570                 575

Glu Leu Asn Glu Leu Ser Asp Glu Glu Phe Gln Lys Val Phe Lys Gln
            580                 585                 590

Tyr Ser Val Tyr Ala Arg Val Ser Pro Glu His Lys Val Arg Ile Val
        595                 600                 605

Lys Ala Trp Gln Asn Glu Gly Lys Val Val Ala Met Thr Gly Asp Gly
    610                 615                 620

Val Asn Asp Ala Pro Ser Leu Lys Thr Ala Asp Ile Gly Ile Gly Met
625                 630                 635                 640

Gly Ile Thr Gly Thr Glu Val Ser Lys Gly Ala Ser Asp Met Val Leu
                645                 650                 655

Ala Asp Asp Asn Phe Ala Thr Ile Ile Val Ala Val Glu Glu Gly Arg
            660                 665                 670

Lys Val Phe Ser Asn Ile Gln Lys Thr Ile Gln Tyr Leu Leu Ser Ala
        675                 680                 685

Asn Met Ala Glu Val Phe Thr Ile Phe Leu Ala Thr Leu Phe Gly Trp
    690                 695                 700

Asp Val Leu Gln Pro Val His Leu Leu Trp Ile Asn Leu Val Thr Asp
705                 710                 715                 720

Thr Leu Pro Ala Ile Ala Leu Gly Val Glu Pro Ala Glu Pro Gly Val
                725                 730                 735

Met Lys His Lys Pro Arg Gly Arg Lys Ser Ser Phe Phe Asp Gly Gly
            740                 745                 750

Val Lys Glu Ala Ile Leu Tyr Gln Gly Ala Phe Gln Thr Ile Leu Val
        755                 760                 765

Leu Gly Val Tyr Gly Phe Ala Leu Met Phe Pro Glu His Thr Ser Tyr
    770                 775                 780

His Asp Val His Ala Asp Ala Leu Thr Met Ala Tyr Val Thr Leu Gly
785                 790                 795                 800

Leu Ile Gln Leu Val His Ala Tyr Asn Val Lys Ser Val Tyr Gln Ser
                805                 810                 815

Ile Phe Thr Val Gly Leu Phe Lys Asn Lys Leu Phe Asn Tyr Ser Ile
            820                 825                 830

Pro Val Ala Phe Val Ala Leu Met Ala Thr Val Val Val Pro Gly Phe
        835                 840                 845

Asn Gln Phe Phe His Val Thr His Leu Thr Ile Thr Gln Trp Leu Val
    850                 855                 860

Val Ile Ile Gly Ser Leu Leu Met Val Val Leu Val Glu Leu Val Lys
865                 870                 875                 880

Ala Val Gln Arg Ser Leu Gly Gln Asp Glu Lys Ala Ile
                885                 890

<210>  13
<211>  893
<212>  PRT
<213>  Streptococcus pyogenes

<400>  13

Met Ser Lys Glu Gln Arg His Glu Ala Phe Tyr Thr Gln Ser Glu Glu
1               5                   10                  15

Thr Val Leu Ala Gln Leu Glu Thr Ser Arg Glu Gly Leu Thr Ser Ala
            20                  25                  30

Gln Ala Lys Glu Arg Leu Ala Glu Tyr Gly Arg Asn Glu Leu Asp Glu
        35                  40                  45

Gly Glu Lys Arg Ser Leu Phe Met Lys Phe Leu Asp Gln Phe Lys Asp
    50                  55                  60

Leu Met Ile Ile Ile Leu Ile Val Ala Ala Leu Leu Ser Val Leu Thr
65                  70                  75                  80

Glu Gly Met Glu Gly Leu Thr Asp Ala Ile Ile Ile Leu Ala Val Val
                85                  90                  95

Ile Leu Asn Ala Ala Phe Gly Val Tyr Gln Glu Gly Gln Ala Glu Ala
            100                 105                 110

Ala Ile Glu Ala Leu Lys Ser Met Ser Ser Pro Leu Ala Arg Ile Arg
        115                 120                 125

Arg Asp Gly His Val Thr Glu Ile Asp Ser Lys Glu Leu Val Pro Gly
    130                 135                 140

Asp Ile Val Leu Leu Glu Ala Gly Asp Val Val Pro Ala Asp Leu Arg
145                 150                 155                 160

Leu Leu Glu Ala Asn Ser Leu Lys Ile Glu Glu Ala Ala Leu Thr Gly
                165                 170                 175

Glu Ser Val Pro Val Glu Lys Asp Leu Ser Thr Ala Val Ser Glu Asp
            180                 185                 190

Ala Ser Ile Gly Asp Arg Val Asn Met Gly Tyr Gln Asn Ser Asn Val
        195                 200                 205

Thr Tyr Gly Arg Gly Ile Gly Val Ile Thr Asn Thr Gly Met Tyr Thr
    210                 215                 220

Glu Val Gly His Ile Ala Gly Met Leu Ala Asn Ala Asp Glu Thr Asp
225                 230                 235                 240

Thr Pro Leu Lys Gln Asn Leu Asp Asn Leu Ser Lys Ile Leu Thr Tyr
                245                 250                 255

Ala Ile Leu Val Ile Ala Ala Val Thr Phe Ala Val Gly Val Phe Leu
            260                 265                 270

Arg Gly Gln His Pro Leu Glu Gly Leu Met Thr Ser Val Ala Leu Ala
        275                 280                 285

Val Ala Ala Ile Pro Glu Gly Leu Pro Ala Ile Val Thr Val Val Leu
    290                 295                 300

Ser Leu Gly Thr Gln Val Leu Ala Lys Arg Asn Ala Ile Ile Arg Lys
305                 310                 315                 320

Leu Pro Ala Val Glu Thr Leu Gly Ser Thr Glu Ile Ile Ala Ser Asp
                325                 330                 335

Lys Thr Gly Thr Leu Thr Met Asn Gln Met Thr Val Glu Lys Val Tyr
            340                 345                 350

Thr Asn Gly Thr Leu Gln Ser Ser Ser Ala Asp Ile Ala Phe Asp Asn
        355                 360                 365

Thr Thr Leu Arg Val Met Asn Phe Ala Asn Asp Thr Lys Val Asp Pro
    370                 375                 380

Ser Gly Lys Leu Ile Gly Asp Pro Thr Glu Thr Ala Leu Val Gln Phe
385                 390                 395                 400

Gly Leu Asp His Asn Phe Asp Val Arg Glu Ala Met Val Ala Glu Pro
                405                 410                 415

Arg Val Ala Glu Leu Pro Phe Asp Ser Asp Arg Lys Leu Met Ser Thr
            420                 425                 430

Ile His Lys Gln Ala Asp Gly Lys Tyr Phe Ile Ala Val Lys Gly Ala
        435                 440                 445

Pro Asp Gln Leu Leu Lys Arg Val Thr Gln Ile Glu Glu Asn Gly Gln
    450                 455                 460

Ile Arg Ser Ile Thr Asp Ala Asp Lys Lys Thr Ile Leu Asp Thr Asn
465                 470                 475                 480

Lys Ser Leu Ala Lys Gln Ala Leu Arg Val Leu Met Met Ala Tyr Lys
                485                 490                 495

Tyr Ser Asp Ala Leu Pro Thr Leu Glu Thr Glu Ile Val Glu Ala Asn
            500                 505                 510

Leu Val Phe Ser Gly Leu Val Gly Met Ile Asp Pro Glu Arg Pro Glu
        515                 520                 525

Ala Ala Gln Ala Val Lys Val Ala Lys Glu Ala Gly Ile Arg Pro Ile
    530                 535                 540

Met Ile Thr Gly Asp His Gln Asp Thr Ala Lys Ala Ile Ala Lys Arg
545                 550                 555                 560

Leu Gly Ile Ile Glu Glu Asp Gly Val Asp His Val Phe Thr Gly Ala
                565                 570                 575

Glu Leu Asn Glu Leu Ser Asp Glu Glu Phe Gln Lys Val Phe Lys Gln
            580                 585                 590

Tyr Ser Val Tyr Ala Arg Val Ser Pro Glu His Lys Val Arg Ile Val
        595                 600                 605

Lys Ala Trp Gln Asn Glu Gly Lys Val Val Ala Met Thr Gly Asp Gly
    610                 615                 620

Val Asn Asp Ala Pro Ser Leu Lys Thr Ala Asp Ile Gly Ile Gly Met
625                 630                 635                 640

Gly Ile Thr Gly Thr Glu Val Ser Lys Gly Ala Ser Asp Met Val Leu
                645                 650                 655

Ala Asp Asp Asn Phe Ala Thr Ile Ile Val Ala Val Glu Glu Gly Arg
            660                 665                 670

Lys Val Phe Ser Asn Ile Gln Lys Thr Ile Gln Tyr Leu Leu Ser Ala
        675                 680                 685

Asn Met Ala Glu Val Phe Thr Ile Phe Leu Ala Thr Leu Phe Gly Trp
    690                 695                 700

Asp Val Leu Gln Pro Val His Leu Leu Trp Ile Asn Leu Val Thr Asp
705                 710                 715                 720

Thr Leu Pro Ala Ile Ala Leu Gly Val Glu Pro Ala Glu Pro Gly Val
                725                 730                 735

Met Lys His Lys Pro Arg Gly Arg Lys Ser Ser Phe Phe Asp Gly Gly
            740                 745                 750

Val Lys Glu Ala Ile Leu Tyr Gln Gly Ala Phe Gln Thr Ile Leu Val
        755                 760                 765

Leu Gly Val Tyr Gly Phe Ala Leu Met Phe Pro Glu His Thr Ser Tyr
    770                 775                 780

His Asp Val His Ala Asp Ala Leu Thr Met Ala Tyr Val Thr Leu Gly
785                 790                 795                 800

Leu Ile Gln Leu Val His Ala Tyr Asn Val Lys Ser Val Tyr Gln Ser
                805                 810                 815

Ile Phe Thr Val Gly Leu Phe Lys Asn Lys Leu Phe Asn Tyr Ser Ile
            820                 825                 830

Pro Val Ala Phe Val Ala Leu Met Ala Thr Val Val Val Pro Gly Phe
        835                 840                 845

Asn Gln Phe Phe His Val Thr His Leu Thr Ile Thr Gln Trp Leu Val
    850                 855                 860

Val Ile Ile Gly Ser Leu Leu Met Val Val Leu Val Glu Leu Val Lys
865                 870                 875                 880

Ala Val Gln Arg Ser Leu Gly Gln Asp Glu Lys Ala Ile
                885                 890

<210>  14
<211>  893
<212>  PRT
<213>  Streptococcus pyogenes

<400>  14

Met Ser Lys Glu Gln Arg His Glu Ala Phe Tyr Thr Gln Ser Glu Glu
1               5                   10                  15

Thr Val Leu Ala Gln Leu Glu Thr Ser Arg Glu Gly Leu Thr Ser Ala
            20                  25                  30

Gln Ala Lys Glu Arg Leu Ala Glu Tyr Gly Arg Asn Glu Leu Asp Glu
        35                  40                  45

Gly Glu Lys Arg Ser Leu Phe Met Lys Phe Leu Asp Gln Phe Lys Asp
    50                  55                  60

Leu Met Ile Ile Ile Leu Ile Val Ala Ala Leu Leu Ser Val Leu Thr
65                  70                  75                  80

Glu Gly Met Glu Gly Leu Thr Asp Ala Ile Ile Ile Leu Ala Val Val
                85                  90                  95

Ile Leu Asn Ala Ala Phe Gly Val Tyr Gln Glu Gly Gln Ala Glu Ala

            100                 105                 110

Ala Ile Glu Ala Leu Lys Ser Met Ser Ser Pro Leu Ala Arg Ile Arg
        115                 120                 125

Arg Asp Gly His Val Thr Glu Ile Asp Ser Lys Glu Leu Val Pro Gly
    130                 135                 140

Asp Ile Val Leu Leu Glu Ala Gly Asp Val Val Pro Ala Asp Leu Arg
145                 150                 155                 160

Leu Leu Glu Ala Asn Ser Leu Lys Ile Glu Glu Ala Ala Leu Thr Gly
                165                 170                 175

Glu Ser Val Pro Val Glu Lys Asp Leu Ser Thr Ala Val Ser Lys Asp
            180                 185                 190

Ala Gly Ile Gly Asp Arg Val Asn Met Gly Tyr Gln Asn Ser Asn Val
        195                 200                 205

Thr Tyr Gly Arg Gly Ile Gly Val Ile Thr Asn Thr Gly Met Tyr Thr
    210                 215                 220

Glu Val Gly His Ile Ala Gly Met Leu Ala Asn Ala Asp Glu Thr Asp
225                 230                 235                 240

Thr Pro Leu Lys Gln Asn Leu Asp Asn Leu Ser Lys Ile Leu Thr Tyr
                245                 250                 255

Ala Ile Leu Val Ile Ala Ala Val Thr Phe Ala Val Gly Val Phe Leu
            260                 265                 270

Arg Gly Gln His Pro Leu Glu Gly Leu Met Thr Ser Val Ala Leu Ala
        275                 280                 285

Val Ala Ala Ile Pro Glu Gly Leu Pro Ala Ile Val Thr Val Val Leu
    290                 295                 300

Ser Leu Gly Thr Gln Val Leu Ala Lys Arg Asn Ala Ile Ile Arg Lys
305                 310                 315                 320

Leu Pro Ala Val Glu Thr Leu Gly Ser Thr Glu Ile Ile Ala Ser Asp
                325                 330                 335

Lys Thr Gly Thr Leu Thr Met Asn Gln Met Thr Val Glu Lys Val Tyr
            340                 345                 350

Thr Asn Gly Ala Leu Gln Ser Ser Ser Ala Asp Ile Ala Phe Asp Asn
        355                 360                 365

Thr Thr Leu Arg Val Met Asn Phe Ala Asn Asp Thr Lys Val Asp Pro

370                 375                 380

Ser Gly Lys Leu Ile Gly Asp Pro Thr Glu Thr Ala Leu Val Gln Phe
385                 390                 395                 400

Gly Leu Asp His Asn Phe Asp Val Arg Glu Ala Met Val Ala Glu Pro
                405                 410                 415

Arg Val Ala Glu Leu Pro Phe Asp Ser Asp Arg Lys Leu Met Ser Thr
            420                 425                 430

Ile His Lys Gln Ala Asp Gly Lys Tyr Phe Ile Ala Val Lys Gly Ala
        435                 440                 445

Pro Asp Gln Leu Leu Lys Arg Val Thr Gln Ile Glu Glu Asn Gly Gln
    450                 455                 460

Ile Arg Pro Ile Thr Asp Ala Asp Lys Lys Thr Ile Leu Asp Thr Asn
465                 470                 475                 480

Lys Ser Leu Ala Lys Gln Ala Leu Arg Val Leu Met Met Ala Tyr Lys
                485                 490                 495

Tyr Ser Asp Ala Leu Pro Thr Leu Glu Thr Glu Ile Val Glu Ala Asn
            500                 505                 510

Leu Val Phe Ser Gly Leu Val Gly Met Ile Asp Pro Glu Arg Pro Glu
        515                 520                 525

Ala Ala Gln Ala Val Lys Val Ala Lys Glu Ala Gly Ile Arg Pro Ile
    530                 535                 540

Met Ile Thr Gly Asp His Gln Asp Thr Ala Lys Ala Ile Ala Lys Arg
545                 550                 555                 560

Leu Gly Ile Ile Glu Glu Asp Gly Val Asp His Val Phe Thr Gly Ala
                565                 570                 575

Glu Leu Asn Glu Leu Ser Asp Glu Glu Phe Gln Lys Val Phe Lys Gln
            580                 585                 590

Tyr Ser Val Tyr Ala Arg Val Ser Pro Glu His Lys Val Arg Ile Val
        595                 600                 605

Lys Ala Trp Gln Asn Glu Gly Lys Val Val Ala Met Thr Gly Asp Gly
    610                 615                 620

Val Asn Asp Ala Pro Ser Leu Lys Thr Ala Asp Ile Gly Ile Gly Met
625                 630                 635                 640

Gly Ile Thr Gly Thr Glu Val Ser Lys Gly Ala Ser Asp Met Val Leu

                645                 650                 655

Ala Asp Asp Asn Phe Ala Thr Ile Ile Val Ala Val Glu Glu Gly Arg
            660                 665                 670

Lys Val Phe Ser Asn Ile Gln Lys Thr Ile Gln Tyr Leu Leu Ser Ala
        675                 680                 685

Asn Met Ala Glu Val Phe Thr Ile Phe Leu Ala Thr Leu Phe Gly Trp
    690                 695                 700

Asp Val Leu Gln Pro Val His Leu Leu Trp Ile Asn Leu Val Thr Asp
705                 710                 715                 720

Thr Leu Pro Ala Ile Ala Leu Gly Val Glu Pro Ala Glu Pro Gly Val
                725                 730                 735

Met Lys His Lys Pro Arg Gly Arg Lys Ser Ser Phe Phe Asp Gly Gly
            740                 745                 750

Val Lys Glu Ala Ile Leu Tyr Gln Gly Ala Phe Gln Thr Ile Leu Val
        755                 760                 765

Leu Gly Val Tyr Gly Phe Ala Leu Met Phe Pro Glu His Thr Ser Tyr
    770                 775                 780

His Asp Val His Ala Asp Ala Leu Thr Met Ala Tyr Val Thr Leu Gly
785                 790                 795                 800

Leu Ile Gln Leu Val His Ala Tyr Asn Val Lys Ser Val Tyr Gln Ser
                805                 810                 815

Ile Phe Thr Val Gly Leu Phe Lys Asn Lys Leu Phe Asn Tyr Ser Ile
            820                 825                 830

Pro Val Ala Phe Val Ala Leu Met Ala Thr Val Val Val Pro Gly Phe
        835                 840                 845

Asn Gln Phe Phe His Val Thr His Leu Thr Ile Thr Gln Trp Leu Val
    850                 855                 860

Val Ile Ile Gly Ser Leu Leu Met Val Val Leu Val Glu Leu Val Lys
865                 870                 875                 880

Ala Val Gln Arg Ser Leu Gly Gln Asp Glu Lys Ala Ile
                885                 890

<210> 15
<211> 894
<212> PRT
<213> Streptococcus agalactiae

<400>  15

Met Leu Lys Glu Gln Lys Lys Ser Leu Phe Tyr Thr Gln Gly Gln Glu
1               5                   10                  15

Glu Val Leu Thr Ser Leu Glu Ser Ser Arg Glu Gly Leu Ser Thr Thr
            20                  25                  30

Glu Ala Lys Asn Arg Leu Glu Met Tyr Gly Arg Asn Glu Leu Glu Glu
        35                  40                  45

Gly Lys Lys Arg Ser Leu Ile Ala Lys Phe Phe Asp Gln Phe Lys Asp
    50                  55                  60

Leu Met Ile Ile Ile Leu Leu Val Ala Ala Ala Leu Ser Val Ile Thr
65                  70                  75                  80

Glu Gly Met His Gly Leu Thr Asp Ala Leu Ile Ile Leu Ala Val Val
                85                  90                  95

Ile Leu Asn Ala Ala Phe Gly Val Tyr Gln Glu Gly Gln Ala Glu Ala
            100                 105                 110

Ala Ile Glu Ala Leu Lys Asp Met Ser Ser Pro Ile Ala Arg Val Arg
        115                 120                 125

Arg Asp Gly His Thr Ile Glu Val Asp Ser Lys Glu Leu Val Pro Gly
    130                 135                 140

Asp Leu Val Met Leu Glu Ala Gly Asp Val Val Pro Ala Asp Leu Arg
145                 150                 155                 160

Leu Leu Glu Ala Ala Ser Leu Lys Ile Glu Glu Ala Ala Leu Thr Gly
                165                 170                 175

Glu Ser Val Pro Val Glu Lys Asp Ile Ser Gln Val Val Ala Glu Asp
            180                 185                 190

Ala Gly Ile Gly Asp Arg Val Asn Met Ala Tyr Gln Asn Ser Asn Val
        195                 200                 205

Thr Tyr Gly Arg Gly Tyr Gly Val Val Thr Asn Thr Gly Met Tyr Thr
    210                 215                 220

Glu Val Gly Lys Ile Ala Asp Met Leu Ala Asn Ala Asp Glu Ser Glu
225                 230                 235                 240

Thr Pro Leu Lys Gln Ser Leu Val Gln Leu Ser Lys Leu Leu Thr Tyr
                245                 250                 255

Leu Ile Val Ile Ile Ala Val Ile Thr Phe Leu Val Gly Ile Phe Val
            260                 265                 270

Arg Lys Glu Gly Trp Ile Glu Gly Leu Met Thr Ser Val Ala Leu Ala
        275                 280                 285

Val Ala Ala Ile Pro Glu Gly Leu Pro Ala Ile Val Thr Ile Val Leu
    290                 295                 300

Ser Met Gly Thr Lys Thr Leu Ala Lys Arg Asn Ser Ile Val Arg Lys
305                 310                 315                 320

Leu Pro Ala Val Glu Thr Leu Gly Ser Thr Glu Ile Ile Ala Ser Asp
                325                 330                 335

Lys Thr Gly Thr Leu Thr Met Asn Gln Met Thr Val Glu Lys Val Tyr
            340                 345                 350

Thr Asn Gly Val Leu Gln Ser Ser Ser Glu Glu Ile Ser Val Asp Asn
        355                 360                 365

Asn Thr Leu Arg Ile Met Asn Phe Ser Asn Asp Thr Lys Ile Asp Pro
    370                 375                 380

Ser Gly Lys Leu Ile Gly Asp Pro Thr Glu Thr Ala Leu Val Gln Phe
385                 390                 395                 400

Gly Leu Asp Lys Asn Phe Asp Val Arg Glu Val Leu Lys Asn Glu Pro
                405                 410                 415

Arg Val Ala Glu Leu Pro Phe Asp Ser Asp Arg Lys Leu Met Ser Thr
            420                 425                 430

Ile His Lys Glu Ser Asp Gly Arg Tyr Phe Ile Ala Val Lys Gly Ala
        435                 440                 445

Pro Asp Gln Leu Leu Lys Arg Val Thr Lys Ile Glu Asp Asn Gly Leu
    450                 455                 460

Val Arg Asp Ile Thr Ala Glu Asp Lys Glu Ala Ile Leu Asn Thr Asn
465                 470                 475                 480

Lys Glu Leu Ala Lys Gln Ala Leu Arg Val Leu Met Met Ala Tyr Lys
                485                 490                 495

Tyr Glu Thr Gln Ile Pro Thr Leu Glu Thr Asp Ile Val Glu Ser Asp
            500                 505                 510

Leu Val Phe Ser Gly Leu Val Gly Met Ile Asp Pro Glu Arg Pro Glu
        515                 520                 525

Ala Ala Glu Ala Val Arg Val Ala Lys Glu Ala Gly Ile Arg Pro Ile
    530                 535                 540

Met Ile Thr Gly Asp His Gln Asp Thr Ala Glu Ala Ile Ala Lys Arg
545                 550                 555                 560

Leu Gly Ile Ile Asp Ala Asn Asp Thr Glu Asp His Val Phe Thr Gly
                565                 570                 575

Ala Glu Leu Asn Glu Leu Ser Asp Glu Glu Phe Gln Lys Val Phe Lys
            580                 585                 590

Gln Tyr Ser Val Tyr Ala Arg Val Ser Pro Glu His Lys Val Arg Ile
        595                 600                 605

Val Lys Ala Trp Gln Asn Asp Gly Lys Val Val Ala Met Thr Gly Asp
    610                 615                 620

Gly Val Asn Asp Ala Pro Ser Leu Lys Thr Ala Asp Ile Gly Ile Gly
625                 630                 635                 640

Met Gly Ile Thr Gly Thr Glu Val Ser Lys Gly Ala Ser Asp Met Val
                645                 650                 655

Leu Ala Asp Asp Asn Phe Ala Thr Ile Ile Val Ala Val Glu Glu Gly
            660                 665                 670

Arg Lys Val Phe Ser Asn Ile Gln Lys Ser Ile Gln Tyr Leu Leu Ser
        675                 680                 685

Ala Asn Met Ala Glu Val Phe Thr Ile Phe Phe Ala Thr Leu Leu Gly
    690                 695                 700

Trp Asp Val Leu Ala Pro Val His Leu Leu Trp Ile Asn Leu Val Thr
705                 710                 715                 720

Asp Thr Leu Pro Ala Ile Ala Leu Gly Val Glu Pro Ala Glu Pro Gly
                725                 730                 735

Val Met Thr His Lys Pro Arg Gly Arg Gln Ser Asn Phe Phe Asp Gly
            740                 745                 750

Gly Val Met Gly Ala Ile Ile Tyr Gln Gly Ile Leu Gln Thr Ile Leu
        755                 760                 765

Val Leu Gly Val Tyr Gly Trp Ala Leu Met Tyr Pro Glu His Ala Gly
    770                 775                 780

Tyr Arg Met Ile His Ala Asp Ala Leu Thr Met Ala Phe Ala Thr Leu
785                 790                 795                 800

Gly Leu Ile Gln Leu Val His Ala Phe Asn Val Lys Ser Val Tyr Gln
                805                 810                 815

Ser Ile Phe Thr Val Gly Ala Phe Lys Asn Arg Thr Phe Asn Trp Ser
            820                 825                 830

Ile Pro Val Ala Phe Ile Leu Leu Met Val Thr Ile Val Val Pro Gly
        835                 840                 845

Phe Asn Lys Leu Phe His Val Thr His Leu Ser Ser Thr Gln Trp Leu
    850                 855                 860

Thr Val Val Ile Gly Ser Leu Leu Met Val Val Leu Thr Glu Ile Val
865                 870                 875                 880

Lys Phe Ile Gln Arg Lys Leu Gly Gln Asp Glu Lys Ala Ile
                885                 890

<210>  16
<211>  894
<212>  PRT
<213>  Streptococcus agalactiae

<400>  16

Met Leu Lys Glu Gln Lys Lys Ser Leu Phe Tyr Thr Gln Gly Gln Glu
1               5                   10                  15

Glu Val Leu Thr Ser Leu Glu Ser Ser Arg Glu Gly Leu Ser Thr Thr
            20                  25                  30

Glu Ala Lys Asn Arg Leu Glu Met Tyr Gly Arg Asn Glu Leu Glu Glu
        35                  40                  45

Gly Lys Lys Arg Ser Leu Ile Ala Lys Phe Phe Asp Gln Phe Lys Asp
    50                  55                  60

Leu Met Ile Ile Ile Leu Leu Val Ala Ala Ala Leu Ser Val Ile Thr
65                  70                  75                  80

Glu Gly Met His Gly Leu Thr Asp Ala Leu Ile Ile Leu Ala Val Val
                85                  90                  95

Ile Leu Asn Ala Ala Phe Gly Val Tyr Gln Glu Gly Gln Ala Glu Ala
            100                 105                 110

Ala Ile Glu Ala Leu Lys Asp Met Ser Ser Pro Ile Ala Arg Val Arg
        115                 120                 125

Arg Asp Gly His Thr Ile Glu Val Asp Ser Lys Glu Leu Val Pro Gly
    130                 135                 140

Asp Leu Val Met Leu Glu Ala Gly Asp Val Val Pro Ala Asp Leu Arg
145                 150                 155                 160

Leu Leu Glu Ala Ala Ser Leu Lys Ile Glu Glu Ala Ala Leu Thr Gly
                165                 170                 175

Glu Ser Val Pro Val Glu Lys Asp Ile Ser Gln Val Val Ala Glu Asp
            180                 185                 190

Ala Gly Ile Gly Asp Arg Val Asn Met Ala Tyr Gln Asn Ser Asn Val
        195                 200                 205

Thr Tyr Gly Arg Gly Tyr Gly Val Val Thr Asn Thr Gly Met Tyr Thr
    210                 215                 220

Glu Val Gly Lys Ile Ala Asp Met Leu Ala Asn Ala Asp Glu Ser Glu
225                 230                 235                 240

Thr Pro Leu Lys Gln Ser Leu Val Gln Leu Ser Lys Leu Leu Thr Tyr
                245                 250                 255

Leu Ile Val Ile Ile Ala Val Ile Thr Phe Leu Val Gly Ile Phe Val
            260                 265                 270

Arg Lys Glu Gly Trp Ile Glu Gly Leu Met Thr Ser Val Ala Leu Ala
        275                 280                 285

Val Ala Ala Ile Pro Glu Gly Leu Pro Ala Ile Val Thr Ile Val Leu
    290                 295                 300

Ser Met Gly Thr Lys Thr Leu Ala Lys Arg Asn Ser Ile Val Arg Lys
305                 310                 315                 320

Leu Pro Ala Val Glu Thr Leu Gly Ser Thr Glu Ile Ile Ala Ser Asp
                325                 330                 335

Lys Thr Gly Thr Leu Thr Met Asn Gln Met Thr Val Glu Lys Val Tyr
            340                 345                 350

Thr Asn Gly Val Leu Gln Ser Ser Ser Glu Glu Ile Ser Val Asp Asn
        355                 360                 365

Asn Thr Leu Arg Ile Met Asn Phe Ser Asn Asp Thr Lys Ile Asp Pro
    370                 375                 380

Ser Gly Lys Leu Ile Gly Asp Pro Thr Glu Thr Ala Leu Val Gln Phe
385                 390                 395                 400

Gly Leu Asp Lys Asn Phe Asp Val Arg Glu Val Leu Lys Asn Glu Pro
                405                 410                 415

Arg Val Ala Glu Leu Pro Phe Asp Ser Asp Arg Lys Leu Met Ser Thr
            420                 425                 430

Ile His Lys Glu Ser Asp Gly Arg Tyr Phe Ile Ala Val Lys Gly Ala
        435                 440                 445

Pro Asp Gln Leu Leu Lys Arg Val Thr Lys Ile Glu Asp Asn Gly Leu
    450                 455                 460

Val Arg Asp Ile Thr Ala Glu Asp Lys Glu Ala Ile Leu Asn Thr Asn
465                 470                 475                 480

Lys Glu Leu Ala Lys Gln Ala Leu Arg Val Leu Met Met Ala Tyr Lys
                485                 490                 495

Tyr Glu Thr Gln Ile Pro Thr Leu Glu Thr Asp Ile Val Glu Ser Asp
            500                 505                 510

Leu Val Phe Ser Gly Leu Val Gly Met Ile Asp Pro Glu Arg Pro Glu
        515                 520                 525

Ala Ala Glu Ala Val Arg Val Ala Lys Glu Ala Gly Ile Arg Pro Ile
    530                 535                 540

Met Ile Thr Gly Asp His Gln Asp Thr Ala Glu Ala Ile Ala Lys Arg
545                 550                 555                 560

Leu Gly Ile Ile Asp Ala Asn Asp Thr Glu Asp His Val Phe Thr Gly
                565                 570                 575

Ala Glu Leu Asn Glu Leu Ser Asp Glu Glu Phe Gln Lys Val Phe Lys
            580                 585                 590

Gln Tyr Ser Val Tyr Ala Arg Val Ser Pro Glu His Lys Val Arg Ile
        595                 600                 605

Val Lys Ala Trp Gln Asn Asp Gly Lys Val Val Ala Met Thr Gly Asp
    610                 615                 620

Gly Val Asn Asp Ala Pro Ser Leu Lys Thr Ala Asp Ile Gly Ile Gly
625                 630                 635                 640

Met Gly Ile Thr Gly Thr Glu Val Ser Lys Gly Ala Ser Asp Met Val
                645                 650                 655

Leu Ala Asp Asp Asn Phe Ala Thr Ile Ile Val Ala Val Glu Glu Gly
            660                 665                 670

Arg Lys Val Phe Ser Asn Ile Gln Lys Ser Ile Gln Tyr Leu Leu Ser
        675                 680                 685

Ala Asn Met Ala Glu Val Phe Thr Ile Phe Phe Ala Thr Leu Leu Gly
    690                 695                 700

Trp Asp Val Leu Ala Pro Val His Leu Leu Trp Ile Asn Leu Val Thr
705                 710                 715                 720

Asp Thr Leu Pro Ala Ile Ala Leu Gly Val Glu Pro Ala Glu Pro Gly
                725                 730                 735

Val Met Thr His Lys Pro Arg Gly Arg Gln Ser Asn Phe Phe Asp Gly
            740                 745                 750

Gly Val Met Gly Ala Ile Ile Tyr Gln Gly Ile Leu Gln Thr Ile Leu
        755                 760                 765

Val Leu Gly Val Tyr Gly Trp Ala Leu Met Tyr Pro Glu His Ala Gly
    770                 775                 780

Tyr Arg Met Ile His Ala Asp Ala Leu Thr Met Ala Phe Ala Thr Leu
785                 790                 795                 800

Gly Leu Ile Gln Leu Val His Ala Phe Asn Val Lys Ser Val Tyr Gln
                805                 810                 815

Ser Ile Phe Thr Val Gly Ala Phe Lys Asn Arg Thr Phe Asn Trp Ser
            820                 825                 830

Ile Pro Val Ala Phe Ile Leu Leu Met Val Thr Ile Val Val Pro Gly
        835                 840                 845

Phe Asn Lys Leu Phe His Val Thr His Leu Ser Ser Thr Gln Trp Leu
    850                 855                 860

Thr Val Val Ile Gly Ser Leu Leu Met Val Val Leu Thr Glu Ile Val
865                 870                 875                 880

Lys Phe Ile Gln Arg Lys Leu Gly Gln Asp Glu Lys Ala Ile
                885                 890

<210>  17
<211>  894
<212>  PRT
<213>  Streptococcus agalactiae

<400>  17

Met Leu Lys Glu Gln Lys Lys Ser Leu Phe Tyr Thr Gln Gly Gln Glu
1               5                   10                  15

Glu Val Leu Thr Ser Leu Glu Ser Ser Arg Glu Gly Leu Ser Thr Thr
            20                  25                  30

Glu Ala Lys Asn Arg Leu Glu Met Tyr Gly Arg Asn Glu Leu Glu Glu
        35                  40                  45

Gly Lys Lys Arg Ser Leu Ile Ala Lys Phe Phe Asp Gln Phe Lys Asp
    50                  55                  60

Leu Met Ile Ile Ile Leu Leu Val Ala Ala Ala Leu Ser Val Ile Thr
65                  70                  75                  80

Glu Gly Met His Gly Leu Thr Asp Ala Leu Ile Ile Leu Ala Val Val
                85                  90                  95

Ile Leu Asn Ala Ala Phe Gly Val Tyr Gln Glu Gly Gln Ala Glu Ala
            100                 105                 110

Ala Ile Glu Ala Leu Lys Asp Met Ser Ser Pro Ile Ala Arg Val Arg
        115                 120                 125

Arg Asp Gly His Thr Ile Glu Val Asp Ser Lys Glu Leu Val Pro Gly
    130                 135                 140

Asp Leu Val Met Leu Glu Ala Gly Asp Val Val Pro Ala Asp Leu Arg
145                 150                 155                 160

Leu Leu Glu Ala Ala Ser Leu Lys Ile Glu Glu Ala Ala Leu Thr Gly
                165                 170                 175

Glu Ser Val Pro Val Glu Lys Asp Ile Ser Gln Val Val Ala Glu Asp
            180                 185                 190

Ala Gly Ile Gly Asp Arg Val Asn Met Ala Tyr Gln Asn Ser Asn Val
        195                 200                 205

Thr Tyr Gly Arg Gly Tyr Gly Val Val Thr Asn Thr Gly Met Tyr Thr
    210                 215                 220

Glu Val Gly Lys Ile Ala Asp Met Leu Ala Asn Ala Asp Glu Ser Glu
225                 230                 235                 240

Thr Pro Leu Lys Gln Ser Leu Val Gln Leu Ser Lys Leu Leu Thr Tyr
                245                 250                 255

Leu Ile Val Ile Ile Ala Val Ile Thr Phe Leu Val Gly Ile Phe Val
            260                 265                 270

Arg Lys Glu Gly Trp Ile Glu Gly Leu Met Thr Ser Val Ala Leu Ala
        275                 280                 285

Val Ala Ala Ile Pro Glu Gly Leu Pro Ala Ile Val Thr Ile Val Leu
    290                 295                 300

Ser Met Gly Thr Lys Thr Leu Ala Lys Arg Asn Ser Ile Val Arg Lys
305                 310                 315                 320

Leu Pro Ala Val Glu Thr Leu Gly Ser Thr Glu Ile Ile Ala Ser Asp
                325                 330                 335

Lys Thr Gly Thr Leu Thr Met Asn Gln Met Thr Val Glu Lys Val Tyr
            340                 345                 350

Thr Asn Gly Val Leu Gln Ser Ser Ser Glu Glu Ile Ser Val Asp Asn
        355                 360                 365

Asn Thr Leu Arg Ile Met Asn Phe Ser Asn Asp Thr Lys Ile Asp Pro
    370                 375                 380

Ser Gly Lys Leu Ile Gly Asp Pro Thr Glu Thr Ala Leu Val Gln Phe
385                 390                 395                 400

Gly Leu Asp Lys Asn Phe Asp Val Arg Glu Val Leu Lys Asn Glu Pro
                405                 410                 415

Arg Val Ala Glu Leu Pro Phe Asp Ser Asp Arg Lys Leu Met Ser Thr
            420                 425                 430

Ile His Lys Glu Ser Asp Gly Arg Tyr Phe Ile Ala Val Lys Gly Ala
        435                 440                 445

Pro Asp Gln Leu Leu Lys Arg Val Thr Lys Ile Glu Asp Asn Gly Leu
    450                 455                 460

Val Arg Asp Ile Thr Ala Glu Asp Lys Glu Ala Ile Leu Asn Thr Asn
465                 470                 475                 480

Lys Glu Leu Ala Lys Gln Ala Leu Arg Val Leu Met Met Ala Tyr Lys
                485                 490                 495

Tyr Glu Thr Gln Ile Pro Thr Leu Glu Thr Asp Ile Val Glu Ser Asp
            500                 505                 510

Leu Val Phe Ser Gly Leu Val Gly Met Ile Asp Pro Glu Arg Pro Glu
        515                 520                 525

Ala Ala Glu Ala Val Arg Val Ala Lys Glu Ala Gly Ile Arg Pro Ile
    530                 535                 540

Met Ile Thr Gly Asp His Gln Asp Thr Ala Glu Ala Ile Ala Lys Arg
545                 550                 555                 560

Leu Gly Ile Ile Asp Ala Asn Asp Thr Glu Asp His Val Phe Thr Gly
                565                 570                 575

Ala Glu Leu Asn Glu Leu Ser Asp Glu Glu Phe Gln Lys Val Phe Lys
            580                 585                 590

Gln Tyr Ser Val Tyr Ala Arg Val Ser Pro Glu His Lys Val Arg Ile
        595                 600                 605

Val Lys Ala Trp Gln Asn Asp Gly Lys Val Val Ala Met Thr Gly Asp
    610                 615                 620

Gly Val Asn Asp Ala Pro Ser Leu Lys Thr Ala Asp Ile Gly Ile Gly
625                 630                 635                 640

Met Gly Ile Thr Gly Thr Glu Val Ser Lys Gly Ala Ser Asp Met Val
                645                 650                 655

Leu Ala Asp Asp Asn Phe Ala Thr Ile Ile Val Ala Val Glu Glu Gly
            660                 665                 670

Arg Lys Val Phe Ser Asn Ile Gln Lys Ser Ile Gln Tyr Leu Leu Ser
        675                 680                 685

Ala Asn Met Ala Glu Val Phe Thr Ile Phe Phe Ala Thr Leu Leu Gly
    690                 695                 700

Trp Asp Val Leu Ala Pro Val His Leu Leu Trp Ile Asn Leu Val Thr
705                 710                 715                 720

Asp Thr Leu Pro Ala Ile Ala Leu Gly Val Glu Pro Ala Glu Pro Gly
                725                 730                 735

Val Met Thr His Lys Pro Arg Gly Arg Gln Ser Asn Phe Phe Asp Gly
            740                 745                 750

Gly Val Met Gly Ala Ile Ile Tyr Gln Gly Ile Leu Gln Thr Ile Leu
        755                 760                 765

Val Leu Gly Val Tyr Gly Trp Ala Leu Met Tyr Pro Glu His Ala Gly
    770                 775                 780

Tyr Arg Met Ile His Ala Asp Ala Leu Thr Met Ala Phe Ala Thr Leu
785                 790                 795                 800

Gly Leu Ile Gln Leu Val His Ala Phe Asn Val Lys Ser Val Tyr Gln
                805                 810                 815

Ser Ile Phe Thr Val Gly Ala Phe Lys Asn Arg Thr Phe Asn Trp Ser
            820                 825                 830

Ile Pro Val Ala Phe Ile Leu Leu Met Val Thr Ile Val Val Pro Gly
        835                 840                 845

Phe Asn Lys Leu Phe His Val Thr His Leu Ser Ser Thr Gln Trp Leu
    850                 855                 860

Thr Val Val Ile Gly Ser Leu Leu Met Val Val Leu Thr Glu Ile Val
865                 870                 875                 880

Lys Phe Ile Gln Arg Lys Leu Gly Gln Asp Glu Lys Ala Ile
                885                 890

<210>  18
<211>  894
<212>  PRT
<213>  Streptococcus agalactiae

<400>  18

Met Leu Lys Glu Gln Lys Lys Ser Leu Phe Tyr Thr Gln Gly Gln Glu
1               5                   10                  15

Glu Val Leu Thr Ser Leu Glu Ser Ser Arg Glu Gly Leu Ser Thr Thr
            20                  25                  30

Glu Ala Lys Asn Arg Leu Glu Met Tyr Gly Arg Asn Glu Leu Glu Glu
        35                  40                  45

Gly Lys Lys Arg Ser Leu Ile Ala Lys Phe Phe Asp Gln Phe Lys Asp
    50                  55                  60

Leu Met Ile Ile Ile Leu Leu Val Ala Ala Ala Leu Ser Val Ile Thr
65                  70                  75                  80

Glu Gly Met His Gly Leu Thr Asp Ala Leu Ile Ile Leu Ala Val Val
                85                  90                  95

Ile Leu Asn Ala Ala Phe Gly Val Tyr Gln Glu Gly Gln Ala Glu Ala
            100                 105                 110

Ala Ile Glu Ala Leu Lys Asp Met Ser Ser Pro Ile Ala Arg Val Arg
        115                 120                 125

Arg Asp Gly His Thr Ile Glu Val Asp Ser Lys Glu Leu Val Pro Gly
    130                 135                 140

Asp Leu Val Met Leu Glu Ala Gly Asp Val Val Pro Ala Asp Leu Arg
145                 150                 155                 160

Leu Leu Glu Ala Ala Ser Leu Lys Ile Glu Glu Ala Ala Leu Thr Gly
                165                 170                 175

Glu Ser Val Pro Val Glu Lys Asp Ile Ser Gln Val Val Ala Glu Asp
            180                 185                 190

Ala Gly Ile Gly Asp Arg Val Asn Met Ala Tyr Gln Asn Ser Asn Val
        195                 200                 205

Thr Tyr Gly Arg Gly Tyr Gly Val Val Thr Asn Thr Gly Met Tyr Thr

210                 215                 220

Glu Val Gly Lys Ile Ala Asp Met Leu Ala Asn Ala Asp Glu Ser Glu
225                 230                 235                 240

Thr Pro Leu Lys Gln Ser Leu Val Gln Leu Ser Lys Leu Leu Thr Tyr
                245                 250                 255

Leu Ile Val Ile Ile Ala Val Ile Thr Phe Leu Val Gly Ile Phe Val
            260                 265                 270

Arg Lys Glu Gly Trp Ile Glu Gly Leu Met Thr Ser Val Ala Leu Ala
        275                 280                 285

Val Ala Ala Ile Pro Glu Gly Leu Pro Ala Ile Val Thr Ile Val Leu
    290                 295                 300

Ser Met Gly Thr Lys Thr Leu Ala Lys Arg Asn Ser Ile Val Arg Lys
305                 310                 315                 320

Leu Pro Ala Val Glu Thr Leu Gly Ser Thr Glu Ile Ile Ala Ser Asp
                325                 330                 335

Lys Thr Gly Thr Leu Thr Met Asn Gln Met Thr Val Glu Lys Val Tyr
            340                 345                 350

Thr Asn Gly Val Leu Gln Ser Ser Ser Glu Glu Ile Ser Val Asp Asn
        355                 360                 365

Asn Thr Leu Arg Ile Met Asn Phe Ser Asn Asp Thr Lys Ile Asp Pro
    370                 375                 380

Ser Gly Lys Leu Ile Gly Asp Pro Thr Glu Thr Ala Leu Val Gln Phe
385                 390                 395                 400

Gly Leu Asp Lys Asn Phe Asp Val Arg Glu Val Leu Lys Asn Glu Pro
                405                 410                 415

Arg Val Ala Glu Leu Pro Phe Asp Ser Asp Arg Lys Leu Met Ser Thr
            420                 425                 430

Ile His Lys Glu Ser Asp Gly Arg Tyr Phe Ile Ala Val Lys Gly Ala
        435                 440                 445

Pro Asp Gln Leu Leu Lys Arg Val Thr Lys Ile Glu Asp Asn Gly Leu
    450                 455                 460

Val Arg Asp Ile Thr Ala Glu Asp Lys Glu Ala Ile Leu Asn Thr Asn
465                 470                 475                 480

Lys Glu Leu Ala Lys Gln Ala Leu Arg Val Leu Met Met Ala Tyr Lys

                485                 490                 495

Tyr Glu Thr Gln Ile Pro Thr Leu Glu Thr Asp Ile Val Glu Ser Asp
            500                 505                 510

Leu Val Phe Ser Gly Leu Val Gly Met Ile Asp Pro Glu Arg Pro Glu
        515                 520                 525

Ala Ala Glu Ala Val Arg Val Ala Lys Glu Ala Gly Ile Arg Pro Ile
    530                 535                 540

Met Ile Thr Gly Asp His Gln Asp Thr Ala Glu Ala Ile Ala Lys Arg
545                 550                 555                 560

Leu Gly Ile Ile Asp Ala Asn Asp Thr Glu Asp His Val Phe Thr Gly
                565                 570                 575

Ala Glu Leu Asn Glu Leu Ser Asp Glu Glu Phe Gln Lys Val Phe Lys
            580                 585                 590

Gln Tyr Ser Val Tyr Ala Arg Val Ser Pro Glu His Lys Val Arg Ile
        595                 600                 605

Val Lys Ala Trp Gln Asn Asp Gly Lys Val Val Ala Met Thr Gly Asp
    610                 615                 620

Gly Val Asn Asp Ala Pro Ser Leu Lys Thr Ala Asp Ile Gly Ile Gly
625                 630                 635                 640

Met Gly Ile Thr Gly Thr Glu Val Ser Lys Gly Ala Ser Asp Met Val
                645                 650                 655

Leu Ala Asp Asp Asn Phe Ala Thr Ile Ile Val Ala Val Glu Glu Gly
            660                 665                 670

Arg Lys Val Phe Ser Asn Ile Gln Lys Ser Ile Gln Tyr Leu Leu Ser
        675                 680                 685

Ala Asn Met Ala Glu Val Phe Thr Ile Phe Phe Ala Thr Leu Leu Gly
    690                 695                 700

Trp Asp Val Leu Ala Pro Val His Leu Leu Trp Ile Asn Leu Val Thr
705                 710                 715                 720

Asp Thr Leu Pro Ala Ile Ala Leu Gly Val Glu Pro Ala Glu Pro Gly
                725                 730                 735

Val Met Thr His Lys Pro Arg Gly Arg Gln Ser Asn Phe Phe Asp Gly
            740                 745                 750

Gly Val Met Gly Ala Ile Ile Tyr Gln Gly Ile Leu Gln Thr Ile Leu

        755                 760                 765

Val Leu Gly Val Tyr Gly Trp Ala Leu Met Tyr Pro Glu His Ala Gly
    770                 775                 780

Tyr Arg Met Ile His Ala Asp Ala Leu Thr Met Ala Phe Ala Thr Leu
785                 790                 795                 800

Gly Leu Ile Gln Leu Val His Ala Phe Asn Val Lys Ser Val Tyr Gln
                805                 810                 815

Ser Ile Phe Thr Val Gly Ala Phe Lys Asn Arg Thr Phe Asn Trp Ser
            820                 825                 830

Ile Pro Val Ala Phe Ile Leu Leu Met Val Thr Ile Val Val Pro Gly
        835                 840                 845

Phe Asn Lys Leu Phe His Val Thr His Leu Ser Ser Thr Gln Trp Leu
    850                 855                 860

Thr Val Val Ile Gly Ser Leu Leu Met Val Val Leu Thr Glu Ile Val
865                 870                 875                 880

Lys Phe Ile Gln Arg Lys Leu Gly Gln Asp Glu Lys Ala Ile
                885                 890

<210> 19
<211> 894
<212> PRT
<213> Streptococcus agalactiae

<400> 19

Met Leu Lys Glu Gln Lys Lys Ser Leu Phe Tyr Thr Gln Gly Gln Glu
1               5                   10                  15

Glu Val Leu Thr Ser Leu Glu Ser Ser Arg Glu Gly Leu Ser Thr Thr
            20                  25                  30

Glu Ala Lys Asn Arg Leu Glu Met Tyr Gly Arg Asn Glu Leu Glu Glu
        35                  40                  45

Gly Lys Lys Arg Ser Leu Ile Ala Lys Phe Phe Asp Gln Phe Lys Asp
    50                  55                  60

Leu Met Ile Ile Ile Leu Leu Val Ala Ala Ala Leu Ser Val Ile Thr
65                  70                  75                  80

Glu Gly Met His Gly Leu Thr Asp Ala Leu Ile Ile Leu Ala Val Val
                85                  90                  95

Ile Leu Asn Ala Ala Phe Gly Val Tyr Gln Glu Gly Gln Ala Glu Ala
            100                 105                 110

Ala Ile Glu Ala Leu Lys Asp Met Ser Ser Pro Ile Ala Arg Val Arg
        115                 120                 125

Arg Asp Gly His Thr Ile Glu Val Asp Ser Lys Glu Leu Val Pro Gly
    130                 135                 140

Asp Leu Val Met Leu Glu Ala Gly Asp Val Val Pro Ala Asp Leu Arg
145                 150                 155                 160

Leu Leu Glu Ala Ala Ser Leu Lys Ile Glu Glu Ala Ala Leu Thr Gly
                165                 170                 175

Glu Ser Val Pro Val Glu Lys Asp Ile Ser Gln Val Val Ala Glu Asp
            180                 185                 190

Ala Gly Ile Gly Asp Arg Val Asn Met Ala Tyr Gln Asn Ser Asn Val
        195                 200                 205

Thr Tyr Gly Arg Gly Tyr Gly Val Val Thr Asn Thr Gly Met Tyr Thr
    210                 215                 220

Glu Val Gly Lys Ile Ala Asp Met Leu Ala Asn Ala Asp Glu Ser Glu
225                 230                 235                 240

Thr Pro Leu Lys Gln Ser Leu Val Gln Leu Ser Lys Leu Leu Thr Tyr
                245                 250                 255

Leu Ile Val Ile Ile Ala Val Ile Thr Phe Leu Val Gly Ile Phe Val
            260                 265                 270

Arg Lys Glu Gly Trp Ile Glu Gly Leu Met Thr Ser Val Ala Leu Ala
        275                 280                 285

Val Ala Ala Ile Pro Glu Gly Leu Pro Ala Ile Val Thr Ile Val Leu
    290                 295                 300

Ser Met Gly Thr Lys Thr Leu Ala Lys Arg Asn Ser Ile Val Arg Lys
305                 310                 315                 320

Leu Pro Ala Val Glu Thr Leu Gly Ser Thr Glu Ile Ile Ala Ser Asp
                325                 330                 335

Lys Thr Gly Thr Leu Thr Met Asn Gln Met Thr Val Glu Lys Val Tyr
            340                 345                 350

Thr Asn Gly Val Leu Gln Ser Ser Ser Glu Glu Ile Ser Val Asp Asn
        355                 360                 365

Asn Thr Leu Arg Ile Met Asn Phe Ser Asn Asp Thr Lys Ile Asp Pro
    370                 375                 380

Ser Gly Lys Leu Ile Gly Asp Pro Thr Glu Thr Ala Leu Val Gln Phe
385                 390                 395                 400

Gly Leu Asp Lys Asn Phe Asp Val Arg Glu Val Leu Lys Asn Glu Pro
                405                 410                 415

Arg Val Ala Glu Leu Pro Phe Asp Ser Asp Arg Lys Leu Met Ser Thr
            420                 425                 430

Ile His Lys Glu Ser Asp Gly Arg Tyr Phe Ile Ala Val Lys Gly Ala
        435                 440                 445

Pro Asp Gln Leu Leu Lys Arg Val Thr Lys Ile Glu Asp Asn Gly Leu
    450                 455                 460

Val Arg Asp Ile Thr Ala Glu Asp Lys Glu Ala Ile Leu Asn Thr Asn
465                 470                 475                 480

Lys Glu Leu Ala Lys Gln Ala Leu Arg Val Leu Met Met Ala Tyr Lys
                485                 490                 495

Tyr Glu Thr Gln Ile Pro Ser Leu Glu Thr Asp Ile Val Glu Ser Asp
            500                 505                 510

Leu Val Phe Ser Gly Leu Val Gly Met Ile Asp Pro Glu Arg Pro Glu
        515                 520                 525

Ala Ala Glu Ala Val Arg Val Ala Lys Glu Ala Gly Ile Arg Pro Ile
    530                 535                 540

Met Ile Thr Gly Asp His Gln Asp Thr Ala Glu Ala Ile Ala Lys Arg
545                 550                 555                 560

Leu Gly Ile Ile Asp Ala Asn Asp Thr Glu Asp His Val Phe Thr Gly
                565                 570                 575

Ala Glu Leu Asn Glu Leu Ser Asp Glu Glu Phe Gln Lys Val Phe Lys
            580                 585                 590

Gln Tyr Ser Val Tyr Ala Arg Val Ser Pro Glu His Lys Val Arg Ile
        595                 600                 605

Val Lys Ala Trp Gln Asn Asp Gly Lys Val Val Ala Met Thr Gly Asp
    610                 615                 620

Gly Val Asn Asp Ala Pro Ser Leu Lys Thr Ala Asp Ile Gly Ile Gly
625                 630                 635                 640

Met Gly Ile Thr Gly Thr Glu Val Ser Lys Gly Ala Ser Asp Met Val
                645                 650                 655

Leu Ala Asp Asp Asn Phe Ala Thr Ile Ile Val Ala Val Glu Glu Gly
            660                 665                 670

Arg Lys Val Phe Ser Asn Ile Gln Lys Ser Ile Gln Tyr Leu Leu Ser
        675                 680                 685

Ala Asn Met Ala Glu Val Phe Thr Ile Phe Phe Ala Thr Leu Leu Gly
    690                 695                 700

Trp Asp Val Leu Ala Pro Val His Leu Leu Trp Ile Asn Leu Val Thr
705                 710                 715                 720

Asp Thr Leu Pro Ala Ile Ala Leu Gly Val Glu Pro Ala Glu Pro Gly
                725                 730                 735

Val Met Thr His Lys Pro Arg Gly Arg Gln Ser Asn Phe Phe Asp Gly
            740                 745                 750

Gly Val Met Gly Ala Ile Ile Tyr Gln Gly Ile Leu Gln Thr Ile Leu
        755                 760                 765

Val Leu Gly Val Tyr Gly Trp Ala Leu Met Tyr Pro Glu His Ala Gly
    770                 775                 780

Tyr Arg Met Ile His Ala Asp Ala Leu Thr Met Ala Phe Ala Thr Leu
785                 790                 795                 800

Gly Leu Ile Gln Leu Val His Ala Phe Asn Val Lys Ser Val Tyr Gln
                805                 810                 815

Ser Ile Phe Thr Val Gly Ala Phe Lys Asn Arg Thr Phe Asn Trp Ser
            820                 825                 830

Ile Pro Val Ala Phe Ile Leu Leu Met Val Thr Ile Val Val Pro Gly
        835                 840                 845

Phe Asn Lys Leu Phe His Val Thr His Leu Ser Ser Thr Gln Trp Leu
    850                 855                 860

Thr Val Val Ile Gly Ser Leu Leu Met Val Val Leu Thr Glu Ile Val
865                 870                 875                 880

Lys Phe Ile Gln Arg Lys Leu Gly Gln Asp Glu Lys Ala Ile
                885                 890

<210> 20
<211> 914
<212> PRT
<213> Streptococcus pneumoniae

<400> 20

Met Thr Glu Arg Glu Ser Val Leu His Thr Met Ser Arg Arg Arg His
1               5                   10                  15

Met Ser Lys Glu Gln Lys Arg Gln Ala Phe Tyr Thr Gln Ser Pro Glu
            20                  25                  30

Glu Val Leu Gln Ala Val Asp Ala Thr Glu Gln Gly Leu Ser Ser Ser
        35                  40                  45

Glu Ala Glu Lys Arg Leu Ala Glu Phe Gly His Asn Glu Leu Glu Glu
    50                  55                  60

Gly Glu Lys Arg Ser Ile Leu Val Lys Phe Ile Glu Gln Phe Lys Asp
65                  70                  75                  80

Leu Met Ile Ile Ile Leu Val Ala Ala Ala Ile Leu Ser Val Val Thr
                85                  90                  95

Ser Gly Gly Glu Asp Ile Ala Asp Ala Ile Ile Ile Leu Ala Val Val
            100                 105                 110

Ile Ile Asn Ala Ala Phe Gly Val Tyr Gln Glu Gly Lys Ala Glu Glu
        115                 120                 125

Ala Ile Glu Ala Leu Lys Ser Met Ser Ser Pro Val Ala Arg Val Leu
    130                 135                 140

Arg Asp Gly His Met Ala Glu Ile Asp Ser Lys Glu Leu Val Pro Gly
145                 150                 155                 160

Asp Ile Val Ala Leu Glu Ala Gly Asp Val Val Pro Ala Asp Leu Arg
                165                 170                 175

Leu Ile Glu Ala Asn Ser Leu Lys Ile Glu Glu Ala Ala Leu Thr Gly
            180                 185                 190

Glu Ser Val Pro Val Glu Lys Asp Leu Ser Val Glu Leu Ala Thr Asp
        195                 200                 205

Ala Gly Ile Gly Asp Arg Val Asn Met Ala Phe Gln Asn Ser Asn Val
    210                 215                 220

Thr Tyr Gly Arg Gly Met Gly Val Val Val Asn Thr Gly Met Tyr Thr
225                 230                 235                 240

Glu Val Gly His Ile Ala Gly Met Leu Gln Asp Ala Asp Glu Thr Asp
                245                 250                 255

Thr Pro Leu Lys Gln Asn Leu Asn Asn Leu Ser Lys Val Leu Thr Tyr
            260                 265                 270

Ala Ile Leu Val Ile Ala Leu Val Thr Phe Val Val Gly Val Phe Ile
        275                 280                 285

Gln Gly Lys Asn Pro Leu Gly Glu Leu Leu Thr Ser Val Ala Leu Ala
    290                 295                 300

Val Ala Ala Ile Pro Glu Gly Leu Pro Ala Ile Val Thr Ile Val Leu
305                 310                 315                 320

Ser Leu Gly Thr Gln Val Leu Ala Lys Arg His Ser Ile Val Arg Lys
                325                 330                 335

Leu Pro Ala Val Glu Thr Leu Gly Ser Thr Glu Ile Ile Ala Ser Asp
            340                 345                 350

Lys Thr Gly Thr Leu Thr Met Asn Lys Met Thr Val Glu Lys Val Phe
        355                 360                 365

Tyr Asp Ala Val Leu His Asp Ser Ala Asp Asp Ile Glu Leu Gly Leu
    370                 375                 380

Glu Met Pro Leu Leu Arg Ser Val Val Leu Ala Asn Asp Thr Lys Ile
385                 390                 395                 400

Asp Val Glu Gly Asn Leu Ile Gly Asp Pro Thr Glu Thr Ala Phe Ile
                405                 410                 415

Gln Tyr Ala Leu Asp Lys Gly Tyr Asp Val Lys Gly Phe Leu Glu Lys
            420                 425                 430

Tyr Pro Arg Val Ala Glu Leu Pro Phe Asp Ser Asp Arg Lys Leu Met
        435                 440                 445

Ser Thr Val His Pro Leu Pro Asp Gly Arg Phe Leu Val Ala Val Lys
    450                 455                 460

Gly Ala Pro Asp Gln Leu Leu Lys Arg Cys Leu Leu Arg Asp Lys Ala
465                 470                 475                 480

Gly Asp Ile Ala Pro Ile Asp Glu Lys Val Thr Asn Leu Ile Arg Thr
                485                 490                 495

Asn Asn Ser Glu Met Ala His Gln Ala Leu Arg Val Leu Ala Gly Ala
            500                 505                 510

Tyr Lys Ile Ile Asp Ser Ile Pro Glu Asn Leu Thr Ser Glu Glu Leu
        515                 520                 525

Glu Asn Asp Leu Ile Phe Thr Gly Leu Ile Gly Met Ile Asp Pro Glu
    530                 535                 540

Arg Pro Glu Ala Ala Glu Ala Val Arg Val Ala Lys Glu Ala Gly Ile
545                 550                 555                 560

Arg Pro Ile Met Ile Thr Gly Asp His Gln Asp Thr Ala Glu Ala Ile
                565                 570                 575

Ala Lys Arg Leu Gly Ile Ile Asp Ala Asn Asp Thr Glu Gly His Val
            580                 585                 590

Leu Thr Gly Ala Glu Leu Asn Glu Leu Ser Asp Glu Glu Phe Glu Lys
        595                 600                 605

Val Val Gly Gln Tyr Ser Val Tyr Ala Arg Val Ser Pro Glu His Lys
    610                 615                 620

Val Arg Ile Val Lys Ala Trp Gln Lys Gln Gly Lys Val Val Ala Met
625                 630                 635                 640

Thr Gly Asp Gly Val Asn Asp Ala Pro Ala Leu Lys Thr Ala Asp Ile
                645                 650                 655

Gly Ile Gly Met Gly Ile Thr Gly Thr Glu Val Ser Lys Gly Ala Ser
            660                 665                 670

Asp Met Ile Leu Ala Asp Asp Asn Phe Ala Thr Ile Ile Val Ala Val
        675                 680                 685

Glu Glu Gly Arg Lys Val Phe Ser Asn Ile Gln Lys Thr Ile Gln Tyr
    690                 695                 700

Leu Leu Ser Ala Asn Thr Ala Glu Val Leu Thr Ile Phe Leu Ser Thr
705                 710                 715                 720

Leu Phe Gly Trp Asp Val Leu Gln Pro Val His Leu Leu Trp Ile Asn
                725                 730                 735

Leu Val Thr Asp Thr Phe Pro Ala Ile Ala Leu Gly Val Glu Pro Ala
            740                 745                 750

Glu Pro Gly Val Met Asn His Lys Pro Arg Gly Arg Lys Ala Ser Phe
        755                 760                 765

Phe Ser Gly Gly Val Leu Ser Ser Ile Ile Tyr Gln Gly Val Leu Gln
    770                 775                 780

Ala Ala Leu Val Met Ser Val Tyr Gly Leu Ala Ile Ala Tyr Pro Val
785                 790                 795                 800

His Val Gly Asp Asn His Ala Ile His Ala Asp Ala Leu Thr Met Ala
                805                 810                 815

Phe Ala Thr Leu Gly Leu Ile Gln Leu Phe His Ala Tyr Asn Val Lys
            820                 825                 830

Ser Val Tyr Gln Ser Ile Leu Thr Val Gly Pro Phe Lys Ser Lys Thr
        835                 840                 845

Phe Asn Trp Ser Ile Leu Val Ser Phe Ile Leu Leu Met Ala Thr Ile
    850                 855                 860

Val Val Glu Pro Leu Glu Gly Ile Phe His Val Thr Lys Leu Asp Leu
865                 870                 875                 880

Ser Gln Trp Gly Ile Val Met Ala Gly Ser Phe Ser Met Ile Ile Ile
                885                 890                 895

Val Glu Ile Val Lys Phe Ile Gln Arg Lys Leu Gly Phe Asp Lys Asn
            900                 905                 910

Ala Ile

<210>  21
<211>  914
<212>  PRT
<213>  Streptococcus pneumoniae

<400>  21

Met Thr Glu Arg Glu Ser Val Leu His Thr Met Ser Arg Arg Arg His
1               5                   10                  15

Met Ser Lys Glu Gln Lys Arg Gln Ala Phe Tyr Thr Gln Ser Pro Glu
            20                  25                  30

Glu Val Leu Gln Ala Val Asp Ala Thr Glu Gln Gly Leu Ser Ser Ser
        35                  40                  45

Glu Ala Glu Lys Arg Leu Ala Glu Phe Gly His Asn Glu Leu Glu Glu
    50                  55                  60

Gly Glu Lys Arg Ser Ile Leu Val Lys Phe Ile Glu Gln Phe Lys Asp
65                  70                  75                  80

Leu Met Ile Ile Ile Leu Val Ala Ala Ala Ile Leu Ser Val Val Thr
                85                  90                  95

Ser Gly Gly Glu Asp Ile Ala Asp Ala Ile Ile Ile Leu Ala Val Val
            100                 105                 110

Ile Ile Asn Ala Ala Phe Gly Val Tyr Gln Glu Gly Lys Ala Glu Glu
        115                 120                 125

Ala Ile Glu Ala Leu Lys Ser Met Ser Ser Pro Val Ala Arg Val Leu
    130                 135                 140

Arg Asp Gly His Met Ala Glu Ile Asp Ser Lys Glu Leu Val Pro Gly
145                 150                 155                 160

Asp Ile Val Ala Leu Glu Ala Gly Asp Val Val Pro Ala Asp Leu Arg
                165                 170                 175

Leu Ile Glu Ala Asn Ser Leu Lys Ile Glu Glu Ala Ala Leu Thr Gly
            180                 185                 190

Glu Ser Val Pro Val Glu Lys Asp Leu Ser Val Glu Leu Ala Thr Asp
        195                 200                 205

Ala Gly Ile Gly Asp Arg Val Asn Met Ala Phe Gln Asn Ser Asn Val
    210                 215                 220

Thr Tyr Gly Arg Gly Met Gly Val Val Val Asn Thr Gly Met Tyr Thr
225                 230                 235                 240

Glu Val Gly His Ile Ala Gly Met Leu Gln Asp Ala Asp Glu Thr Asp
                245                 250                 255

Thr Pro Leu Lys Gln Asn Leu Asn Asn Leu Ser Lys Val Leu Thr Tyr
            260                 265                 270

Ala Ile Leu Val Ile Ala Leu Val Thr Phe Val Val Gly Val Phe Ile
        275                 280                 285

Gln Gly Lys Asn Pro Leu Gly Glu Leu Leu Thr Ser Val Ala Leu Ala
    290                 295                 300

Val Ala Ala Ile Pro Glu Gly Leu Pro Ala Ile Val Thr Ile Val Leu
305                 310                 315                 320

Ser Leu Gly Thr Gln Val Leu Ala Lys Arg His Ser Ile Val Arg Lys
                325                 330                 335

Leu Pro Ala Val Glu Thr Leu Gly Ser Thr Glu Ile Ile Ala Ser Asp
            340                 345                 350

Lys Thr Gly Thr Leu Thr Met Asn Lys Met Thr Val Glu Lys Val Phe
        355                 360                 365

Tyr Asp Ala Val Leu His Asp Ser Ala Asp Asp Ile Glu Leu Gly Leu
    370                 375                 380

Glu Met Pro Leu Leu Arg Ser Val Val Leu Ala Asn Asp Thr Lys Ile
385                 390                 395                 400

Asp Val Glu Gly Asn Leu Ile Gly Asp Pro Thr Glu Thr Ala Phe Ile
                405                 410                 415

Gln Tyr Ala Leu Asp Lys Gly Tyr Asp Val Lys Gly Phe Leu Glu Lys
            420                 425                 430

Tyr Pro Arg Val Ala Glu Leu Pro Phe Asp Ser Asp Arg Lys Leu Met
        435                 440                 445

Ser Thr Val His Pro Leu Pro Asp Gly Arg Phe Leu Val Ala Val Lys
    450                 455                 460

Gly Ala Pro Asp Gln Leu Leu Lys Arg Cys Leu Leu Arg Asp Lys Ala
465                 470                 475                 480

Gly Asp Ile Ala Pro Ile Asp Glu Lys Val Thr Asn Leu Ile His Thr
                485                 490                 495

Asn Asn Ser Glu Met Ala His Gln Ala Leu Arg Val Leu Ala Gly Ala
            500                 505                 510

Tyr Lys Ile Ile Asp Ser Ile Pro Glu Asn Leu Thr Ser Glu Glu Leu
        515                 520                 525

Glu Asn Asp Leu Ile Phe Thr Gly Leu Ile Gly Met Ile Asp Pro Glu
    530                 535                 540

Arg Pro Glu Ala Ala Glu Ala Val Arg Val Ala Lys Glu Ala Gly Ile
545                 550                 555                 560

Arg Pro Ile Met Ile Thr Gly Asp His Gln Asp Thr Ala Glu Ala Ile
                565                 570                 575

Ala Lys Arg Leu Gly Ile Ile Asp Ala Asn Asp Thr Glu Gly His Val
            580                 585                 590

Leu Thr Gly Ala Glu Leu Asn Glu Leu Ser Asp Glu Glu Phe Glu Lys
        595                 600                 605

Val Val Gly Gln Tyr Ser Val Tyr Ala Arg Val Ser Pro Glu His Lys
    610                 615                 620

Val Arg Ile Val Lys Ala Trp Gln Lys Gln Gly Lys Val Val Ala Met
625                 630                 635                 640

Thr Gly Asp Gly Val Asn Asp Ala Pro Ala Leu Lys Thr Ala Asp Ile
                645                 650                 655

Gly Ile Gly Met Gly Ile Thr Gly Thr Glu Val Ser Lys Gly Ala Ser
            660                 665                 670

Asp Met Ile Leu Ala Asp Asp Asn Phe Ala Thr Ile Ile Val Ala Val
        675                 680                 685

Glu Glu Gly Arg Lys Val Phe Ser Asn Ile Gln Lys Thr Ile Gln Tyr
    690                 695                 700

Leu Leu Ser Ala Asn Thr Ala Glu Val Leu Thr Ile Phe Leu Ser Thr
705                 710                 715                 720

Leu Phe Gly Trp Asp Val Leu Gln Pro Val His Leu Leu Trp Ile Asn
                725                 730                 735

Leu Val Thr Asp Thr Phe Pro Ala Ile Ala Leu Gly Val Glu Pro Ala
            740                 745                 750

Glu Pro Gly Val Met Asn His Lys Pro Arg Gly Arg Lys Ala Ser Phe
        755                 760                 765

Phe Ser Gly Gly Val Leu Ser Ser Ile Ile Tyr Gln Gly Val Leu Gln
    770                 775                 780

Ala Ala Leu Val Met Ser Val Tyr Gly Leu Ala Ile Ala Tyr Pro Val
785                 790                 795                 800

His Val Gly Asp Asn His Ala Ile His Ala Asp Ala Leu Thr Met Ala
                805                 810                 815

Phe Ala Thr Leu Gly Leu Ile Gln Leu Phe His Ala Tyr Asn Val Lys
            820                 825                 830

Ser Val Tyr Gln Ser Ile Leu Thr Val Gly Pro Phe Lys Ser Lys Thr
        835                 840                 845

Phe Asn Trp Ser Ile Leu Val Ser Phe Ile Leu Leu Met Ala Thr Ile
    850                 855                 860

Val Val Glu Pro Leu Glu Gly Ile Phe His Val Thr Lys Leu Asp Leu
865                 870                 875                 880

Ser Gln Trp Gly Ile Val Met Ala Gly Ser Phe Ser Met Ile Ile Ile
                885                 890                 895

Val Glu Ile Val Lys Phe Ile Gln Arg Lys Leu Gly Phe Asp Lys Asn
            900                 905                 910

Ala Ile


<210>  22
<211>  898
<212>  PRT
<213>  Streptococcus pneumoniae

<400> 22

Met Ser Lys Glu Gln Lys Arg Gln Ala Phe Tyr Thr Gln Ser Pro Glu
1               5                   10                  15

Glu Val Leu Gln Ala Val Asp Ala Thr Glu Gln Gly Leu Ser Ser Ser
            20                  25                  30

Glu Ala Glu Lys Arg Leu Ala Glu Phe Gly His Asn Glu Leu Glu Glu
        35                  40                  45

Gly Glu Lys Arg Ser Ile Leu Val Lys Phe Ile Glu Gln Phe Lys Asp
    50                  55                  60

Leu Met Ile Ile Ile Leu Val Ala Ala Ala Ile Leu Ser Val Val Thr
65                  70                  75                  80

Ser Gly Gly Glu Asp Ile Ala Asp Ala Ile Ile Ile Leu Ala Val Val
                85                  90                  95

Ile Ile Asn Ala Ala Phe Gly Val Tyr Gln Glu Gly Lys Ala Glu Glu
            100                 105                 110

Ala Ile Glu Ala Leu Lys Ser Met Ser Ser Pro Val Ala Arg Val Leu
        115                 120                 125

Arg Asp Gly His Met Ala Glu Ile Asp Ser Lys Glu Leu Val Pro Gly
    130                 135                 140

Asp Ile Val Ala Leu Glu Ala Gly Asp Val Val Pro Ala Asp Leu Arg
145                 150                 155                 160

Leu Ile Glu Ala Asn Ser Leu Lys Ile Glu Glu Ala Ala Leu Thr Gly
                165                 170                 175

Glu Ser Val Pro Val Glu Lys Asp Leu Ser Val Asp Leu Ala Thr Asp
            180                 185                 190

Ala Gly Ile Gly Asp Arg Val Asn Met Ala Phe Gln Asn Ser Asn Val
        195                 200                 205

Thr Tyr Gly Arg Gly Met Gly Val Val Val Asn Thr Gly Met Tyr Thr
    210                 215                 220

Glu Val Gly His Ile Ala Gly Met Leu Gln Asp Ala Asp Glu Thr Asp
225                 230                 235                 240

Thr Pro Leu Lys Gln Asn Leu Asn Asn Leu Ser Lys Val Leu Thr Tyr
                245                 250                 255

Ala Ile Leu Val Ile Ala Leu Val Thr Phe Val Val Gly Val Phe Ile

            260                 265                 270

Gln Gly Lys Asn Pro Leu Gly Glu Leu Leu Thr Ser Val Ala Leu Ala
        275                 280                 285

Val Ala Ala Ile Pro Glu Gly Leu Pro Ala Ile Val Thr Ile Val Leu
    290                 295                 300

Ser Leu Gly Thr Gln Val Leu Ala Lys Arg His Ser Ile Val Arg Lys
305                 310                 315                 320

Leu Pro Ala Val Glu Thr Leu Gly Ser Thr Glu Ile Ile Ala Ser Asp
                325                 330                 335

Lys Thr Gly Thr Leu Thr Met Asn Lys Met Thr Val Glu Lys Val Phe
            340                 345                 350

Tyr Asp Ala Val Leu His Asp Ser Ala Asp Asp Ile Glu Leu Gly Leu
        355                 360                 365

Glu Met Pro Leu Leu Arg Ser Val Val Leu Ala Asn Asp Thr Lys Ile
    370                 375                 380

Asp Val Glu Gly Asn Leu Ile Gly Asp Pro Thr Glu Thr Ala Phe Ile
385                 390                 395                 400

Gln Tyr Ala Leu Asp Lys Gly Tyr Asp Val Lys Gly Phe Leu Glu Lys
                405                 410                 415

Tyr Pro Arg Val Ala Glu Leu Pro Phe Asp Ser Asp Arg Lys Leu Met
            420                 425                 430

Ser Thr Val His Pro Leu Pro Asp Ser Arg Phe Leu Val Ala Val Lys
        435                 440                 445

Gly Ala Pro Asp Gln Leu Leu Lys Arg Cys Leu Leu Arg Asp Lys Ala
    450                 455                 460

Gly Asp Ile Ala Pro Ile Asp Glu Lys Val Thr Asn Leu Ile His Thr
465                 470                 475                 480

Asn Asn Ser Glu Met Ala His Gln Ala Leu Arg Val Leu Ala Gly Ala
                485                 490                 495

Tyr Lys Ile Ile Asp Ser Ile Pro Glu Asn Leu Thr Ser Glu Glu Leu
            500                 505                 510

Glu Asn Asp Leu Ile Phe Thr Gly Leu Ile Gly Met Ile Asp Pro Glu
        515                 520                 525

Arg Pro Glu Ala Ala Glu Ala Val Arg Val Ala Lys Glu Ala Gly Ile

530                     535                     540

Arg Pro Ile Met Ile Thr Gly Asp His Gln Asp Thr Ala Glu Ala Ile
545                     550                     555                     560

Ala Lys Arg Leu Gly Ile Ile Asp Ala Asn Asp Thr Glu Gly His Val
                565                     570                     575

Leu Thr Gly Ala Glu Leu Asn Glu Leu Ser Asp Glu Glu Phe Glu Lys
            580                     585                     590

Val Val Gly Gln Tyr Ser Val Tyr Ala Arg Val Ser Pro Glu His Lys
        595                     600                     605

Val Arg Ile Val Lys Ala Trp Gln Lys Gln Gly Lys Val Val Ala Met
    610                     615                     620

Thr Gly Asp Gly Val Asn Asp Ala Pro Ala Leu Lys Thr Ala Asp Ile
625                     630                     635                     640

Gly Ile Gly Met Gly Ile Thr Gly Thr Glu Val Ser Lys Gly Ala Ser
                645                     650                     655

Asp Met Ile Leu Ala Asp Asp Asn Phe Ala Thr Ile Ile Val Ala Val
            660                     665                     670

Glu Glu Gly Arg Lys Val Phe Ser Asn Ile Gln Lys Thr Ile Gln Tyr
        675                     680                     685

Leu Leu Ser Ala Asn Thr Ala Glu Val Leu Thr Ile Phe Leu Ser Thr
    690                     695                     700

Leu Phe Gly Trp Asp Val Leu Gln Pro Val His Leu Leu Trp Ile Asn
705                     710                     715                     720

Leu Val Thr Asp Thr Phe Pro Ala Ile Ala Leu Gly Val Glu Pro Ala
                725                     730                     735

Glu Pro Gly Val Met Asn His Lys Pro Arg Gly Arg Lys Ala Ser Phe
            740                     745                     750

Phe Ser Gly Gly Val Leu Ser Ser Ile Ile Tyr Gln Gly Val Leu Gln
        755                     760                     765

Ala Ala Leu Val Met Ser Val Tyr Gly Leu Ala Ile Ala Tyr Pro Val
    770                     775                     780

His Val Gly Asp Asn His Ala Ile His Ala Asp Ala Leu Thr Met Ala
785                     790                     795                     800

Phe Ala Thr Leu Gly Leu Ile Gln Leu Phe His Ala Tyr Asn Val Lys

805                 810                 815

Ser Val Tyr Gln Ser Ile Leu Thr Val Gly Pro Phe Lys Ser Lys Thr
            820                 825                 830

Phe Asn Trp Ser Ile Leu Val Ser Phe Ile Leu Leu Met Ala Thr Ile
        835                 840                 845

Val Val Glu Pro Leu Glu Gly Ile Phe His Val Thr Lys Leu Asp Leu
    850                 855                 860

Ser Gln Trp Gly Ile Val Met Ala Gly Ser Phe Ser Met Ile Ile Ile
865                 870                 875                 880

Val Glu Ile Val Lys Phe Ile Gln Arg Lys Leu Gly Phe Asp Lys Asn
                885                 890                 895

Ala Ile

<210>  23
<211>  898
<212>  PRT
<213>  Streptococcus mitis

<400>  23

Met Ser Lys Glu Gln Lys Arg Gln Ala Phe Tyr Thr Gln Ser Pro Glu
1               5                   10                  15

Glu Val Leu Lys Ala Val Asp Ala Thr Glu Gln Gly Leu Ser Ser Ser
            20                  25                  30

Glu Ala Glu Lys Arg Leu Ala Glu Phe Gly His Asn Glu Leu Glu Glu
        35                  40                  45

Gly Glu Lys Arg Ser Ile Leu Val Lys Phe Ile Glu Gln Phe Lys Asp
    50                  55                  60

Leu Met Ile Ile Ile Leu Val Ala Ala Ala Ile Leu Ser Val Val Thr
65                  70                  75                  80

Ser Gly Gly Glu Asp Ile Ala Asp Ala Ile Ile Ile Leu Ala Val Val
                85                  90                  95

Ile Ile Asn Ala Ala Phe Gly Val Tyr Gln Glu Gly Lys Ala Glu Glu
            100                 105                 110

Ala Ile Glu Ala Leu Lys Ser Met Ser Ser Pro Ala Ala Arg Val Leu
        115                 120                 125

Arg Asp Gly His Met Ala Glu Ile Asp Ser Lys Glu Leu Val Pro Gly
    130                 135                 140

Asp Ile Val Ser Leu Glu Ala Gly Asp Val Val Pro Ala Asp Leu Arg
145                 150                 155                 160

Leu Leu Glu Ala Asn Ser Leu Lys Ile Glu Glu Ala Ala Leu Thr Gly
                165                 170                 175

Glu Ser Val Pro Val Glu Lys Asp Leu Thr Val Glu Leu Ala Ala Asp
            180                 185                 190

Ala Gly Ile Gly Asp Arg Val Asn Met Ala Phe Gln Asn Ser Asn Val
        195                 200                 205

Thr Tyr Gly Arg Gly Met Gly Val Val Val Asn Thr Gly Met Tyr Thr
    210                 215                 220

Glu Val Gly His Ile Ala Gly Met Leu Gln Asp Ala Asp Glu Thr Asp
225                 230                 235                 240

Thr Pro Leu Lys Gln Asn Leu Asn Asn Leu Ser Lys Val Leu Thr Tyr
                245                 250                 255

Ala Ile Leu Val Ile Ala Leu Val Thr Phe Val Val Gly Val Phe Ile
            260                 265                 270

Gln Gly Lys Asn Pro Leu Gly Glu Leu Met Thr Ser Val Ala Leu Ala
        275                 280                 285

Val Ala Ala Ile Pro Glu Gly Leu Pro Ala Ile Val Thr Ile Val Leu
    290                 295                 300

Ala Leu Gly Thr Gln Val Leu Ala Lys Arg Asn Ser Ile Val Arg Lys
305                 310                 315                 320

Leu Pro Ala Val Glu Thr Leu Gly Ser Thr Glu Ile Ile Ala Ser Asp
                325                 330                 335

Lys Thr Gly Thr Leu Thr Met Asn Lys Met Thr Val Glu Lys Val Phe
            340                 345                 350

Tyr Asp Ala Ile Leu His Asp Ser Ala Asp Asp Ile Glu Leu Gly Leu
        355                 360                 365

Glu Met Pro Leu Leu Arg Ser Val Val Leu Ala Asn Asp Thr Lys Ile
    370                 375                 380

Asp Val Glu Gly Asn Leu Ile Gly Asp Pro Thr Glu Thr Ala Phe Ile
385                 390                 395                 400

Gln Tyr Ala Leu Asp Lys Gly Tyr Asp Val Lys Gly Phe Leu Asp Lys
                405                 410                 415

Tyr Pro Arg Val Ala Glu Leu Pro Phe Asp Ser Asp Arg Lys Leu Met
            420                 425                 430

Ser Thr Val His Pro Leu Pro Asp Gly Lys Phe Leu Val Ala Val Lys
        435                 440                 445

Gly Ala Pro Asp Gln Leu Leu Lys Arg Cys Val Leu Arg Asp Lys Ala
    450                 455                 460

Gly Asp Ile Ala Pro Ile Asp Glu Lys Val Thr Asn Leu Ile His Thr
465                 470                 475                 480

Asn Asn Ser Glu Met Ala His Gln Ala Leu Arg Val Leu Ala Gly Ala
                485                 490                 495

Tyr Lys Ile Ile Asp Ser Ile Pro Glu Asn Leu Thr Ser Glu Glu Leu
            500                 505                 510

Glu Asn Asp Leu Ile Phe Thr Gly Leu Ile Gly Met Ile Asp Pro Glu
        515                 520                 525

Arg Pro Glu Ala Ala Glu Ala Val Arg Val Ala Lys Glu Ala Gly Ile
    530                 535                 540

Arg Pro Ile Met Ile Thr Gly Asp His Gln Asp Thr Ala Glu Ala Ile
545                 550                 555                 560

Ala Lys Arg Leu Gly Ile Ile Asp Ala Asn Asp Thr Glu Gly His Val
                565                 570                 575

Leu Thr Gly Ala Glu Leu Asn Glu Leu Ser Asp Glu Asp Phe Glu Lys
            580                 585                 590

Val Val Gly Gln Tyr Ser Val Tyr Ala Arg Val Ser Pro Glu His Lys
        595                 600                 605

Val Arg Ile Val Lys Ala Trp Gln Lys Gln Gly Lys Val Val Ala Met
    610                 615                 620

Thr Gly Asp Gly Val Asn Asp Ala Pro Ala Leu Lys Thr Ala Asp Ile
625                 630                 635                 640

Gly Ile Gly Met Gly Ile Thr Gly Thr Glu Val Ser Lys Gly Ala Ser
                645                 650                 655

Asp Met Ile Leu Ala Asp Asp Asn Phe Ala Thr Ile Ile Val Ala Val
            660                 665                 670

Glu Glu Gly Arg Lys Val Phe Ser Asn Ile Gln Lys Thr Ile Gln Tyr
        675                 680                 685

Leu Leu Ser Ala Asn Thr Ala Glu Val Leu Thr Ile Phe Leu Ser Thr
    690                 695                 700

Leu Phe Gly Trp Asp Val Leu Gln Pro Val His Leu Leu Trp Ile Asn
705                 710                 715                 720

Leu Val Thr Asp Thr Phe Pro Ala Ile Ala Leu Gly Val Glu Pro Ala
                725                 730                 735

Glu Pro Gly Val Met Asn His Lys Pro Arg Gly Arg Lys Ala Ser Phe
            740                 745                 750

Phe Ser Gly Gly Val Val Ser Ser Ile Ile Tyr Gln Gly Val Leu Gln
        755                 760                 765

Ala Ala Ile Val Met Ser Val Tyr Gly Leu Ala Ile Ala Tyr Pro Val
    770                 775                 780

His Val Gly Asp Asn His Ala Ile His Ala Asp Ala Leu Thr Met Ala
785                 790                 795                 800

Phe Ala Thr Leu Gly Leu Ile Gln Leu Phe His Ala Tyr Asn Val Lys
                805                 810                 815

Ser Val Tyr Gln Ser Ile Leu Thr Val Gly Pro Phe Lys Ser Lys Thr
            820                 825                 830

Phe Asn Trp Ser Ile Leu Val Ser Phe Ile Leu Leu Met Ala Thr Ile
        835                 840                 845

Val Val Glu Pro Leu Glu Gly Ile Phe His Val Thr Lys Leu Asp Leu
    850                 855                 860

Ser Gln Trp Gly Ile Val Met Gly Gly Ser Phe Ser Met Ile Ile Ile
865                 870                 875                 880

Val Glu Ile Val Lys Phe Ile Gln Arg Lys Leu Gly Phe Asp Lys Asn
                885                 890                 895

Ala Ile


<210>  24
<211>  7
<212>  PRT
<213>  Artificial

<220>
<223>  mgtA domain


<220>

<221>  VARIANT
<222>  (6)..(6)
<223>  I, V, M

<220>
<221>  VARIANT
<222>  (7)..(7)
<223>  I

<400>  24

Asp Lys Thr Gly Thr Leu Thr
1               5


<210>  25
<211>  23
<212>  DNA
<213>  Artificial

<220>
<223>  primer Dcap.control.fw

<400>  25
ggcagacaga gttaggagtt gag                                              23


<210>  26
<211>  19
<212>  DNA
<213>  Artificial

<220>
<223>  primer Dcap.control.rev

<400>  26
cccagtcttc gccagcagc                                                   19


<210>  27
<211>  20
<212>  DNA
<213>  Artificial

<220>
<223>  primer Kana.1

<400>  27
gtgaattgga gttcgtcttg                                                  20


<210>  28
<211>  20
<212>  DNA
<213>  Artificial

<220>
<223>  primer Kana.2

<400>  28
tatggacagt tgcggatgta                                                  20


<210>  29
<211>  28
<212>  DNA
<213>  Artificial

<220>
<223>  primer bgaA-1

<400>  29
gctctagaca aatcgttgaa cgaggtgg                                          28


<210>  30
<211>  28
<212>  DNA
<213>  Artificial

<220>
<223>  primer bgaA-4

<400>  30
gaagatctgt aatttgatag tcttgacg                                          28


<210>  31
<211>  20
<212>  DNA
<213>  Artificial

<220>
<223>  primer Sp1550for

<400>  31
gcacctgctt gccagaatag                                                   20


<210>  32
<211>  40
<212>  DNA
<213>  Artificial

<220>
<223>  primer Sp1550rev

<400>  32
agttatcggc ataatcgtta tcggatgtca gcatatcaat                             40


<210>  33
<211>  40
<212>  DNA
<213>  Artificial

<220>
<223>  primer Sp1552for

<400>  33
gagatctaat cgatgcatgc cggaatgacg acactatcac                             40


<210>  34
<211>  20
<212>  DNA
<213>  Artificial

<220>
<223>  primer Sp1552rev

<400>  34
gcaccaagag gatcaatgac                                                   20


<210>  35

<211>  20
<212>  DNA
<213>  Artificial

<220>
<223>  primer Eryfor

<400>  35
taacgattat gccgataact                                                    20


<210>  36
<211>  20
<212>  DNA
<213>  Artificial

<220>
<223>  primer Eryrev

<400>  36
gcatgcatcg attagatctc                                                    20


<210>  37
<211>  31
<212>  DNA
<213>  Artificial

<220>
<223>  primer mgtacomplsta

<400>  37
catgccatgg cagaaagaga gtctgttttg c                                       31


<210>  38
<211>  24
<212>  DNA
<213>  Artificial

<220>
<223>  primer mgtAstop

<400>  38
tctagacctt aaatcgcatt cttg                                               24

## Description

### FIELD OF THE INVENTION

The present invention is in the field of prophylactic or therapeutic treatment of an infectious diseases, in particular of infections by a meningeal pathogen. The invention further relates to vaccine formulations and pharmaceutical compositions for use in such methods as described in more defined herein below.

### BACKGROUND OF THE INVENTION

Infection with the Gram-positive bacterium *Streptococcus pneumoniae* is a common cause of respiratory disease and serious invasive diseases such as pneumonia, sepsis and meningitis. Especially infants, the elderly and immuno-compromised individuals are at risk. Annually, over 1 million children die of pneumonia and meningitis caused by *Haemophilus influenza* (Gram negative), *Neisseria meningitides* (Gram negative) or *S. pneumoniae (Gram positive),* and in the US alone 40.000 of these are caused by pneumonia or meningitis caused by *S. pneumoniae.* In addition, this pneumonococcal meningitis has a high mortality and morbidity rate compared to other pathogenic bacteria.

The pathway of infection for any etiological agent of meningitis begins with the establishment of localized infection in the host. Most meningeal pathogens are transmitted via the respiratory route. The pathogen must then evade host immune responses and enter the submucosa. Finally, the infectious agent must cross the blood-brain barrier to cause meningitis and access the cerebrospinal fluid (CSF). This is most often accomplished by invasion of the bloodstream and subsequent seeding of the central nervous system (CNS).

The integrity of body compartments is maintained in part by boundaries of epithelial and endothelial cells. For instance, microvascular endothelial cells form the major substrate of the blood brain barrier and *S*. *pneumoniae* can only cause meningitis if it traverses the microvascular endothelial cells. Thus, in order to become invasive, *S. pneumoniae* has to cross epithelial or endothelial cell layers, which occurs through a specific and receptor mediated processes called transcytosis (See below).

The specific receptor for *S. pneumoniae* on epithelial cells is the polymeric immunoglobulin receptor (pIgR) and on endothelial cells it is the platelet activating factor receptor (PAFR). Invasion takes place after binding to the receptors, when the bacteria are engulfed by either cell type and end up in a vesicle inside the eukaryotic cells. Generally, as soon as the vesicles are formed, conditions within change rapidly as divalent cations and other nutrients are pumped out. The vesicles are either recycled back to the cell surface or fuse with the lysosome, after which noxious substances are released that destroy the contents. However, not all vesicles containing *S. pneumoniae* fuse with the lysosome but most are circulated for some time within the cell and eventually transported to the other side of the cells where the contents are released. In this way the bacteria are transcytosed and have become invasive.

Adhesion to eukaryotic cells and interaction with the receptors is mediated primarily by phosphorylcholine and the cholin binding protein A (CbpA). However, virtually nothing is known about which genes of *S. pneumoniae* play a role in the invasion, let alone during transcytosis. Without wishing to be bound by any theory, it is believed that - although the bacteria do not replicate within the eukaryotic cells - they have an active response to ensure that they survive the conditions within the vesicles and are transcytosed. *S. pneumoniae* strains that are more likely to cause invasive disease differ from others, which strongly suggests that there are specific genetic determinants that predispose to transcytosis. In addition, there are indications that invasion of eukaryotic cells by related streptococcal species requires an active response. Altogether, this strongly suggests that *S*. *pneumoniae* has an active response to the translocation of endothelial cells and that its disruption will cripple the ability of *S. pneumoniae* to cause serious invasive disease.

A significant stress encountered by *S. pneumoniae* within the endothelial cell vesicles is probably Mg²⁺ limitation. Conditions within eukaryotic vesicles are not precisely known, but several bacteria experience Mg²⁺ limitation inside eukaryotic cells and induce the expression of Mg²⁺ transporters. More importantly, mutants in Mg²⁺ homeostasis genes in a wide variety of bacteria are less virulent and impaired in their ability to survive within eukaryotic cells. Several transporters are involved in Mg²⁺ homeostasis and their induction upon Mg²⁺ limitation is under complex regulatory control. During transcytosis, *S. pneumoniae* has to survive inside the endosomal vesicles, which qualifies as a low Mg²⁺ environment. Therefore it is conceivable that Mg²⁺ uptake is essential for this process. The response to Mg²⁺ limitation is important for virulence as mutation of the involved genes almost invariably leads to virulence defects and specifically to an inability to survive in eukaryotic cells. In Salmonella and other bacteria, several transporters are involved in Mg2+ homeostasis. One is the primary transporter CorA, which is thought to be a constitutively active pore in the membrane and whose expression is not regulated. In contrast, the ABC transporters MgtA and MgtB are induced upon encountering Mg²⁺ limiting conditions and import Mg²⁺ against the concentration gradient. Of particular interest is MgtC, a small membrane protein, whose function is unknown but is involved in Mg²⁺ homeostasis and has an essential role in virulence and intracellular survival in Salmonella and other bacteria. The induction upon Mg²⁺ limitation of *mgtA, mgtB* and *mgtC* genes is under complex regulatory control of several transcription factors that respond to the host environment. Analysis of the published *S. pneumoniae* genomes revealed that they contain homologues of these transporters. In this study we examined the role of one of these transporters in *S. pneumoniae* Mg²⁺ homeostasis and in the interaction of the bacteria and Human Brain Microvascular Endothelial Cells (HBMEC).

### SUMMARY OF THE INVENTION

The present inventors have now found that the *mgtA* gene encodes the main Mg²⁺ uptake mechanism in *S. pneumoniae,* have shown that this gene product plays a role in adhesion and invasion, and further have shown that expression of this gene is essential for normal growth and transcytosis of the cells, i.e. for the traversal of the endothelial cell layer. Predictive analysis shows that the *mgtA* gene product, the MgtA protein, is a membrane protein. This means that MgtA is immunogenic and accessible for antibodies and thus suitable for use as a vaccine. Also, it has been discovered that antibodies against MgtA recognize the bacteria and will protect the host against infection, either directly through immune clearance, or indirectly by blocking the activity of the protein, thereby inhibiting the growth of the bacteria. As a secondary effect, blocking the function of the protein reduces the adhesion abilities of the cells which reduces colonisation and invasion. Further, blocking of the activity will inhibit the ability of the bacteria to transcytosis and thus to cause invasive disease. The invention thus relates to MgtA-based vaccines as well as to anti-MgtA antibodies, both of which may be used in prophylactic or therapeutic treatment of an infectious diseases, in particular of infections by a meningeal pathogen, wherein the MgtA is derived from *S. pneumoniae* or homologs thereof.

In a first aspect, the present invention relates to a vaccine formulation providing protection against infection of a subject by a meningeal pathogen, said formulation comprising an effective amount of the protein MgtA or an immunogenic part thereof together with at least one of a pharmaceutically acceptable diluent, carrier, excipient or adjuvant therefore.

In a preferred embodiment, the immunogenic part is an antigenic determinant of the pathogen.

In another preferred embodiment, the formulation comprises an essentially cell-free preparation of said pathogen comprising said protein or said part thereof.

In another preferred embodiment, the immunogenic part of the protein MgtA comprises a part that is exposed on the membrane of said pathogen.

In still another preferred embodiment, the vaccine provides protections against meningitis, pneumonia, and/or sepsis caused by a meningeal pathogen. In a particularly preferred embodiment, the vaccine provides protections against meningeal streptococci, preferably selected form the group consisting of β-haemolytic *Streptococcus* spp. belonging to Lancefield group C, *S. acidominimus, S agalactiae, S. bovis, S. constellatus, S. equinus, S. equisimilis, S. mitis, S. oralis, S. pneumoniae, S. pyogenes, S. salivarius, S. sanguis, S. suis* and *S. uberis,* more preferably selected form the group consisting of *S agalactiae, S. pneumoniae, S. pyogenes, S. suis* and *S. uberis.*

In another aspect, the present invention relates to the protein MgtA or an immunogenic part thereof, for use as a vaccine.

In yet another aspect, the present invention relates to an anti-MgtA antibody or fragment thereof, preferably a humanized anti-MgtA antibody or fragment thereof.

In still another aspect, the present invention relates to an anti-MgtA antibody or fragment thereof, preferably a humanized anti-MgtA antibody or fragment thereof, for use as a medicament for the prophylactic or therapeutic treatment of an infection by a meningeal pathogen in a subject.

In still another aspect, the present invention relates to the use of anti-MgtA antibody or fragment thereof, preferably a humanized anti-MgtA antibody or fragment thereof, for the manufacture of a medicament for the prophylactic or therapeutic treatment of an infection by a meningeal pathogen in a subject.

In a further aspect, the present invention provides a pharmaceutical composition comprising an anti-MgtA antibody or fragment thereof, preferably a humanized anti-MgtA antibody or fragment thereof, and a pharmaceutically acceptable carrier.

In still a further aspect, the present invention provides a method for prophylactic or therapeutic treatment of an infection by a meningeal pathogen in a subject comprising administering to a subject in need of such treatment an effective amount of a vaccine formulation according to the invention and/or an effective amount of a pharmaceutical composition as defined herein above.

In still a further aspect, the present invention provides a method for preparing an MgtA-based vaccine formulation, the said method comprising bringing into association, an effective amount of the protein MgtA or an immunogenic part thereof and at least one of a pharmaceutically acceptable diluent, carrier, excipient or adjuvant therefore.

In still a further aspect, the present invention provides a method for preparing a pharmaceutical composition as defined herein above, the said method comprising bringing into association, an effective amount of an anti-MgtA antibody, preferably a humanized anti-MgtA antibody, or fragment thereof, and a pharmaceutically acceptable carrier.

### DESCRIPTION OF THE DRAWINGS

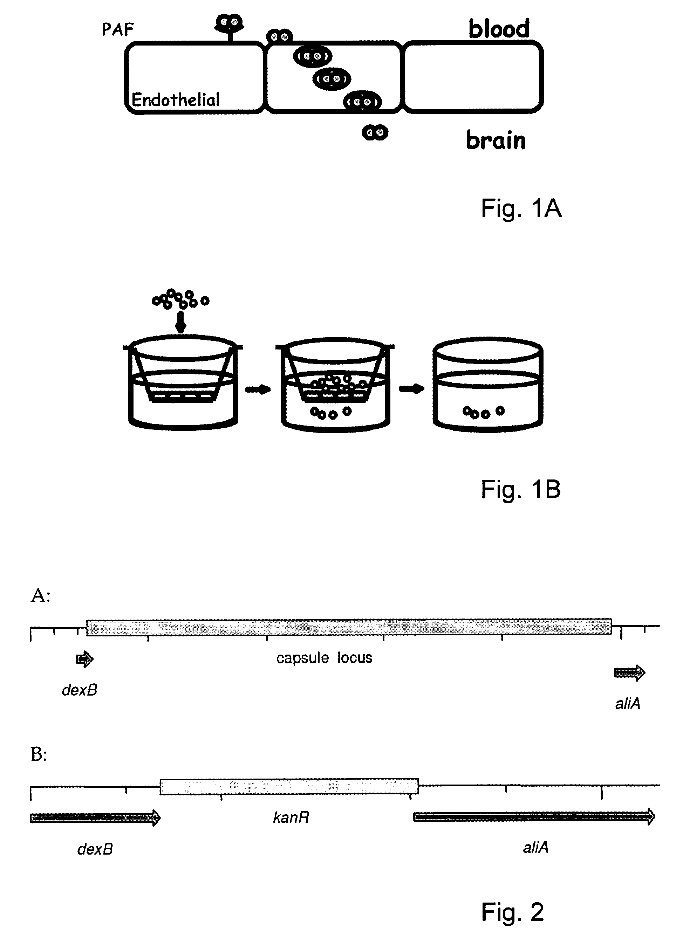

Figure 1 exemplifies the transcytosis process. A: Transcytosis is a specific and receptor mediated process. B: The transcytosis system. Endothelial cells (blue rectangles) are grown to a confluent monolayer on a membrane (Transwell® permeable supports, Corning Incorporated, Corning New York). Only bacteria that are able to transcytose are able to reach the lower compartment. Subsequently, the membrane with the cells and non-transcytosed bacteria will be removed and the amount of translocated bacteria can be analyzed.
Figure 2 displays the chromosomal organization of D39 and D39Δ*cps*. A: Chromosomal organization of D39 before integration of kanR in the capsule locus. B: Chromosomal organization of D39 after integration of kanR in the capsule locus. kanR: kanamycin resistance gene.

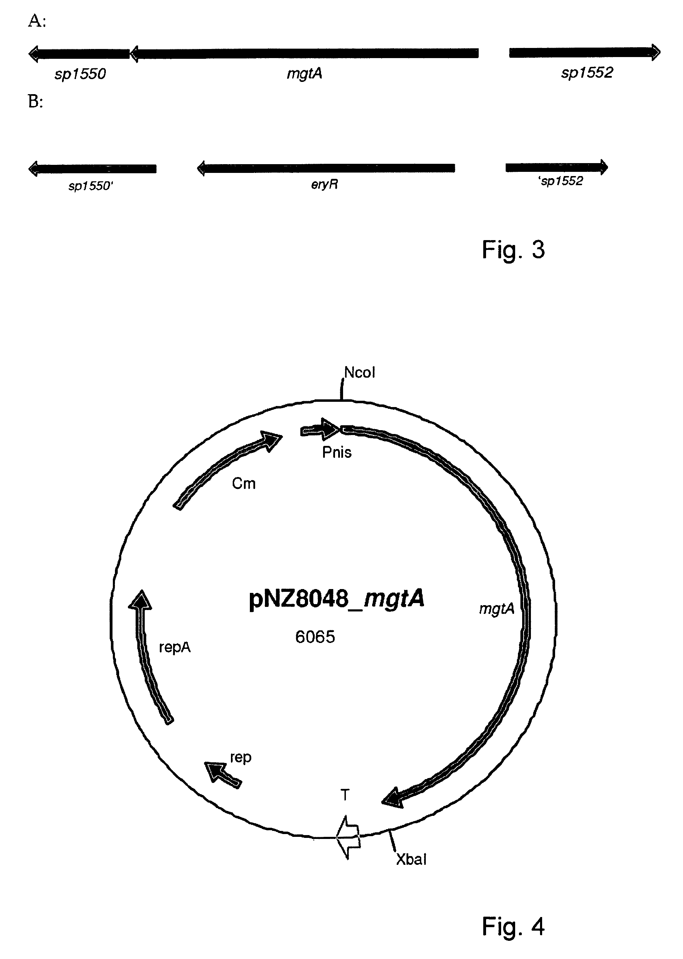

Figure 3 displays the chromosomal organisation of D39Δ*cps* and D39Δ*cps*Δ*mgtA*. A: Chromosomal organisation of D39Δ*cps*, before transformation and integration of the PCR product containing the *mgtA* knockout. B: Chromosomal organisation of D39Δ*cps*, after transformation and integration of the PCR product containing the replacement of *mgtA* by the erythromycin resistance gene. eryR: erythromycin resistance gene.
Figure 4 shows the map of the plasmid pNZ8048::*mgtA* containing *mgtA* under control of PnisA. The complete *mgtA* gene was ligated into the plasmid by making use of NcoI and XbaI restriction sites.

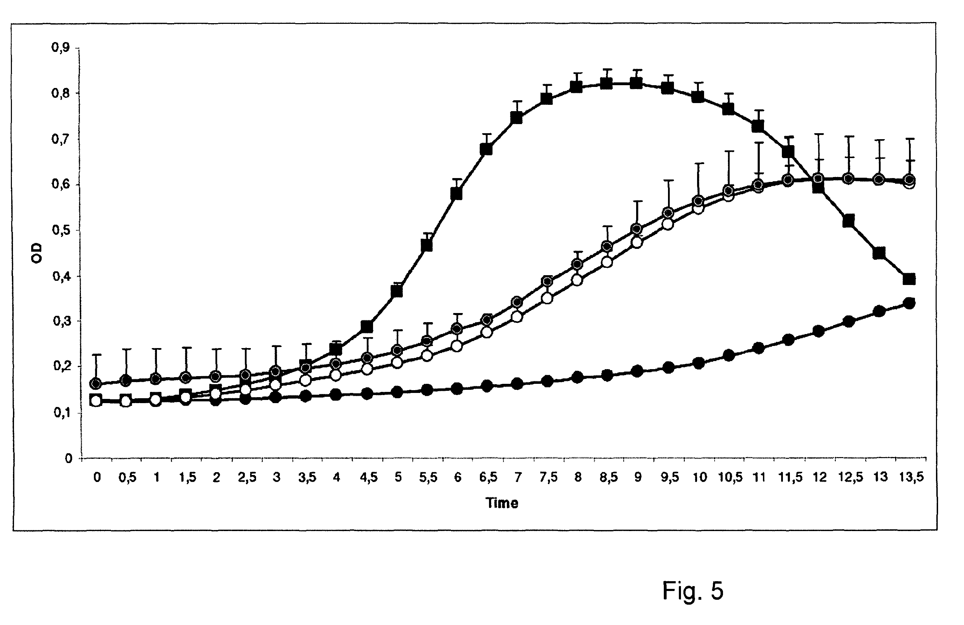

Figure 5: *MgtA* is the main Mg²⁺ importer for *S. pneumoniae* under normal growth conditions. Growth of the wildtype (black squares) in GM17 compared to growth of the mutant in GM17 (black circles), GM17 containing 5 mM MgCl₂ (white circles) and 50 mM MgCl₂ (grey circles). Results are the mean of a duplicate experiment + the standard deviation and are representative of at leas three experiments performed in duplicate.

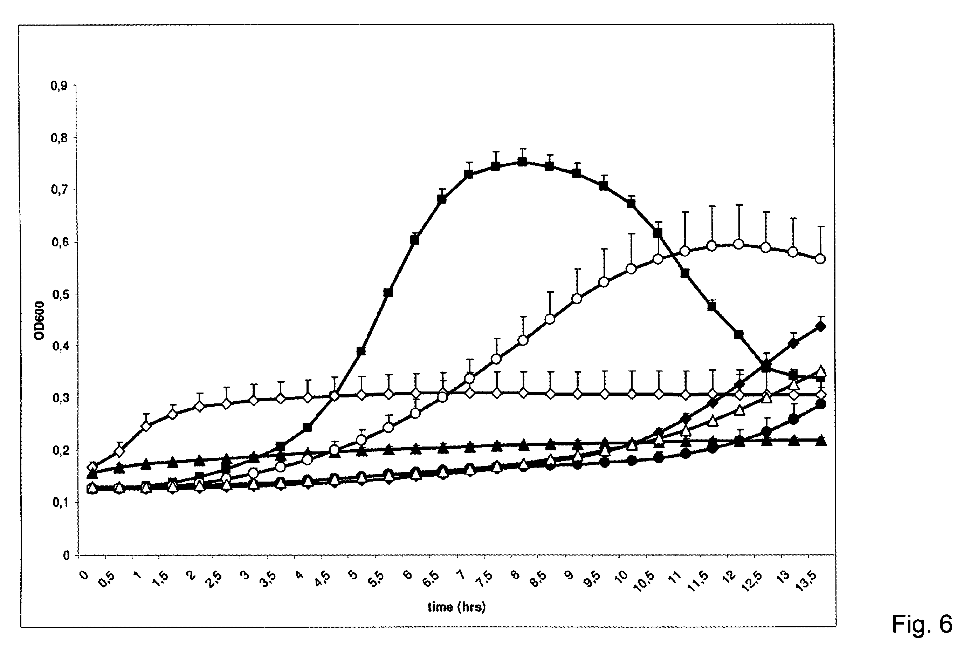

Figure 6: *MgtA* specifically transports Mg²⁺ in *S. pneumoniae* under normal growth conditions. Growth of the wildtype (black squares) in GM17 compared to growth of the mutant in GM17 (black circles), GM17 containing 5 mM MgCl₂ (white circles), GM17 containing 5 mM CaCl₂ (black triangles), GM17 containing 5 mM MnCl₂ (white diamonds), GM17 containing 50 µM CuCl₂ (white triangles) and GM17 containing 50 µM ZnCl₂ (black diamonds). Results are the mean of a duplicate experiment plus the standard deviation and are representative of at least three experiments performed in duplicate.

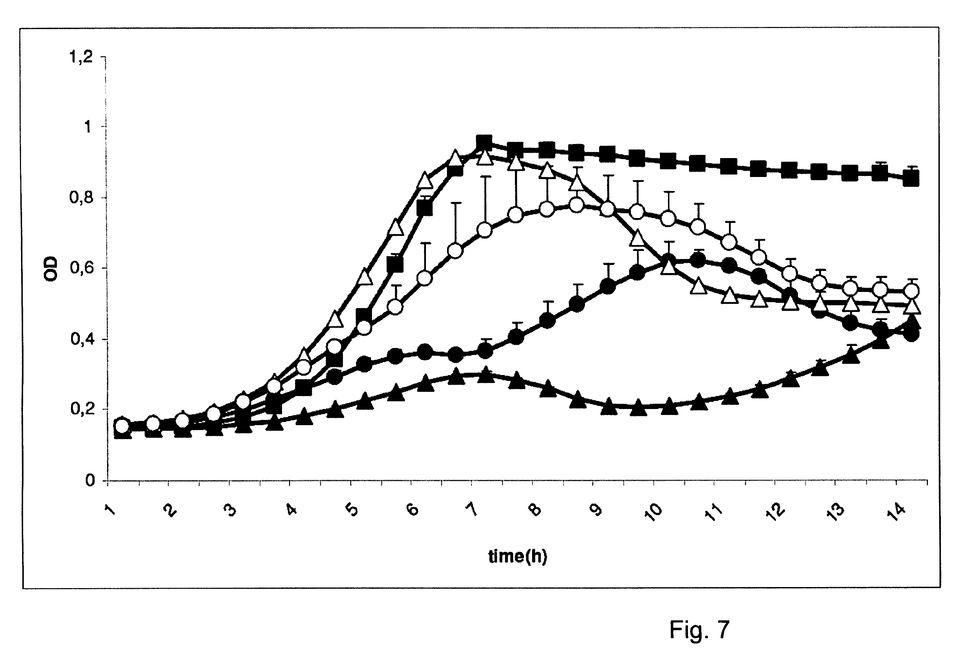

Figure 7: *MgtA* is the main Mg²⁺ importer for strain D39Δ*cps* under normal growth conditions and heterologous expression of the *mgtA* gene fully restores growth in GM17 in D39Δ*cps*. Growth of the wildtype (black squares) in GM17 compared to growth of the mutant in GM17 (black circles), GM17 containing 5 mM MgCl₂ (white circles), compared to the growth of D39NisRKΔ*cps*Δ*mgtA* pNZ8048 (black triangles) and D39NisRKΔ*cps*Δ*mgtA* pNZ*mgtA* (white triangles).

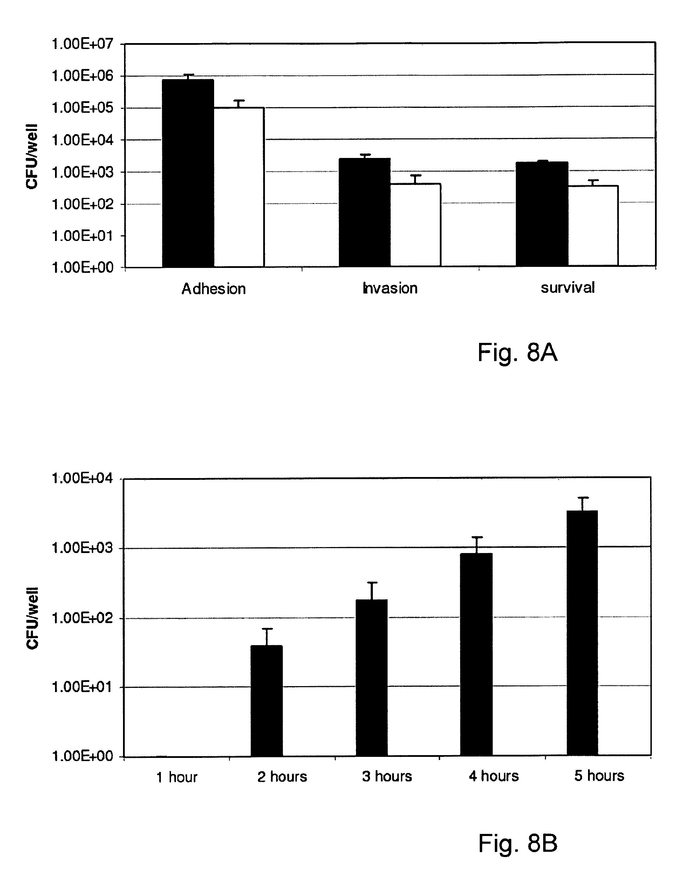

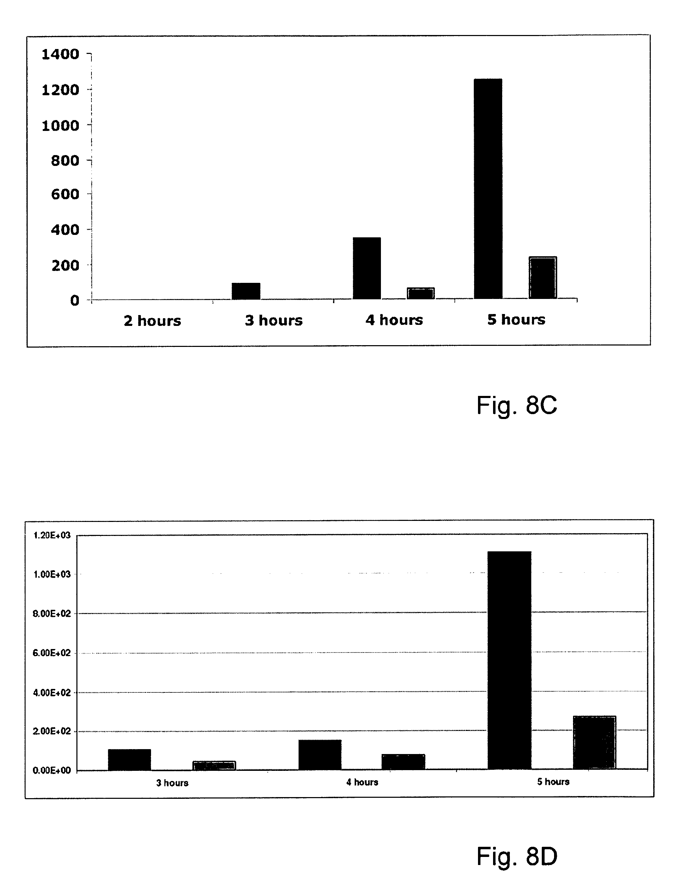

Figure 8: Magnesium homeostasis of *S. pneumoniae* is essential for transcytosis. **A:** The Δ*mgtA* mutant has a similar adhesion to, invasion and survival in HBMEC as the parent strain. Adhesion, invasion and survival after 5 hrs of strains D39Δ*cps* (black bars) and D39Δ*cps*Δ*mgtA* (white bars). Results are the mean of three independent experiments, vertical bars represent the standard deviations. **B:** Appearance over 5 hours of strains strains D39Δ*cps* (black bars) and D39Δ*cps*Δ*mgtA* (white bars) in the lower compartment. Results are the mean of two independent experiments, vertical bars represent the standard deviations **C:** Appearance in the lower compartment of D39Δ*cps bgaA*::NisRK (black bars), D39Δ*cps bgaA*::NisRK Δ*mgtA* pNG8048E (white bars), D39Δ*cps bgaA*::NisRK *ΔmgtA* p*mgtA.* **D:** Appearance of D39Dcps while 10 mM MgCl2 is added to the tissue culture medium (black bars), D39*ΔcpsΔmgtA* when no extra MgCl₂ is added to the medium.(white bars) and the same strain when 10 mM MgCl₂ is present in the tissue culture medium during the assay (grey bars).

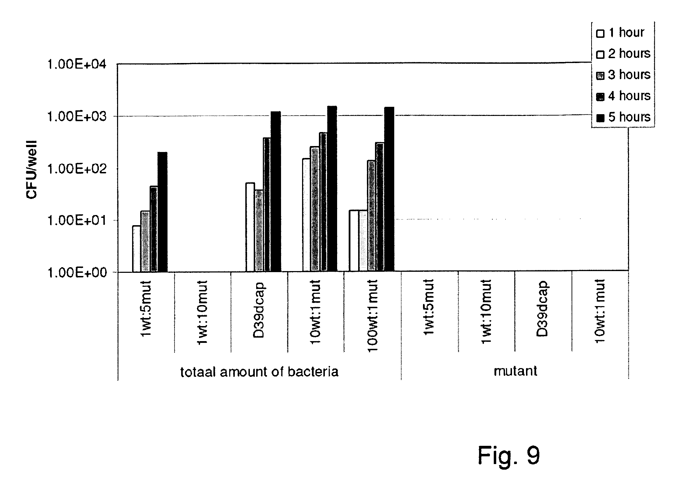

Figure 9: Appearance in the lower compartment over time of the total amount of bacteria (left part) and the *mgtA* mutant (right part) over time.

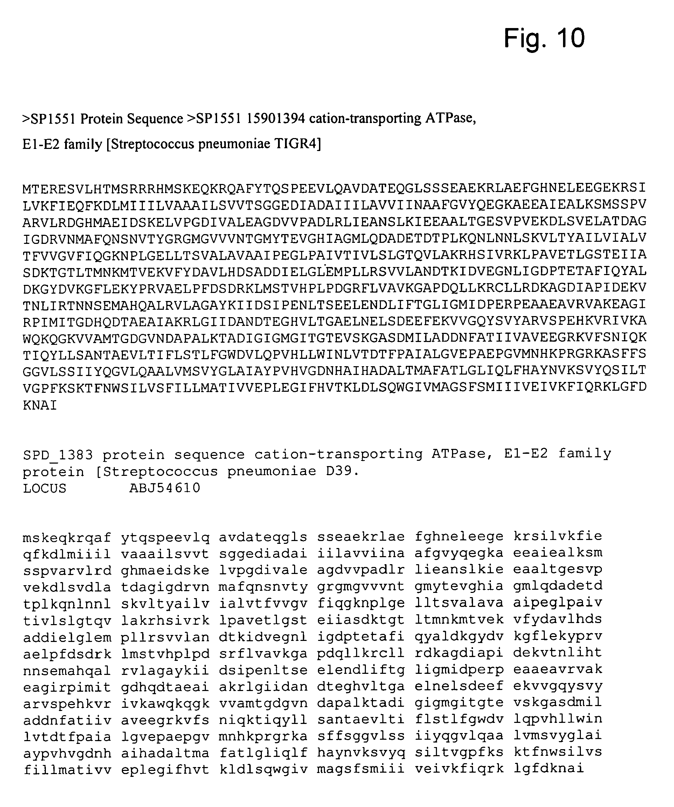

Fig. 10 shows two *S*. *pneumoniae* amino acid sequences coding for MgtA. Shown are the protein of *S. pneumoniae* TIGR4, indicated as SP1551 (TIGR code) and the protein of *S. pneumoniae* D39, indicated as SPD 1383

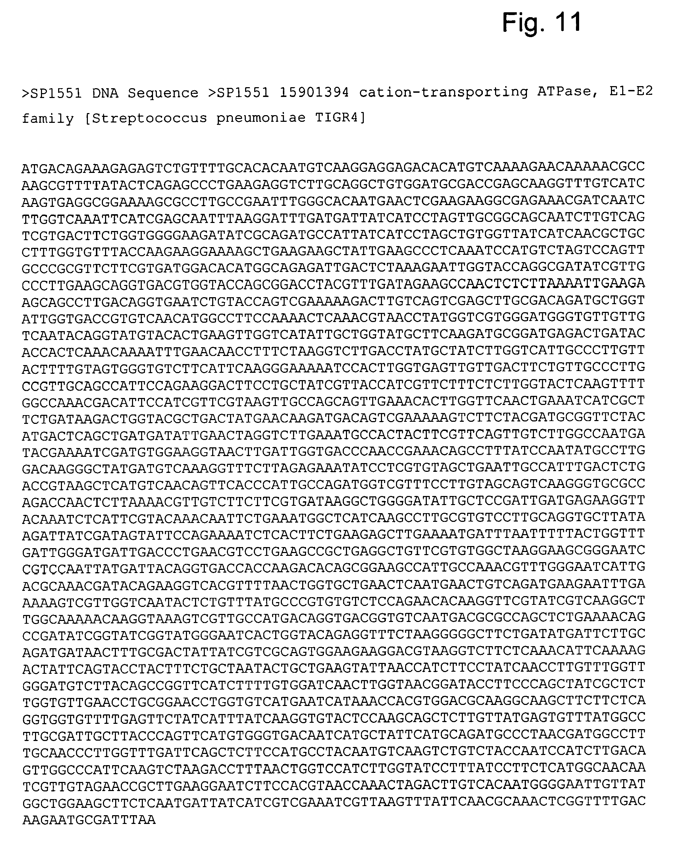

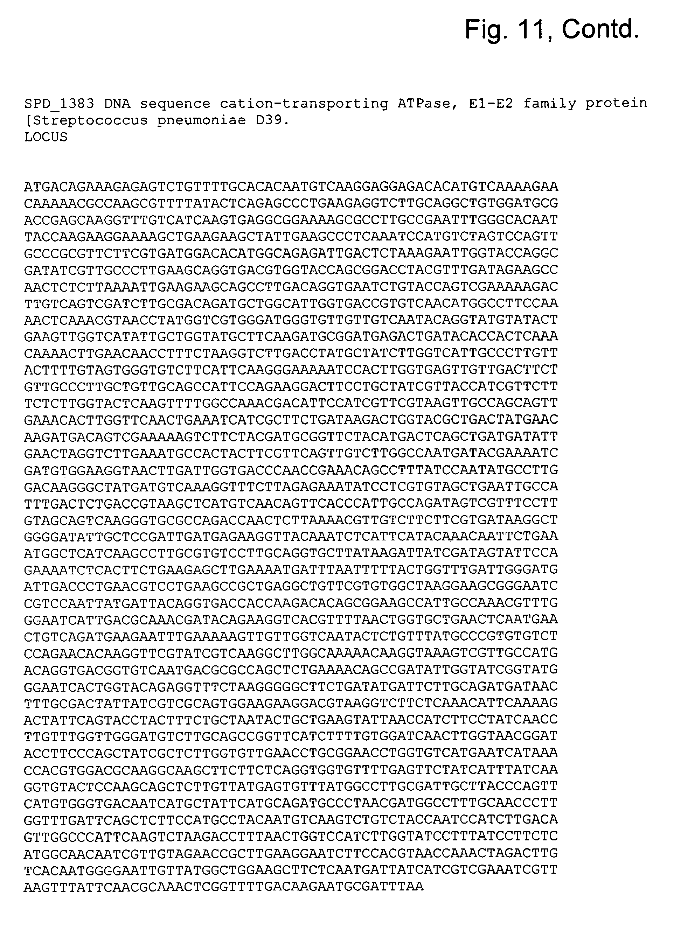

Fig 11 shows the corresponding nucleic acid sequence encoding the protein sequences displayed in Fig. 10.

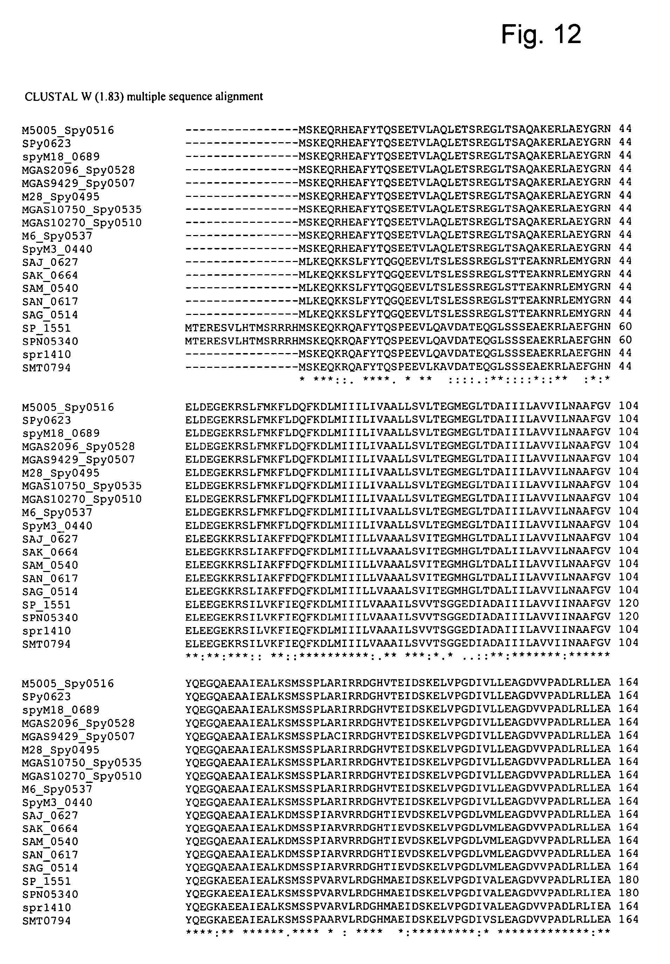

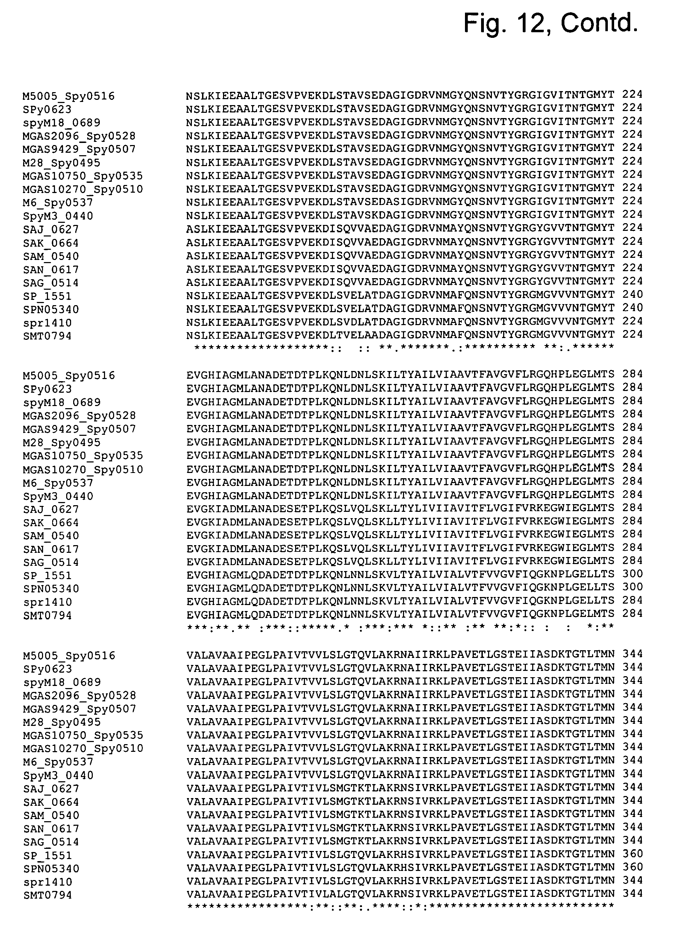

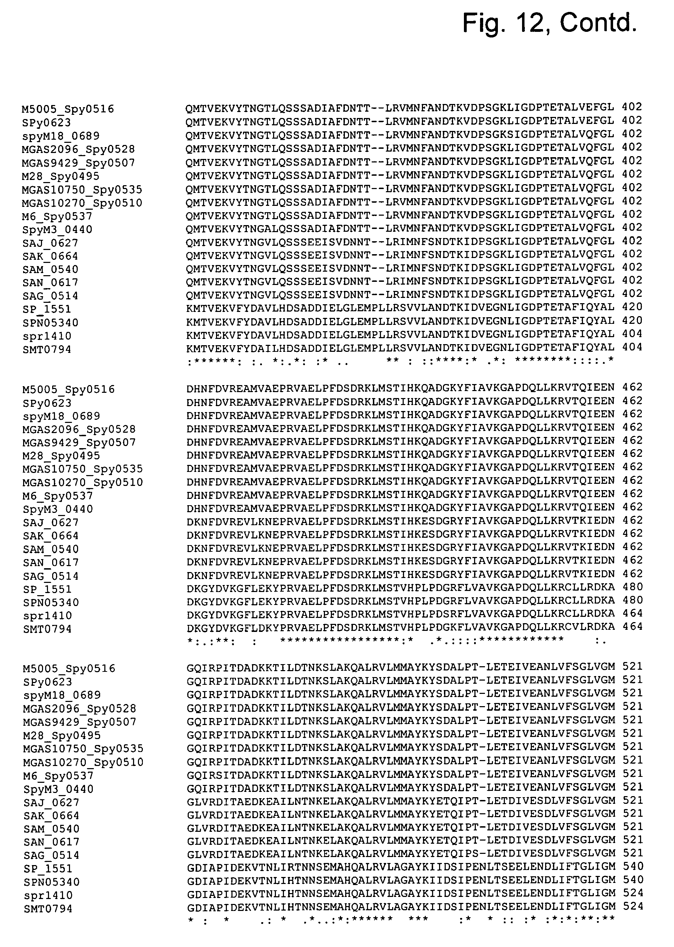

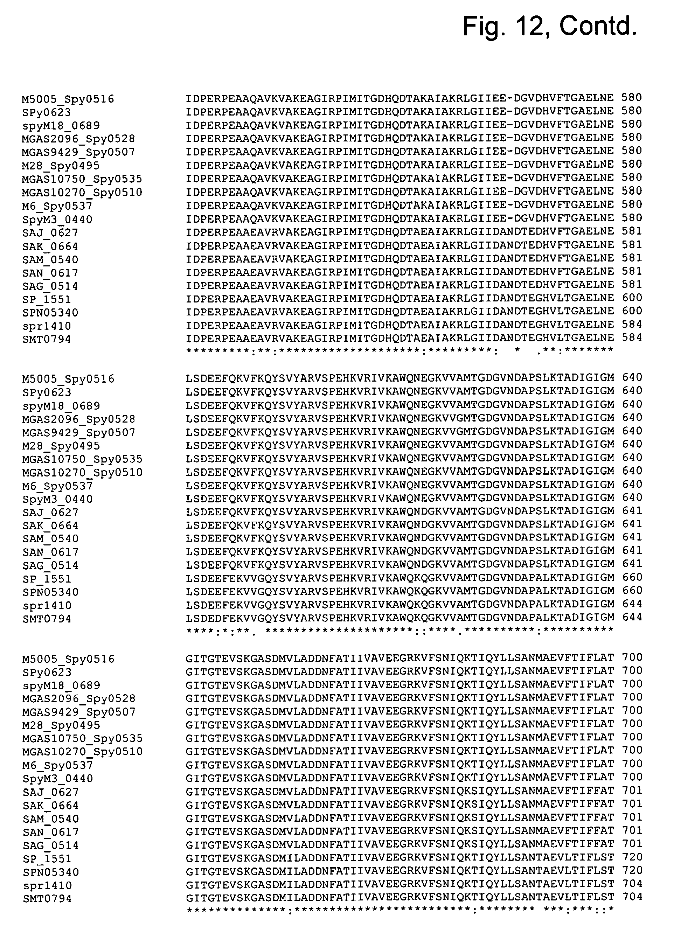

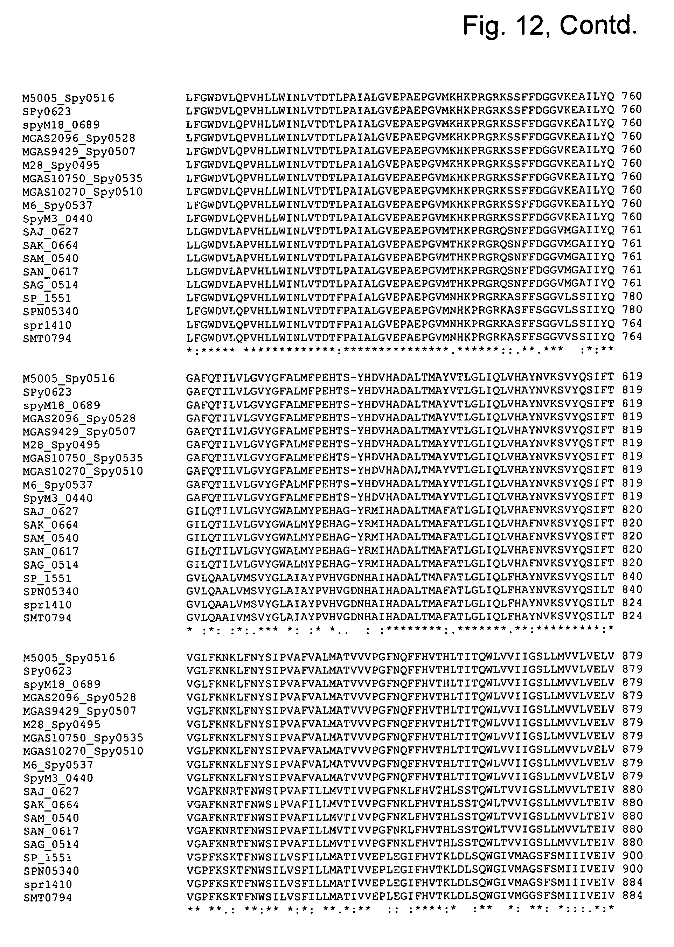

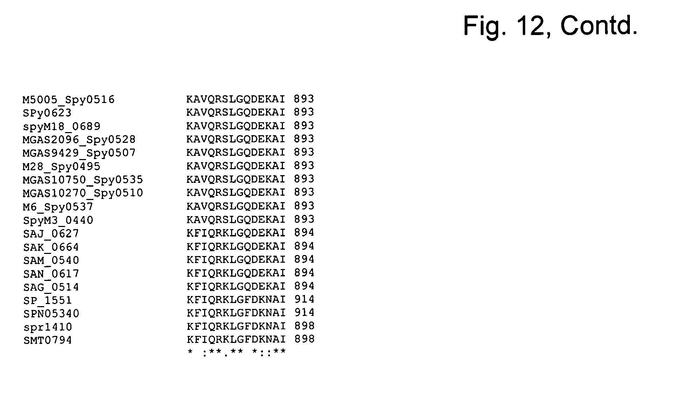

Fig. 12 provides the Clustal-W alignment of 8P1551 with highly homologous proteins.
M5005_Spy0516 : *Streptococcus pyogenes MGAS5005* Primary Locus: M5005_Spy0516 | TIGR Locus: NT10SP0571 | SWISS-PROT/TrEMBL AC: Q9A0T9 | GenBank ID: AAZ51134.1
SPy0623: *Streptococcus pyogenes SF370 serotype M1* Primary Locus: SPy0623 | TIGR Locus: NT01SP0493 | SWISS-PROT/TrEMBL AC: Q9A0T9 | GenBank ID: AAK33594.1
spyM18_0689:*Streptococcus pyogenes MGAS8232* Primary Locus: spyM18_0689 | TIGR Locus: NT03SP0621 | SWISS-PROT/TrEMBL AC: Q8P1V8 | GenBank ID: AAL97362.1
MGAS2096_Spy0528: *Streptococcus pyogenes MGAS2096:* Primary Locus: MGAS2096_Spy0628 | TIGR Locus: NT18SP0572 | SWISS-PROT/TrEMBL AC: Q1JCS8 | GenBank ID: ABF35580.1
MGAS9429_Spy0507 : *Streptococcus pyogenes MGAS9429* Primary Locus: MGAS9429_Spy0507 | TIGR Locus: NT19SP0543 | SWISS-PROT/TrEMBL AC: Q1JMQ4 | GenBank ID: ABF31695.1
M28_Spy0495 : *Streptococcus pyogenes MGAS6180* Primary Locus: M28_Spy0495 | TIGR Locus: NT13SP4547 | SWISS-PROT/TrEMBL AC: Q48UJ7 | GenBank ID: AAX71609.1
MGAS10750_Spy0535 : *Streptococcus pyogenes MGAS6180* Primary Locus: M28_Spy0495 | TIGR Locus: NT13SP0547 | SWISS-PROT/TrEMBL AC: Q48UJ7 | GenBank ID: AAX71609.1
MGAS10270_Spy0510: *Streptococcus pyogenes MGAS10270*MGASI0270_Spy0510| TIGR Locus: NT16SP0551 | SWISS-PROT/TrEMBL AC: Q1JHU8 | GenBank ID: ABF33575.1
M6_Spy0537: *Streptococcus pyogenes MGAS10394* Primary Locus: M6_Spy0537 | TIGR Locus: NT11SP0590 | SWISS-PROT/TrEMBL AC: Q5XD41 | GenBank ID: AAT86672.1
SpyM3_0440 *Streptococcus pyogenes MGAS315* Primary Locus: SpyM3_0440 | TIGR Locus: NT04SP0525 | SWISS-PROT/TrEMBL AC: Q8K867 | GenBank ID: AAM79047.1
SAJ_0627 *Streptococcus agalactiae 18RS21* Primary Locus: None | TIGR Locus: SAJ_0627 | SWISS-PROT/TrEMBL AC: None I GenBank ID: None
SAK_0664 *Streptococcus agalactiae A909* Primary Locus: None | TIGR Locus: SAK_0664 | SWISS-PROT/TrEMBL AC: Q3K2F7 | GenBank ID: ABA45439.1
SAM_0540 *Streptococcus agalactiae CJB111* Primary Locus: None | TIGR Locus: SAM_0540 | SWISS-PROT/TrEMBL AC: None | GenBank ID: None
SAN_061 *Streptococcus agalactiae COH1* Primary Locus: None | TIGR Locus: SAN_0617 | SWISS-PROT/TrEMBL AC: None | GenBank ID: None
SAG_0514 *Streptococcus agalactiae 2603V*/*R* Primary Locus: None | TIGR Locus: SAG_0614 | SWISS-PROT/TrEMBL AC: Q8E148 | GenBank ID: AAM99416.1
SP_1551 *Streptococcus pneumoniae TIGR4* Primary Locus: None | TIGR Locus: SP_1551 | SWISS-PROT/TrEMBL AC: Q97PQ2 | GenBank ID: AAK75638.1
SPN05340: *Streptococcus pneumoniae G54* Primary Locus: SPN05340 | TIGR Locus: NT05SP1364 | SWISS-PROT/TrEMBL AC: None | GenBank ID: None.
spr1410 *Streptococcus pneumoniae R6* Primary Locus: spr1410 | TIGR Locus: NT02SP1528 | SWISS-PROT/TrEMBL AC: Q8DP20 | GenBank ID: AAL00214.1
SMT0794: *Streptococcus mitis NCTC 12261* Primary Locus: None | TIGR Locus: SMT0794 | SWISS-PROT/TrEMBL AC: None | GenBank ID: None

### DETAILED DESCRIPTION OF THE PREFERRED EMBODIMENTS

The term "MgtA" refers to a gene product necessary for the uptake of Mg ²⁺ and translocation of endothelial cell layers by *S. pneumoniae.* The amino acid sequence is depicted in Figure 10. Functional homologues of the protein are present in other meningeal pathogens.

The term "meningeal pathogen" refers to any bacterial species capable of causing meningitis in a mammal. In addition thereto, or alternatively, the term refers to any bacterial species of which the *mgtA* gene product, or a functional homologue thereof, is a virulence factor. Examples of meningeal pathogens include, but are not limited to *Streptococcus pneumoniae, Haemophilus influenzae, Neisseria meningitidis, Neisseria gonorrhoea, Escherichia coli* (in particular strain K1), *Listeria monocytogenes, Staphylococcus aureus, Pseudomonas aeruginosa, Salmonella* spp., *Shigella* spp., *Clostridium* spp. and *Acinetobacter calcoaceticus*. These species are known to be associated with meningitis in mammals. Also, many of these bacteria comprise proteins that are functional MgtA homologues (i.e. that have homologous amino acid sequences; see below). The term is thus not restricted to bacteria causing meningitis, but also includes reference to bacteria in which an *mgtA* gene product is expressed which is known to play a role in the process of Mg ²⁺ uptake and /or transcytosis.

A "virulence factor" is referred to herein as a property of a pathogen that allows it to cause disease. Virulence factors may distinguish a pathogenic microorganism from otherwise identical non-pathogenic microorganisms by allowing pathogens to invade, adhere to, and colonize a host, and then harm the host, as for an organism to be pathogenic it must be able to invade a host, multiply in the host, evade host defences, and harm the host in some way. As used herein *mgtA* gene product, or a functional homologue thereof, is a virulence factor.

The terms "invade" and "invasion" refer to the growing of infections into tissues, i.e., through and then beneath epithelial tissues in particular it encompasses the process of transcytosis.

The term "transcytosis" is used herein in its art-recognized meaning (see e.g. also e.g. Ring A et al. J. Clin. Invest 102, 347 (1998); Zhang JR et al., Cell 102, 827 (2000)) and refers to the transport of macromolecules and bacterial cells from one side of a cell to the other within a membrane-bounded carrier(s). Transcytosis is used by multicellular organisms to selectively move material between two different environments while maintaining the distinct compositions and integrity of those environments (see Figure 1A).

The terms "meningeal streptococcus" and its plural "meningeal streptococci" are used to indicate *Streptococcus* spp. that are capable of causing meningitis, i.e. that are capable of transcytosis. Examples of meningeal streptococci are β-haemolytic streptococci belonging to Lancefield Group C, *S. acidominimus, S agalactiae* (a.k.a. β-haemolytic streptococci belonging to Lancefield Group B), *S. bovis, S. constellatus, S. equinus, S. equisimilis, S. mitis, S. oralis, S. pneumoniae, S. pyogenes* (a.k.a. β-haemolytic streptococci belonging to Lancefield Group A), *S. salivarius, S. sanguis, S. suis* and *S. uberis.* In preferred embodiments of aspects of the present invention, the meningeal streptococcus is selected from the group consisting of *S agalactiae, S. pneumoniae, S. pyogenes, S. suis* and *S. uberis.* Most preferably, it is *S. pneumoniae.*

The term "functional homologue" refers to a protein which retains the activity of the functional MgtA protein and preferably has a sequence homology of at least 60%. The homology is preferably determined using BLAST analysis, well known in the art. It is further preferred that the homologue has at least 70%, more preferably at least 80%, still more preferably at least 90% and most preferably 95% sequence homology to the sequence as displayed in Fig. 10. Further comprised in functional homologues are sequences having the activity of a functional MgtA protein (i.e. a bacterial Mg²⁺ ATP-ase) and having the so-called mgtA domain

### D K T G T [LIVM] [TI]

Polypeptides have "homologous" sequences if the sequence of amino acids in the two sequences is the same when aligned for maximum correspondence as described herein. Sequence comparison between two or more polypeptides is generally performed by comparing portions of the two sequences over a comparison window to identify and compare local regions of sequence similarity. The comparison window is generally from about 7 to 1000 contiguous amino acids. The "percentage of sequence homology" for polypeptides, such as 50, 60, 70, 80, 90, 95, 98, 99 or 100 percent sequence homology may be determined by comparing two optimally aligned sequences over a comparison window, wherein the portion of the amino acid sequence in the comparison window may include additions or deletions (i.e. gaps) as compared to the reference sequence (which does not comprise additions or deletions) for optimal alignment of the two sequences. The percentage is calculated by: (a) determining the number of positions at which the identical amino acid occurs in both sequences to yield the number of matched positions; (b) dividing the number of matched positions by the total number of positions in the window of comparison; and (c) multiplying the result by 100 to yield the percentage of sequence homology. Optimal alignment of sequences for comparison may be conducted by computerized implementations of known algorithms, or by inspection. Fully analogous to amino acid sequence comparison, nucleic acid sequence comparison may occur to determine sequence homology between two or more polynucleotides, for instance the polynucleotides encoding the MgtA protein. Readily available sequence comparison and multiple sequence alignment algorithms are, respectively, the Basic Local Alignment Search Tool (BLAST) (Altschul, S.F. et al. 1990. J. Mol. Biol. 215:403; Altschul, S.F. et al. 1997. Nucleic Acid Res. 25:3389-3402) and ClustalW programs both available on the internet. Other suitable programs include GAP, BESTFIT and FASTA in the Wisconsin Genetics Software Package (Genetics Computer Group (GCG), Madison, WI, USA).

Included in the definition of functional homologues are the proteins listed in Fig. 12.

The term "functional fragment" refers to a shortened version of the protein which is a functional variant or functional derivative. A "functional variant" or a "functional derivative" of a protein is a protein the amino acid sequence of which can be derived from the amino acid sequence of the original protein by the substitution, deletion and/or addition of one or more amino acid residues in a way that, in spite of the change in the amino acid sequence, the functional variant retains at least a part of at least one of the biological activities of the original protein that is detectable for a person skilled in the art. A functional variant is generally at least 60% homologous (preferably the amino acid sequence is at least 60% identical), advantageously at least 70% homologous and even more advantageously at least 90% homologous to the protein from which it can be derived. A functional variant may also be any functional part of a protein; the function in the present case being particularly but not exclusively its antigenic property, i.e. that it is capable of eliciting antibodies which block or inhibit the biological function of the full length active protein. Preferably the amino acid sequence differs from the sequence of Fig. 10 (the protein sequence) mainly or only by conservative substitutions. More preferably the protein comprises an amino acid sequence having 90% or more, still more preferably 95%, sequence identity with the sequence of Fig. 10 and optimally 100% identity with those sequences.

The expression "conservative substitutions" as used with respect to amino acids relates to the substitution of a given amino acid by an amino acid having physicochemical characteristics in the same class. Thus where an amino acid in the sequence as provided in Fig. 10 has a hydrophobic characterising group, a conservative substitution replaces it by another amino acid also having a hydrophobic characterising group; other such classes are those where the characterising group is hydrophilic, cationic, anionic or contains a thiol or thioether. Such substitutions are well known to those of ordinary skill in the art, i.e. see US 5,380,712. Conservative amino acid substitutions may be made, for example within the group of aliphatic nonpolar amino acids (Gly, Ala, Pro, Ile, Leu, Val), the group of polar uncharged amino acids (Cys, Ser, Thr, Met, Asn, Gln), the group of polar charged amino acids (Asp, Glu, Lys, Arg) or the group of aromatic amino acids (His, Phe, Tyr, Trp).

The term " immunogenic part" includes reference to any part of the MgtA protein, or a functional homologue or (functional) fragment thereof, which is capable of eliciting an immune response in a mammal. Said immunogenic part preferably corresponds to an antigenic determinant of said pathogen.

As used herein, the term "antigen" refers to a molecule capable of being bound by an antibody or a T cell receptor (TCR) if presented by moleculas of the major histocompatibility complex (MHC). The term "antigen", as used herein, also encompasses T-cell epitopes. A T-cell epitope is recognized by a T-cell receptor in the context of a MHC class I, present on all cells of the body except erythrocytes, or class II, present on immune cells and in particular antigen presenting cells. This recognition event leads to activation of T-cells and subsequent effector mechanisms such as proliferation of the T-cells, cytokine secretion, perforin secretion etc. An antigen is additionally capable of being recognized by the immune system and/or being capable of inducing a humoral immune response and/or cellular immune response leading to the activation of B- and/or T-lymphocytes. This may, however, require that, at least in certain cases, the antigen contains or is linked to a T-Helper cell epitope and is given in adjuvant. An antigen can have one or more epitopes (Band T-epitopes). The specific reaction referred to above is meant to indicate that the antigen will preferably react, typically in a highly selective manner, with its corresponding antibody or TCR and not with the multitude of other antibodies or TCRs which may be evoked by other antigens. Antigens as used herein may also be mixtures of several individual antigens.

As used herein, the term "antigenic determinant" is meant to refer to that portion of an antigen that is specifically recognized by either B- or T-lymphocytes. B-lymphocytes respond to foreign antigenic determinants via antibody production, whereas T-lymphocytes are the mediator of cellular immunity. Thus, antigenic determinants or epitopes are those parts of an antigen that are recognized by antibodies, or in the context of an MHC, by T-cell receptors. An antigenic determinant may contain one or more epitopes. Epitopes may be present on the intracellular (inside), transmembrane spanning (transmembrane), as well as extracellular (outside) regions of a protein molecule. It is expected that antigenic determinants are associated with in particular those regions of the transmembrane protein MgtA (the antigen) which are on the outside of the cytoplasmic membrane of the bacteria. These regions can be predicted from the sequence as provided in Fig. 10, by using for instance the software program TMHMM, e.g. version 2.0c, which provides a method for prediction transmembrane helices based on a hidden Markov model. Using this program, it was determined that the 914 amino acids of MgtA were predicted to form a protein having 8 transmembrane helices (residues 81-98, 102-121, 269-288, 298-320, 766-788, 808-830, 851-873, and 883-902. The four 'outside' regions, preferred as antigenic determinants or immunogenic parts in the present invention, include the residues 99-101, 289-297, 789-807, and 874-882. Also included are the corresponding outside regions from the proteins listed in Fig. 12 and other homologues.

The term "prophylactic or therapeutic treatment of an infection by a meningeal pathogen" refers to both prophylactic or therapeutic treatments wherein the transcytosis of the pathogen is blocked or diminished, but also to treatments wherein antibodies against MgtA recognize the bacteria and will protect the host against infection, either directly through immune clearance, or indirectly by blocking the activity of the protein, thereby inhibiting the in vivo growth of the bacteria. Also, the term refers to blocking the function of the MgtA protein *in vivo* thereby reducing the adhesion abilities of the pathogen with a concomitant reduction in colonisation and invasion capabilities. The term thus includes inducing immune responses in subjects using vaccine formulations of the invention, as well as inhibiting growth of the pathogen *in vivo* by using antibodies of the present invention as an active compound in a pharmaceutical composition administered to the subject.

The term "antibody" refers to molecules which are capable of binding an epitope or antigenic determinant and includes reference to antigen binding forms of antibodies (e. g., Fab, F(ab)2). The term "antibody" frequently refers to a polypeptide substantially encoded by an immunoglobulin gene or immunoglobulin genes, or fragments thereof which specifically bind and recognize an analyte (antigen). However, while various antibody fragments can be defined in terms of the digestion of an intact antibody, one of skill will appreciate that such fragments may be synthesized de novo either chemically or by utilizing recombinant DNA methodology. Thus, the term antibody, as used herein, also includes antibody fragments such as single chain Fv, chimeric antibodies (i. e., comprising constant and variable regions from different species), humanized antibodies (i. e., comprising a complementarity determining region (CDR) from a non-human source) and heteroconjugate antibodies (e. g., bispecific antibodies). The antibody can be monoclonal or polyclonal and can be prepared by techniques that are well known in the art such as immunization of a host and collection of sera (polyclonal) (see, e.g., Parker, Radioimmunoassay of Biologically Active Compounds, Prentice-Hall (Englewood Cliffs, N.J., U.S., 1976), Butler, J. Immunol. Meth. 7, 1-24 (1975); Broughton and Strong, Clin. Chem. 22, 726-732 (1976); and Playfair, et al., Br. Med. Bull. 30, 24-31 (1974)) or by preparing continuous hybrid cell lines and collecting the secreted protein (monoclonal) (see, *e.g.,* Kohler et al in Nature 256, 495-497 (1975) and Eur. J. Immunol. 6, 511-519 (1976); by Milstein et al. Nature 266, 550-552 (1977); and by Walsh Nature 266, 495 (1977)) or by cloning and expressing nucleotide sequences or mutagenized versions thereof coding at least for the amino acid sequences required for specific binding of natural antibodies. Antibodies may include a complete immunoglobulin or fragment thereof, which immunoglobulins include the various classes and isotypes, such as IgA, IgD, IgE, IgG1, IgG2a, lgG2b and lgG3, IgM, etc. Fragments thereof may include Fab, Fv and F(ab')2, Fab', and the like. In addition, aggregates, polymers, and conjugates of immunoglobulins or their fragments can be used where appropriate so long as binding affinity for a particular molecule is maintained.

As used herein, the term "monoclonal antibody" refers to an antibody composition having a homogeneous antibody population. The term is not limited regarding the species or source of the antibody, nor is it intended to be limited by the manner in which it is made. The term encompasses whole immunoglobulins as well as fragments such as F.sub.ab, F.sub.(ab')2, F.sub.v, and others, such as CDR fragments, which retain the antigen binding function of the antibody. Monoclonal antibodies of any mammalian species can be used in this invention. In practice, however, the antibodies will typically be of rat or murine origin because of the availability of rat or murine cell lines for use in making the required hybrid cell lines or hybridomas to produce monoclonal antibodies.

As used herein, the term "humanized monoclonal antibodies" means that at least a portion of the exposed amino acids in the framework regions of the antibody (or fragment), which do not match with the corresponding amino acids in the most homologous human counterparts, are changed, such as by site directed mutagenesis of the DNA encoding the antibody. Because these exposed amino acids are on the surface of the molecule, this technique is called "resurfacing." Moreover, because the amino acids on the surface of the molecule are the ones most likely to give rise to an immune response, this resurfacing decreases the immunogenicity of the monoclonal antibody when administered to a species whose cell line was not used to generate the antibody, such as a human. The term "humanized monoclonal antibody" also includes chimeric antibody wherein the light and heavy variable regions of a monoclonal antibody generated by a hybridoma from a non-human cell line are each attached, via recombinant technology, to one human light chain constant region and at least one heavy chain constant region, respectively. The preparation of such chimeric (i. e., humanized) antibodies are well known in the art.

The term "specifically recognizing", includes reference to a binding reaction between an antibody and a protein having an epitope recognized by the antigen binding site of the antibody. This binding reaction is determinative of the presence of a protein having the recognized epitope amongst the presence of a heterogeneous population of proteins and other biologics. Thus, under designated immunoassay conditions, the specified antibodies bind to an analyte having the recognized epitope to a substantially greater degree (e. g., at least 2-fold over background) than to substantially all analytes lacking the epitope which are present in the sample. Specific binding to an antibody under such conditions may require an antibody that is selected for its specificity for a particular protein. For example, antibodies raised to the polypeptides of the present invention can be selected to obtain antibodies specifically recognizing polypeptides of the present invention. The proteins used as immunogens can be in native conformation or denatured so as to provide a linear epitope. A variety of immunoassay formats may be used to select antibodies specifically recognizing a particular protein (or other analyte). For example, solid-phase ELISA immunoassays are routinely used to select monoclonal antibodies specifically immunoreactive with a protein. See Harlow and Lane, Antibodies, A Laboratory Manual, Cold Spring Harbor Publications, New York (1988), for a description of immunoassay formats and conditions that can be used to determine selective reactivity.

A "subject" as referred to herein includes reference to is meant to include mammals and other animals, wherein mammals include for example, humans, apes, monkeys, horses, cattle, pigs, goats, dogs, cats, rats, mice, and sheep. The term "non-human animal" is meant to disclaim humans.

### Methods for purification of MgtA proteins and amino acid sequencing.

The MgtA protein may be used to produce vaccines or antibodies of the invention. A suitable source of MgtA protein is for instance *S. pneumoniae.* The protein may be used with or without the presence of detergents and or lipid analogs non-purified (associated with in intact cells), partially purified (associated with membrane fragments or other cellular constituents), or purified,( i.e. isolated and essentially free of other cellular constituents). Having prepared purified or partially purified MgtA protein it is possible to prepare a substantially pure preparation of MgtA protein. Although numerous methods and strategies for protein purification are known in the art it will be most convenient to purify MgtA protein by either electrophoresis using for instance a sodium dodecylsulphate-polyacrylamide gel (SDS-PAGE) or by affinity chromatography. Each of these methods will be described below.

MgtA protein may be separated from other proteins by electrophoresis using for instance Tricine-SDS-PAGE (Schagger and Von Jagow (1987) Analytical Biochemistry 166, 368-379) or Glycine-SDS-PAGE (Laemmli (1970) Nature 227, 680-685). Other electrophoresis systems that are capable of resolving the various proteins comprised in a bacterial lysate, or transcribed from its genome and expressed in a suitable expression system, may of course also be employed, such as non-denaturing gel electrophoresis. The area of the PAGE gel including the target protein may be excised and the target polypeptides may be eluted therefrom. The protein of interest may be identified by its mobility relative to reference polypeptides in a gel. To increase purity the eluted protein may be run on a second SDS-PAGE gel and eluted a second time. The protein or peptide contained in the excised gel fragment may then be eluted again and is suitable for use in immunization or in protein sequencing.

MgtA protein may also be purified by affinity chromatography using an antibody (such as a monoclonal antibody) that specifically binds to a MgtA protein. The antibody may be covalently coupled to solid supports such as celluloses, polystyrene, polyacrylamide, cross-linked dextran, beaded agarose or controlled pore glass using bifunctional coupling agents that react with functional groups on the support and functional groups (*i*.*e*., reactive amino acid side chains) on the antibody molecule. Such methods are readily available to the skilled person. The resulting antibody-bearing solid phase is contacted with purified or partially purified MgtA protein under reducing conditions using pH, ionic strength, temperature and residence times that permit the MgtA protein to bind to the immobilized antibody. The protein is eluted from the column by passing an eluent that dissociates hydrogen bonds through the bed. Buffers at specific pH or NaCl solutions above about 2 M are commonly used eluents.

Methods for carrying out affinity chromatography using antibodies as well as other methods for immunoaffinity purification of proteins are well known in the art (see *e*.*g*., Harlow and Lane, (1988) Antibodies: A Laboratory Manual. Cold Spring Harbor Laboratory, Cold Spring Harbor, NY).

With the teachings provided herein, the skilled person is capable of isolating a MgtA protein, determining the amino acid sequence of for instance the N-terminal part of said protein, designing a set of degenerate probes (for the degeneracy of the genetic code) to hybridise with the DNA coding for a region of said protein, using these probes on an array of genes in a genomic library produced from the meningeal pathogen, obtaining positive hybridisations and locating the corresponding genes. The skilled person is then capable of identifying the structural region of the gene and optionally upstream and downstream sequences thereof. Thereafter the skilled person is capable of establishing the correct sequence of the amino acid residues that form the protein in any meningeal pathogen, and establish the presence of functional homologues of MgtA.

### Antibody production

Antibodies, either monoclonal or polyclonal, can be generated to a purified or partially purified protein or peptide fragment of a meningeal pathogen in a variety of ways known to those skilled in the art including injection of the protein as an antigen in animals, by hybridoma fusion, and by recombinant methods involving bacteria or phage systems (see Harlow and Lane (1988) *supra*.; Marks et al., (1992) Journal of Biological Chemistry, 267, 16007-16010; Marks et al., (1992) Biotechnology 10: 779:783; Lowman et al., (1991) Biochem. 30(45): 10832-8; Lerner et al., (1992) Science 258:1313-1314, each of which reference discloses suitable methods).

Antibodies against MgtA or functional homologues thereof, may be produced by immunizing an appropriate vertebrate, preferably mammalian host, e.g., rabbits, goats, rats and mice or chicken with the protein alone or in conjunction with an adjuvant. Usually two or more immunizations will be involved, and the blood or spleen will be harvested a few days after the last injection. For polyclonal antisera, the immunoglobulins may be precipitated, isolated and (affinity) purified. For monoclonal antibodies, the splenocytes will normally be fused with an immortalized lymphocyte, e.g., a myeloid line, under selective conditions for hybridomas. The hybridomas may then be cloned under limiting dilution conditions and their supernatants screened for antibodies having the desired specificity. Techniques for producing (monoclonal) antibodies and methods for their preparation and use in various procedures are well known in the literature (see *e.g.* U.S. Pat. Nos. 4,381,292, 4,451,570, and 4,618,577; Harlow, E. and Lane, D. (1988) Antibodies: A Laboratory Manual. Cold Spring Harbor Laboratory, Cold Spring Harbor, NY.; Ausubel, F.M., Brent, R., Kingston, R.E., Moore, D.D.,Seidman, J.G., Smith, J.A., Struhl, K. eds. (1998) Current protocols in molecular biology. V.B. Chanda, series ed. New York: John Wiley & Sons; Rose, N., DeMacrio, E., Fahey, J., Friedman, H., Penn, G. (1997) Manual of Clinical Laboratory Immunology. American Soc. Microbiology Press, Washington, D.C Coligan, J.E., Kruisbeek, A.M., Margulies, D.H., Shevach, E.M. Strober, W. (Eds.) (1997) Current Protocols in Immunology. John Wiley & Sons Inc. Baltimore). Typically, an antibody directed against a protein will have a binding affinity of at least 1x10⁵-1x10⁷ M⁻¹.

A recombinant MgtA protein or functional homologues thereof, such as may be obtained by expressing a MgtA-encoding genomic sequence in a suitable expression system, is preferred as the antigen in methods for producing an antibody. However, purified proteins may also be used, as well as protein fragments. Antigens suitable for antibody production include any fragment of MgtA protein that elicits an immune response in a mammal exposed to said protein. Preferred antigens of the invention include those fragments that comprise the antigenic determinenants, although any region of the MgtA protein may in principle be used.

Methods for cloning genomic sequences, for manipulating the genomic sequences to and from expression vectors, and for expressing the protein encoded by the genomic sequence in a heterologous host are well-known, and these techniques can be used to provide the expression vectors, host cells, and the cloned genomic sequences encoding MgtA protein, functional homologues or fragments thereof, which sequences are to be expressed in a host to produce antibodies for use in methods of the present invention (see for instance Sambrook, J., Russell D.W., Sambrook, J. (2001) Molecular Cloning: a Laboratory Manual. Cold Spring Harbor Laboratory Press, Plainview, N.Y., and Ausubel, *et al., supra*).

A variety of expression systems may be used to produce antigens for use in methods of the present invention. For instance, a variety of expression vectors suitable to produce proteins in *E.coli, B.subtilis,* yeast, insect cells, plant cells and mammalian cells have been described, any of which might be used to produce an antigen suitable to produce an anti-MgtA antibody or fragment thereof. Of course *Streptococcus pneumoniae* itself may also be used as an expression vector for this purpose.

One use of antibodies of the invention is to provide active ingredients for a pharmaceutical composition capable of inhibiting growth of a meningeal pathogen. Another use of antibodies of the invention is to screen cDNA expression libraries for identifying clones containing cDNA inserts that encode proteins of interest or structurally-related, immuno-cross-reactive proteins. Such screening of cDNA expression libraries is well known in the art (see *e*.*g*. Young R.A., Davis, R.W. (1983) Proc. Natl. Acad. Sci. U.S.A. 80:1194-1198), to which reference is made in this context, as well as other published sources. Another use of these antibodies is for use in affinity chromatography for purification of MgtA protein or functional homologues thereof. These antibodies are also useful for assaying for infection with meningeal pathogens.

### MgtA Antigen Epitopes

The MgtA antigen epitopes of this invention, which alone or together form an MgtA-based antigenic determinant of the meningeal pathogen, are molecules that are immunoreactive with anti-MgtA monoclonal antibodies and whose binding to a MgtA antigen located on the surface of a meningeal bacterial pathogen cell prevents the growth and/or transcytosis of said cell. Systematic techniques for identifying these epitopes are known in the art, as described in US 4,708,871, which is incorporated herein by reference. Typically, these epitopes are short amino acid sequences. These sequences may be embedded in the sequence of longer peptides or proteins, as long as they are accessible.

The epitopes of the invention may be prepared by standard peptide synthesis techniques, such as solid-phase synthesis. Alternatively, the sequences of the invention may be incorporated into larger peptides or proteins by recombinant methods. This is most easily accomplished by preparing a DNA cassette which encodes the sequence of interest, and ligating the cassette into DNA encoding the protein to be modified at the appropriate site. The sequence DNA may be synthesized by standard synthetic techniques, or may be excised from the phage pIII gene using the appropriate restriction enzymes. Epitopes identified herein may be prepared by simple solid-phase techniques. The minimum binding sequence may be determined systematically for each epitope by standard methods, for example, employing the method described in US 4,708,871. Briefly, one may synthesize a set of overlapping oligopeptides derived from the MgtA antigen bound to a solid phase array of pins, with a unique oligopeptide on each pin. The pins are arranged to match the format of a 96-well microtiter plate, permitting one to assay all pins simultaneously, e.g., for binding to an anti-MgtA monoclonal antibody. Using this method, one may readily determine the binding affinity for every possible subset of consecutive amino acids.

### Antibody Formulations and Methods of Administration

The antibodies of this invention are administered at a concentration that is therapeutically effective to prevent or treat infections by meningeal pathogens. To accomplish this goal, the antibodies may be formulated using a variety of acceptable excipients known in the art. Typically, the antibodies are administered by injection, either intravenously or intraperitoneally. Methods to accomplish this administration are known to those of ordinary skill in the art. It may also be possible to obtain compositions which may be topically or orally administered, or which may be capable of transmission across mucous membranes.

Before administration to patients, formulants (components other than the active ingredient in a product have many functions, such as carrier and excipients) may be added to the antibodies. A liquid formulation is preferred. For example, these formulants may include oils, polymers, vitamins, carbohydrates, amino acids, salts, buffers, albumin, surfactants, or bulking agents.

Additionally, antibodies can be chemically modified by covalent conjugation to a polymer to increase their circulating half-life, for example. Preferred polymers are polyethylene glycol (PEG) and polyoxyethylated polyols, such as polyoxyethylated sorbitol, polyoxyethylated glucose, polyoxyethylated glycerol (POG). The PEG has a preferred average molecular weight between 1,000 and 40,000, more preferably between 2,000 and 20,000, most preferably between 3,000 and 12,000.

Another drug delivery system for increasing circulatory half-life is the liposome. Methods of preparing liposome delivery systems are discussed in Gabizon et al., Cancer Research (1982) 42:4734; Cafiso, Biochem. Biophys. Acta (1981) 649:129; and Szoka, Ann. Rev. Biophys. Eng. (1980) 9:467. Other drug delivery systems are known in the art and are described in e.g., Poznansky et al., Drug Delivery Systems (R. L. Juliano, Ed., Oxford, N.Y. 1980), pp. 253-315; M. L. Poznansky, Pharm. Revs. (1984) 36:277.

After a liquid pharmaceutical composition is prepared, it is preferably lyophilized to prevent degradation and to preserve sterility. Methods for lyophilizing liquid compositions are known to those of ordinary skill in the art. Just prior to use, the composition may be reconstituted with a sterile diluent (Ringer's solution, distilled water, or sterile saline, for example) which may include additional ingredients. Upon reconstitution, the composition is preferably administered to subjects using those methods that are known to those skilled in the art.

As stated above, the antibodies and compositions of this invention are used to treat mammalian, and preferably human subjects to prevent or treat meningeal pathogenic infections. The preferred route of administration is parenterally. In parenteral administration, the compositions of this invention will be formulated in a unit dosage injectable form such as a solution, suspension or emulsion, in association with a pharmaceutically acceptable parenteral vehicle. Such vehicles are inherently nontoxic and nontherapeutic. Examples of such vehicles are saline, Ringer's solution, dextrose solution, and Hanks' solution. Nonaqueous vehicles such as fixed oils and ethyl oleate may also be used. A preferred vehicle is 5% dextrose in saline. The vehicle may contain minor amounts of additives such as substances that enhance isotonicity and chemical stability, including buffers and preservatives.

The dosage and mode of administration will depend on the individual. Generally, the compositions are administered so that antibodies are given at a dose between 1 µg/kg and 20 mg/kg, more preferably between 20 µg/kg and 10 mg/kg, most preferably between 1 and 7 mg/kg. Preferably, it is given as a bolus dose, to increase circulating levels by 10-20 fold and for 4-6 hours after the bolus dose. Continuous infusion may also be used after the bolus dose. If so, the antibodies may be infused at a dose between 5 and 20 µg/kg/minute, more preferably between 7 and 15 µg/kg/minute.

The antibody of the present invention may be used prior to infection as a precaution, or after infection has occurred as a therapeutic treatment. Preferably, the therapeutic use of the antibodies as described herein or fragments thereof include administration prior or during the acute invasive phase of the disease.

### Vaccine Formulations and Methods of Administration

The vaccine antigens of this invention are administered at a concentration that is therapeutically effective to prevent or treat infections by meningeal pathogens. To accomplish this goal, the vaccines may be formulated using a variety of acceptable excipients known in the art. Typically, the vaccines are administered by injection, either intravenously or intraperitoneally. Methods to accomplish this administration are known to those of ordinary skill in the art.

Preferably the vaccine contains at least 50 µg of antigenic mass per dose, and most preferably 80 µg per dose. The antigenic mass being the mass of the antigen protein. Vaccines according to the present invention with an antigenic mass up to 275 µg per dose could even be prepared, and such vaccines may still not elicit local reactions at the injection site. Of course even more micrograms of antigen can be put in a vaccine dose of a vaccine according to the invention, but if the protection obtained with the vaccine is not improved with a higher dose the increase in antigenic load only results in the vaccine being more expensive than necessary. In addition an increasing dose of antigen may eventually lead to unacceptable local reactions at the injection site, which should be avoided.

A vaccine according to the invention may contain a (partially) purified or recombinant MgtA protein or an antigenic part thereof, wherein said recombinant protein is preferably produced by way of expression from an expression vector in suitable host cells, such as bacterial, fungal, insect or mammalian cells, said expression vector containing the *mgtA* gene sequence or an immunogenic part thereof under control of a suitable promoter. Suitable protein expression systems are known in the art and may be used as applied nromally by a person skilled in the art to prepare a vaccine component according to the invention.

A vaccine according to the invention may further comprise a suitable adjuvant. Many adjuvant systems are known in the art, for example commonly used oil in water adjuvant systems. Any suitable oil may be used, for example a mineral oil known in the art for use in adjuvantia. The oil phase may also contain a suitable mixture of different oils, either mineral or non-mineral. Suitable adjuvantia may also comprise vitamin E, optionally mixed with one or more oils. The water phase of an oil in water adjuvanted vaccine will contain the antigenic material. Suitable formulations will usually comprise from about 25-60% oil phase (40-75% water phase). Examples of suitable formulations may comprise 30% water phase and 70% oil phase or 50% of each.

The vaccine formulations of the present invention may be used in prophylactic methods of the invention by immunizating an animal by introducing said formulations into an animal subcutaneously, intramuscularly, intranasally, intradermally, intravenously, transdermally, transmucosally, orally, or directly into a lymph node. In another embodiment, the composition may be applied locally, near a local pathogen reservoir against which one would like to vaccinate.

The present invention further provides a method for the manufacture of a vaccine intended for the protection of a subject against infection by a meningeal pathogen, wherein said vaccine is combined with a pharmaceutically acceptable diluent, carrier, excipient or adjuvant therefore such that a formulation is provided which can provide a dose of at least 20 µg/of MgtA in a single administration event.

A vaccine (prepared by a method) according to the invention can be used in a method to protect a subject against infection by a meningeal pathogen.

To provide adequate protection the vaccine is preferably administered in a 2 shot vaccination regimen, whereby the first shot (priming vaccination) and second shot (boosting vaccination) are given to the subject with a interval of about 3 weeks. In this way the subject will have obtained full protection against infection by a meningeal pathogen. The vaccination is very favourable for young children.

The vaccine of the present invention can also advantageously comprise other antigens or antibodies which would be useful to combat infection for the same pathogen or multiple pathogens . Such antigens or antibodies can be any antigens or antibodies which are commonly used to prevent or treat the infection. Further, the vaccine of the invention can be advantageously co-administered with any antibiotic treatment. The vaccine of the invention will inhibit the growth of the bacteria, in particular *S. pneumoniae* and prevent transcytosis, which means that the bacteria will be present in the body in places (such as the circulation), where they are prone to antibiotic treatment.

The vaccine formulations and pharmaceutical compositions of the present invention can thus be used for preventing transcytosis, and thus are ideal for preventing or treating meningitis.
Further, the vaccine formulations and pharmaceutical compositions of the present invention will inhibit the growth of meningeal pathogens, and will therefore be suited for prevention or treatment of pneumonia, sepsis, pericarditis and any other infection caused by the meningeal pathogens, whereby the vaccine formulations and pharmaceutical compositions of the invention can be co-administered with other immunogenic determinants or with antibiotics.

### EXAMPLES

### Materials & Methods

### Bacterial strains and growth conditions

Strains used in this study are listed in Table 1 and were stored in 10 % glycerol at -80 °C. *S. pneumoniae* was grown as standing cultures in M17 broth supplemented with 0.5 % glucose (GM17) at 37 °C. For growth on plates, 3 % defibrinated sheep blood (Johnny Rottier, Kloosterzande, The Netherlands) was added to GM17 agar. When needed the antibiotics erythromycin, kanamycin, chloramphenicol and tripmethoprim were added at concentrations of, respectively, 0.25 ug/ml, 500 ug/ml, 2 µg/ml and 15 ug/ml for *S. pneumoniae*.

**Table 1: Used strains and plasmids in this study**

| **Strains/Plasmid** | **Description** | **Reference or source** |
|---|---|---|
| ***S. pneumoniae*** | | |
| D39 | Serotype 2 strain | Avery et al., Mol. Med. 1:344 (1995) |
| R6 | Unencapsulated serotype 2; labstrain | Hoskins et al., J. Bacteriol. 183:5709 (2001) |
| D39Δ*cps* | D39 lacking capsule locus, Kan^{R} | Example 1 |
| D39*ΔcpsΔmgtA* | D39Δ*cps* lacking *mgtA*, Kan^{R}, Ery^{R} | Example 1 |
| D39*ΔcpsnisRK* | D39 *ΔcpsΔbgaA::nisRK,* Kan^{R}, Trmp^{R} | Example 1 |
| D39*ΔcpsΔmgtAnisRK* | D39 *ΔcpsΔmgtAΔbgaA::nisRK,* Kan^{R}, Ery^{R}, Trmp^{R} | Example 1 |
| ***L. lactis*** | | |
| NZ9000 | MG1363 *ΔpepN::nisRK* | Kuipers et al., Journal of Biotechnology 64:15 (1998) |
| **rPlasmids** | | |
| pNZ8048 | Cm^{R}; nisin-inducible P*nisA* | De Ruyter et al., Appl. Environ. Microbiol. 62:3662 (1996) |
| pNZ8048::*mgtA* | Cm^{R}; *mgtA* under nisin-inducible PnisA | Example 1 |
| pTK3 | Ery^{R} Trmp^{R}; containing 3'and 5' fragments of D39 *bgaA*, harbouring *nisRK* genes in between | Kloosterman et al. Microbiology 152:351 (2006) |

| | | |
|---|---|---|
| Kan^{R}: Kanamycin resistance, Ery^{R}: Erythromycin resistance, Trmp^{R}: Trimethoprim resistance, Cm^{R}: Chloramphenicol resistance. | | |

### Strain and plasmid construction

### Production of Δcps mutant in D39

*S. pneumoniae* D39Δ*cps* was produced by replacement of the capsule locus by a kanamycin resistance gene, as described by Pearce et al. (29). The resulting Δ*cps* mutation (see Figure 2) was confirmed by PCR, using primers annealing upstream of *dexB* and at the 3' site of *kanR* (dcps.control.fw/Kana2) and using primers annealing at the 5' site of *kanR* and downstream of *aliA* (Kana 1/d*cps*.control.rev) (Table 2), resulting in fragments of 2033 and 2408 bp, respectively.

### Generation of ΔmgtA (sp1551) mutant in D39Δcps

For the production of a knockout strain of *mgtA,* the 3' and 5' flancking regions of *mgtA* (SPD_1383,sp1551) in *S. pneumoniae* D39Δ*cps* were amplified using primers SP1550for/SP1550rev and SP1552for/SP1552rev (Table 2),, respectively. The erythromycin resistance gene was amplified from plasmid pORI13 by making use of the primers Eryfor and Eryrev. The 3 different PCR fragments were fused by making use of overlapping sequences for the erythromycin resistance gene in the primers SP1550rev and SP1552for, using the outward primers SP1550for and SP1552rev for amplification (Table 2),. This resulting PCR product of 2052 bp was transferred to D39Δ*cps* and the bacteria were plated on GM17 plates containing 3% sheep blood, erythromycin (0.25 ug/ml) and 10 mM MgCl₂. The deletion of *mgtA* from the genome (see Figure 3) was confirmed by PCR using SP1550fw and SP1552rev (Table 2), and Southern blotting.

### Generation of nisRK integration in D39Δcps and D39ΔcpsΔmgtA

For the production of D39Δ*cpsnisRK* and D39Δ*cps*Δ*mgtAnisRK*, the nisRK genes were amplified by making use of the primers *bgaA*-1 and *bgaA*-4 (Table 2), used with pTK3 as a template. The resulting PCR product was integrated via double cross-over in the *bgaA* locus, and the correct replacement was confirmed by Southern blotting.

### Generation of pNZ8048:mgtA

To be able to complement the mgtA knockout mutant, a plasmid was constructed containing the *mgtA* gene under control of a nisin inducible promoter (P*nisA*) (see Figure 4). The *mgtA* gene was amplified from the chromosome of R6 by making use of the primers mgtacomplsta and mgtastop (Table 2),. The resulting 2749 bp fragment was digested with NcoI and XbaI and ligated into the complementary sites of pNZ8048. This ligation mix was transferred to *L. lactis* NZ9000. When restriction analysis and sequencing confirmed that the right construct was produced, the plasmid pNZ8048::mgtA was transferred to D39*ΔcpsΔmgtAnisRK* for complementation studies of the *mgtA* knockout mutant.

**Table 2: Primers used in this study**

| Primer | Nucleotide sequence (5' to 3') | Restriction site |
|---|---|---|
| Dcap.control.fw | GGCAGACAGAGTTAGGAGTTGAG | |
| Dcap.control.rev | CCCAGTCTTCGCCAGCAGC | |
| Kana.1 | GTGAATTGGAGTTCGTCTTG | |
| Kana.2 | TATGGACAGTTGCGGATGTA | |
| *bgaA*-1 | GCTCTAGACAAATCGTTGAACGAGGTGG | XbaI |
| *bgaA*-4 | GAAGATCTGTAATTTGATAGTCTTGACG | BglII |
| Sp1550 for | GCACCTGCTTGCCAGAATAG | |
| Sp1550rev | AGTTATCGGCATAATCGTTATCGGATGTCAGC ATATCAAT | |
| Sp1552for | GAGATCTAATCGATGCATGCCGGAATGACGAC ACTATCAC | |
| Sp1552rev | GCACCAAGAGGATCAATGAC | |
| Eryfor | TAACGATTATGCCGATAACT | |
| Eryrev | GCATGCATCGATTAGATCTC | |
| mgtacomplsta | CATGCCATGGCAGAAAGAGAGTCTGTTTTGC | NcoI |
| *mgtA*stop | TCTAGACCTTAAATCGCATTCTTG | XbaI |

### Growth experiments with D39Δcps and D39ΔcpsΔmgtA

Overnight cultures of *S. pneumoniae* used for growth experiments were diluted 50 times in GM-17 and grown until OD₆₀₀ 0.2. Until use, the samples were supplemented with 10% glycerol and stored at -80°C. Growth experiments were performed in 94 wells microtiter plates containing 5 µl bacterial culture and 195 µl GM17 containing various concentrations of different metal ions as described in the results. Growth of the strains was followed by OD₅₉₅ measurements (GENios, Tecan) for 15 hours using an interval time of 30 minutes 37°C ±0.5°C. Prior to every measurement the plate was shaked for 3 seconds with a normal intensity and orbital mode.

### Endothelial cell line

In this study, immortalized Human Brain Microvascular Endothelial Cells (HBMEC) were used up to passage 36. The cells were obtained from Kwang S. Kim (Johns Hopkins Univ School of Medicine, Baltimore, USA) Cells were cultivated in RPMI1640 (Biochrom), supplemented with 10% FCS (Biochrom), 10% Nu-serum (BD Biosciences), 2 mM L-glutamin (Gibco), 1 mM Na-pyruvate (Gibco), 1% MEM-vitamines (Gibco) and 1% non-essential amino acids (Gibco) at 37°C, 5% CO2. The cells were subcultured every third or fourth day, by first washing the cells with PBS followed by incubation with 1.5 ml prewarmed Trypsin/EDTA (Sigma). Following the removal of trypsin after 0.5 minutes, the cells were incubated at 37C until they lifted off from the culture flask (usually 3 to 4 minutes). They were then resuspended in fresh medium and seeded in the right dilution (usually 1:4 or 1:6) for further growth.

### Endothelial cell adherence and invasion

For adherence and invasion studies of HBMEC were seeded in a concentration of 1*10⁴ cells per well and incubated for 2 days. When the monolayer was 100% confluent the cell layer was washed twice with 500 ul PBS/well and incubated for 1 hour with culture medium containing 10 ng/ml TNFα to activate the expression of the PAF receptor. 1*10⁷ CFU/well *S. pneumoniae* from an aliquot of OD60000.2 was washed with 1 ml DPBS with Mg²⁺ and Ca²⁺ (Cambrex) and added to each well in 500 ul cell culture medium, also containing 10 ng/ml TNFα. After 2 hours of incubation, HBMEC cells were washed 2 times with DPBS with Mg and Ca (Cambrex) to remove unadhered bacteria and lysed by 5 minutes incubation in 100 ul DPBS/0.1% Triton X-100. Serial dilutions of harvested bacteria were plated on Columbia agar plates containing 3% sheep blood and 10 mM MgCl₂.

To kill extracellular *S. pneumoniae*, the monolayers in the remaining wells were washed twice with DPBS containing Mg²⁺ and Ca²⁺, and the medium was supplemented with 200 ug/ml gentamycin. After 1 hour of incubation the medium was plated on Columbia agar plates containing 3% sheep blood and 10 mM MgCl₂ to ensure that the antibiotic concentration was sufficient to kill the extracellular bacteria. The monolayer was washed twice with DPBS containing Mg²⁺ and Ca²⁺ and lysed by 3 minutes incubation in 100 ul DPBS/0.1%Triton X-100. Serial dilutions of harvested bacteria were plated on Columbia agar plates containing 3% sheep blood and 10 mM MgCl₂. To quantify the amount and viability of the detached HBMEC cells when 1E8 *S. pneumoniae* D39Fcps or D39FcpsFmgtA was added, trypan blue (0.4%) was used in a 1:1 dilution. The culture medium was analysed after 2 or 3 hours of infection, for the presence of detached HBMEC by microscopy.

### Transcytosis assay

To study the transcytosis of *S. pneumoniae* through HBMEC, a double chamber culture system was set up using Transwell polycarbonate filters (3um pore size, Cat. no. 3402, Corning Costar Corp.) placed into a 12 wells plate (Ring A et al. J. Clin. Invest 102, 347 (1998)). The HBMEC cells were seeded at a concentration of approximately 1*10⁵ cells/well and these wells were cultivated for 3 days in a petridish to prevent growth of the HBMEC cells on the wrong side of the filter. Routinely 0.5 ml of fluid was kept in the upper chamber and 1.5 ml in the lower chamber throughout the experiment.

Monolayers were incubated for 30 minutes in culture medium containing 10 ng/ml TNFα to activate the expression of the PAF receptor (Ring et al J. Clin. Invest. 1998). The monolayers were infected for 2 hours with 1*10⁷ CFU/well of unencapsulated *S. pneumoniae* strains from an aliquot of OD₆₀₀≈0.2 as described before. After two hours the monolayer was washed twice with DPBS with Mg²⁺ and Ca²⁺ and antibiotic-containing medium (10 ug/ml penicillin G and 200 ug/ml gentamycin) was added to both chambers for 1 hour to kill extracellular bacteria. The monolayers were washed again twice with DPBS with Mg²⁺ and Ca²⁺ and culture medium containing 0.04 ug/ml penicillin G (Sigma) was added to the upper chamber of the transwell® system. Culture medium without antibiotics was added to the lower chamber of the system to let the bacteria transcytose when possible. During this assay, at various stages monolayers were sacrificed to analyse the number of adhered or invaded bacteria, as described in the previous section. Serial dilutions of these samples were plated on Columbia agar plates containing 3% sheep blood and 10 mM MgCl₂.

### Permeability measurements

To determine the stability of the monolayer of HBMEC during the transcytosis assay, the diffusion of Lucifer Yellow through the monolayer at different stages of the experiment was monitored. Therefore 0.5 ml of 400 ug/ml Lucifer Yellow dipotassium salt (Sigma) was added to the upper chamber and 1.5 ml of DPBS with Mg²⁺ and Ca²⁺ was added to the lower chamber. After 5 minutes of incubation at room temperature, the fluorescence of the solution in the lower chamber was measured at 535 nm emmision and 485 nm excitation wavelength, in a 96 wells plate.

### Results

### MgtA is the main Mg²⁺ transporter in S. pneumoniae under in vitro growth conditions.

ORF SP1551 (SPD_1383, spr1401) putatively encodes a cation-transporter domain, a P-type ATP-ase domain and haloacid dehalogenase-like hydrolase domain and has been designated as *mgtA*. Comparison of the Salmonella MgtA amino acid sequence, which has been shown to be a Mg²⁺ transporter, with the *S. pneumoniae* genome, showed that SPD_1383 is the ORF with the highest similarity with the salmonella *mgtA* gene of all transporters in the genome. To determine whether SP1551(spr1401, SPD_1383) is indeed an *mgtA* homologue the gene was deleted from the genome of R6 using PCR mutagenesis. Interestingly, colonies obtained after transformation grew slow and remained small and this effect was reversed after addition of extra MgCl₂ (1 mM) to the blood plates. The deletion of ORF SP1551 was confirmed by Southern blot and subsequently growth of the parent strains and the mutants was compared in GM17 broth. As on plates growth of the R6 mutants was poor, but could be partially restored by the addition of 5 mM MgCl₂ to the growth medium, addition of ten times more MgCl₂ (50 mM) did not further improve growth (Fig. 5). This indicates that *mgtA* is the main Mg²⁺ importer of *S. pneumoniae* under in vitro growth conditions, but that there are also other low affinity transporters present that can partially compensate for the deletion of *mgtA* when an excess of MgCl₂ is present.

Addition of either CaCl₂, MnCl₂, CuCl₂ and ZnCl₂ did not restore the growth of the *mgtA* mutant in GM17, whereas the parent strain grew normal under these conditions (data not shown) (Fig. 6). The initial increase in OD observed in the medium where MnCl₂ was added, was due to complex formation of the Mn²⁺ and a component in the GM17, as the same was observed in medium without bacteria (data not shown). Lower concentrations of MnCl2 did not show a restoration of growth of the mutant.These data indicate that mgtA specifically transports magnesium.

To study the effect of the deletion of *mgtA* on the interaction of *S*. *pneumoniae* and HBMEC, we first transferred the deletion into strain D39Δ*cps* as deletion of the capsule greatly enhances the adherence of *S. pneumoniae* to eukaryotic cells. Again colonies were small on plate and grew slower than the parent strain. As in strain R6, growth of the mutant in broth was poor, and this growth defect could be reversed by the addition of MgCl₂ to the medium (Fig 7).

Interestingly, the D39*ΔcpsΔmgtA* mutant does not seem as impaired as the R6 mutant, which indicates that in D39 there are either other transporters, they have other affinities or they are differentially regulated. To determine whether this phenotype was solely due to the deletion of *mgtA* and not secondary effects, the *mgtA* gene was cloned into the pNZ8048 vector (see above) and introduced in strain D39*ΔcpsΔbgaAnisRKΔmgtA*. As a control the plasmid pNZ8048 without an insert was also introduced into this strain.

After transformation, colonies that had received the plasmid with the *mgtA* gene grew better than those that had received plasmid pNZ8048, which indicated that the heterologous expression of the *mgtA* gene in the *ΔmgtA* mutant restored growth to wild type levels. This was confirmed when the growth of D39*ΔcpsΔbgaAnisRKΔmgtA* containing pNZ8048 and D39*ΔcpsΔbgaAnisRKΔmgtA* containing p*mgtA* was compared to that of D39*ΔcpsΔbgaAnisRK* in GM17 broth (Fig. 7). As observed before, growth of the mutant containing pNZ8048 was impaired in GM17. In contrast, growth of the mutant was completely restored to wild type levels when the plasmid contained the *mgtA* gene (Fig. 7). This demonstrates that the phenotype of the *ΔmgtA* mutant is solely due to the disruption of the *mgtA* gene.

### A mgtA mutant is unable to translocate monolayers of Human Brain Microvascular Endothelial cells

Human Brain Microvascular Endothelial Cells (HBMEC) were grown as described previously (Stins et al. 2001. Microb. Pathog. 30:19) into a tight monolayer in a transwell® system that consists of a membrane with a pore size that allows for the passage of bacteria but not eukaryotic cells. The bacteria are co-incubated with the cells in the upper compartment of this system, which enables them to adhere to and subsequently invade the endothelial cells. The ability to translocate is assessed by following the appearance of bacteria in the lower compartment over time (Fig 1B). To increase the efficiency of adherence and invasion, unencapsulated strains were used. Co-incubation of D39Δ*cps* with the HBMEC cells in a transwell® system resulted in the appearance of the bacteria in the lower compartment over time, while the stability of the monolayer was unaffected, demonstrating that the bacteria went through the endothelial cells before ending up in the lower compartment (data not shown).

Subsequently, equal amounts of strains D39Δ*cps* and D39*ΔcpsΔmgtA* were added to the HBMEC and adhesion to, invasion, survival in and translocation of the endothelial cells of both strains was compared.

Adhesion of the *ΔmgtA* mutant was about one log lower than that of the parent strain and, subsequently invasion was slightly lower than that of the parent strain (Fig 8A). However, the percentage of adherent bacteria that invaded and survived were similar in both strains, indicating that the mutant is not impaired in invasion of and survival in HBMEC. This is in contrast to what was found when the transcytosis of both strains was compared. Over time, as observed before, the parent strain appeared in the lower compartment. However, the mutant failed completely to reach the lower compartment (Fig. 8B), indicating that it is unable to traverse the cell layer. This was not due to a trivial reason such as the mutant sticking to the membrane as it was able to traverse the transwell when no endothelial cells were present (data not shown).

To determine that this effect was also solely due to the mutation of *mgtA*, the same strains as for the complementation assay in GM17 were used in the transcytosis assay. To ensure optimal *mgtA* expression, aliquots of all three strains were grown with nisin present in the growth medium. In these experiments, the *mgtA* mutant containing the empty vector was unable to cross the eukaryotic cell layer. Strain D39 *Δcps bgaA*::NisRK and strain D39*bgaA*:;NisRK *ΔcpsΔmgtA* containing the plasmid with the *mgtA* gene did cross the eukaryotic cell layer as they appeared in the lower compartment over time (Figure 8C), demonstrating that the observed defect in translocation is indeed due to the deletion of *mgtA.*

To further demonstrate that it is the uptake of magnesium which is important for the translocation of *S. pneumoniae* over the cell layer and not some other putative function of *mgtA,* we determined what happened to the mutant when extra magnesium was added to the culture medium while the bacteria were co- incubated with the HBMEC. Addition of Mg²⁺, will ensure that the bacteria are constantly in a condition of high Mg concentration as the medium will also be present in the vesicles when the bacteria are taken up. As is clear in figure 8D, addition of 10 mM MgCl₂ to the tissue culture medium in the upper compartment restored translocation of the mutant. This indicates that the Mg²⁺ uptake by *MgtA* is important for transcytosis.

### Coinfection of HBMEC with the mgtA mutant and parent strain does not restore translocation of the mutant.

To determine whether the simultaneous presence of the wildtype and *ΔmgtA* mutant could restore translocation of the mutant, the HBMEC were infected with mixtures of the parent strain and the mutant in various ratios. To determine the ratio between the mutant and the parent strain, bacteria were plated in replicate, once on normal Columbia agar plates and once on Columbia agar plates with erythrymocin, to specifically detect the mutant. To ensure that all *mgtA* mutants were detected, 10 mM MgCl₂ was added to the all plates as well. As can be observed in Figure 9 even a 100 fold surplus of the wildtype did not result in the appearance of the mutant in the lower compartment, indicating that the role of *mgtA* in the translocation process cannot be complemented by the presence of bacteria that posses a functioning protein.

Furthermore, it seems that a surplus of the mutant inhibits the translocation of the parent strain.

## Claims

1. A vaccine formulation providing protection against infection of a subject by a meningeal pathogen, said formulation comprising an effective amount of the protein MgtA or a functional homologue or an immunogenic part thereof together with at least one of a pharmaceutically acceptable diluent, carrier, excipient or adjuvant therefore.

2. A formulation according to claim 1, wherein said immunogenic part is an antigenic determinant of said pathogen.

3. A formulation according to claim 1 or 2, wherein said formulation comprises an essentially cell-free preparation of said pathogen comprising said protein or said part thereof.

4. A formulation according to claim 2 or 3, wherein said immunogenic part of said protein comprises a part that is exposed on the outer membrane of said pathogen.

5. A formulation according to any one of claims 1-4, wherein said formulation provides protections against meningitis, pneumonia, and/or sepsis caused by a meningeal pathogen.

6. A formulation according to any one of the preceding claims, wherein the formulation provides protections against meningeal streptococci, preferably selected from the group consisting of β-haemolytic *Streptococcus* spp. belonging to Lancefield group C, *S. acidominimus, S agalactiae, S. bovis, S. constellatus, S. equinus, S. equisimilis, S. mitis, S. oralis, S. pneumoniae, S. pyogenes, S. salivarius, S. sanguis, S. suis* and *S. uberis,* more preferably selected form the group consisting of *S agalactiae, S. pneumoniae, S. pyogenes, S. suis* and *S. uberis.*

7. The protein MgtA or an immunogenic part thereof, for use as a vaccine.

8. An anti-MgtA antibody or fragment thereof, preferably a humanized anti-MgtA antibody or fragment thereof.

9. An anti-MgtA antibody or fragment thereof, preferably a humanized anti-MgtA antibody or fragment thereof, for use as a medicament for the prophylactic or therapeutic treatment of an infection by a meningeal pathogen in a mammalian subject, preferably a human subject.

10. Use of anti-MgtA antibody or fragment thereof, preferably a humanized anti-MgtA antibody or fragment thereof, for the manufacture of a medicament for the prophylactic or therapeutic treatment of an infection by a meningeal pathogen in a mammalian subject, preferably a human subject.

11. A pharmaceutical composition comprising an anti-MgtA antibody or fragment thereof, preferably a humanized anti-MgtA antibody or fragment thereof, and a pharmaceutically acceptable carrier.

12. A method for prophylactic or therapeutic treatment of an infection by a meningeal pathogen in a mammalian subject, preferably a human subject comprising administering to said subject in need of such treatment an effective amount of a vaccine formulation as defined in any one of claims 1-6 and/or an effective amount of a pharmaceutical composition as defined in claim 11.

13. A method for preparing an MgtA-based vaccine formulation, the said method comprising bringing into association, an effective amount of the protein MgtA or an immunogenic part thereof and at least one of a pharmaceutically acceptable diluent, carrier, excipient or adjuvant therefore.

14. A method for preparing a pharmaceutical composition as defined in claim 11, the said method comprising bringing into association, an effective amount of an anti-MgtA antibody, preferably a humanized anti-MgtA antibody, or fragment thereof, and a pharmaceutically acceptable carrier.
